(19) Europäisches Patentamt
European Patent Office
Office européen des brevets

(11) EP 3 042 903 B1

(12) **EUROPEAN PATENT SPECIFICATION**

(45) Date of publication and mention of the grant of the patent:
**14.08.2019 Bulletin 2019/33**

(51) Int Cl.:
*C07D 471/04* (2006.01) *A61K 31/553* (2006.01)
*C07D 487/04* (2006.01)

(21) Application number: **15155832.7**

(22) Date of filing: **19.02.2015**

(54) **Substituted hetero-bicyclic compounds, compositions and medicinal applications thereof**

Substituierte heterobicyclische Verbindungen, Zusammensetzungen und medizinische Anwendungen davon

Composés hétéro-bicycliques substitués, compositions et leurs applications médicales

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.01.2015 IN MA00972015**

(43) Date of publication of application:
**13.07.2016 Bulletin 2016/28**

(73) Proprietor: **Impetis Biosciences Ltd.**
**Mumbai City, Maharashtra 400001 (IN)**

(72) Inventors:
- **Thakkar, Mahesh**
  **411057 Maharashtra (IN)**
- **Koul, Summon**
  **411057 Maharashtra (IN)**
- **Bhuniya, Debnath**
  **411057 Maharashtra (IN)**
- **Mookhtiar, Kasim**
  **411057 Maharashtra (IN)**
- **Kurhade, Santosh**
  **411057 Maharashtra (IN)**
- **Munot, Yogesh**
  **411057 Maharashtra (IN)**
- **Mengawade, Tanaji**
  **411057 Maharashtra (IN)**
- **Kulkarni, Bheemashankar A.**
  **411057 Maharashtra (IN)**

(74) Representative: **Harris, Jennifer Lucy**
**Kilburn & Strode LLP**
**Lacon London**
**84 Theobalds Road**
**London WC1X 8NL (GB)**

(56) References cited:
**WO-A1-2010/000633** **WO-A1-2010/006947**
**WO-A1-2010/080481** **WO-A1-2010/100070**
**WO-A1-2010/122038** **WO-A1-2013/157022**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 042 903 B1

## Description

### Technical Field

The present disclosure relates to a class of substituted hetero-bicyclic compounds of formula (I), their tautomers, polymorphs, stereoisomers, solvates, hydrates, N-oxides, co-crystals, pharmaceutically acceptable salts and pharmaceutical compositions containing them. The disclosure also relates to the process of preparation of compounds of Formula (I). These compounds are useful in the treatment, prevention, prophylaxis, management, or adjunct treatment of all medical conditions related to inhibition of Bruton's tyrosine kinase (Btk), such as inflammatory and/or autoimmune disorder, cell proliferation, rheumatoid arthritis, psoriasis, psoriatic arthritis, transplant rejection, graft-versus-host disease, multiple sclerosis, inflammatory bowel disease, allergic diseases, asthma, chronic obstructive pulmonary disease (COPD) type 1 diabetes, myasthenia gravis, hematopoetic disfunction, B-cell malignancies, systemic lupus erythematosus, non-Hodgkin's lymphoma, chronic lymphocytic leukemia, Mantle cell lymphoma or other disorders.

### Background

Bruton's tyrosine kinase (Btk) belongs to tyrosine kinases, a subfamily of protein kinases that phoshporylate protein on the phenolic moiety of tyrosine residue. It is a non-receptor cytoplasmic tyrosine kinase and a key regulator for B-cell development, activation, signaling and survival (Schaeffer and Schwartzberg, Curr. Op. Imm. 2000, 282; Niiro and Clark, Nature Rev. Immumol. 2002, 945; Di Paolo and Currie, Nat. Chem. Biol. 2011, 7). Btk is expressed in all hematopoietic cell types, excluding plasma cells, T lymphocytes and natural killer cells. After activation by upstream B-cell antigen receptor or Toll like receptor-4, Btk induces PLC-$\gamma$2 phosphorylation, which ultimately results in activation of nuclear factor $\kappa$B (NP$\kappa$B) and nuclear factor of activated T cell dependent pathways. Additionally, Btk is crucial for number of other hematopoetic cell signaling; such as, Fc$\gamma$-mediated inflammatory cytokine production (such as TNF-$\alpha$, IL1$\beta$ and IL6) in monocytes/macrophages, IgE mediated signaling in mast cells, inhibition of Fas/APO-1 apoptotic signaling in B-lineage lymphoid cells *etc* (Jeffries et al. J. Biol. Chem. 2003, 26258; Horwood et al., J. Experimental Med. 2003, 1603).

Use of B-cell depleting protein based therapeutics (for example, Rituxan, a CD20 antibody) for the treatment of a number of autoimmune and/or inflammatory diseases provides enough evidence for the role of B-cells and the humoral immune system in the pathogenesis of autoimmune and/or inflammatory disease. And as Btk is essential for B-cell activation, this also provides an indirect rational for the role of Btk in autoimmune and/or inflammatory diseases.

Additional rationales for the involvement of Btk in various autoimmune and/or inflammatory diseases come from Btk deficient mouse models; for example, Btk deficiency has been shown to result in a marked amelioration of disease progression, in murine preclinical model of systemic lupus erythematosus (SLE). BTK deficient mice are resistant to develop collagen-induced arthritis (Jansson and Holmdahl Clin. Exp. Immumol. 1993, 459).

Pharmacological validation for the involvement of Btk in various autoimmune and/or inflammatory diseases comes from two different class of Btk inhibitors, for example, (a) an irreversible Btk inhibitor (Pan et al, Chem. Med. Chem. 2007, 58), and (b) a reversible Btk inhibitor (Di Paolo, Nat. Chem. Biol. 2010, 41). Both the type of inhibitors have demonstrated efficacy in a mouse model of arthritis. PCI-32765 (an irreversible Btk inhibitor), has shown promising clinical activity in chronic lymphocytic leukemia (CLL) and small lymphocytic lymphoma (SLL) (Burger et al, Blood 2010, 32 & J Clin Oncol 2011, 6508). Thus, small molecule Btk inhibitors are expected to be useful to treat disease processes that involve B-cell activation.

Several prior art literature describe the discovery of small molecule Btk inhibitors (for a recent review, Lou et al. J. Med. Chem. 2012). As for example, amino-pyrazolo-pyrimdine (WO2011046964A2) and imidazo[1,5-a]quinoxalines (Kim et al, Bio-org. Med. Chem. Lett., 2011, 6258) based compounds, which utilize its acrylamide functionality for covalent modification of the target protein, are published as irreversible inhibitors. There are literature prior art disclosing reversible Btk inhibitors, such as, substituted amino-pyrimidine analogs (WO2010123870A1); imidazo[1,2-f][1,2,4]triazine core based amide compounds (WO2010068810A2); various heterocyclic (e. g. imidazo[1,2-a]pyrazine, aminopyrimidine, 2-pyridone *etc*) amide derivatives (WO2010068788A1, WO2010068806A1); benzo[f][1,4]oxazepin analogs (WO2010122038A1); 3,4-dihydro-2H-isoquinoline or 2H-isoquinolin-1-one based analogs (WO2010100070A1, WO2013024078); phenylimidazopyrazine alalogs (WO2010006970A1); 3,5-disubstituted pyrid-2-one analogs (WO2007027729A1); azaindazole compounds (WO2011019780A1); nicotinamide compounds (WO2010144647A1); carbazole carboxamide analogs (WO2010080481A1), pyrrolopyrimidine derivatives (WO2013008095).

WO2013157022 discloses substituted hetero-bicyclic compounds and their therapeutic applications in the treatment, prevention and management of conditions associated with Bruton's tyrosine kinase (Btk).

WO2010000633 discloses 5-phenyl-1H-pyrazin-2-one derivatives that have been shown to modulate activity of Bruton's tyrosine kinase (Btk) and treat diseases associated with excessive Btk activity. Compounds disclosed herein have also been shown to treat inflammatory and auto immune diseases associated with aberrant B-cell proliferation

such as rheumatoid arthritis.

6-Phenyl-imidazo[1,2-a]pyridine and 6-phenyl-imidazo[1,2-b]pyridazine derivatives such as those disclosed in WO2010006947, and 5-phenyl-1H-pyridin-2-one, 6-phenyl-2H-pyridazin-3-one, and 5-phenyl- 1H-pyrazin-2-one derivatives such as those disclosed in WO2010100070 have also been shown to modulate the activity of Btk and treat diseases associated with excessive Btk activity. Further, the disclosed compounds have also been shown to treat inflammatory and auto immune diseases associated with aberrant B-cell proliferation such as rheumatoid arthritis.

Despite several discoveries in this area, there is no small molecule Btk inhibitor available in the market. The most advanced compounds in the clinical trials are irreversible inhibitors of Btk. Such an irreversible mechanism of action often encounters nonspecific binding with proteins including off-targets leading to safety concern particularly upon long term used such as off-target related adverse side effects, general hepatotoxicity and organ toxicity, antibody mediated reactions. Therefore, there is a need of Btk inhibitors with safer mechanism of action such as reversible mechanism of inhibition. These compounds will have medical application in the disease area of inflammation, autoimmune disorder and cell proliferation, rheumatoid arthritis, psoriasis, psoriatic arthritis, transplant rejection, graft-versus-host disease, multiple sclerosis, inflammatory bowel disease, allergic diseases and asthma, type 1 diabetes, myasthenia gravis, hematopoetic disfunction, B-cell malignancies, systemic lupus erythematosus.

## Summary

The present disclosure provides hetero-bicyclic compounds of formula (I), their tautomers, polymorphs, stereoisomers, solvates, hydrates, N-oxides, co-crystals, pharmaceutically acceptable salts, pharmaceutical compositions containing them and compounds for use in treating conditions and diseases that are mediated by Bruton's tyrosine kinase (Btk) activity, such as autoimmune disorders, inflammatory diseases, allergic diseases, airway diseases, such as asthma and chronic obstructive pulmonary disease (COPD), transplant rejection; diseases in which antibody production, antigen presentation, cytokine production or lymphoid organogenesis are abnormal or are undesirable; such as rheumatoid arthritis, systemic onset juvenile idiopathic arthritis (SOJIA), gout, pemphigus vulgaris, idiopathic thrombocytopenic purpura, systemic lupus erythematosus, multiple sclerosis, myasthenia gravis, Sjogren's syndrome, autoimmune hemolytic anemia, anti-neutrophil cytoplasmic antibodies (ANCA)-associated vasculitides, cryoglobulinemia, thrombotic thrombocytopenic purpura, chronic autoimmune urticaria, allergy (atopic dermatitis, contact dermatitis, allergic rhinitis), atherosclerosis, type 1 diabetes, type 2 diabetes, inflammatory bowel disease, ulcerative colitis, morbus Crohn, pancreatitis, glomerulonephritis, Goodpasture's syndrome, Hashimoto's thyroiditis, Grave's disease, antibody-mediated transplant rejection (AMR), graft versus host disease, B cell-mediated hyperacute, acute and chronic transplant rejection; thromboembolic disorders, myocardial infarct, angina pectoris, stroke, ischemic disorders, pulmonary embolism; cancers of haematopoietic origin such as multiple myeloma; leukemia; acute myelogenous leukemia; chronic myelogenous leukemia; lymphocytic leukemia; myeloid leukemia; non-Hodgkin lymphoma; lymphomas; polycythemia vera; chronic lymphocytic leukemia; Mantle cell lymphoma, essential thrombocythemia; myelofibrosis with myeloid metaplasia; and Waldenstroem disease,

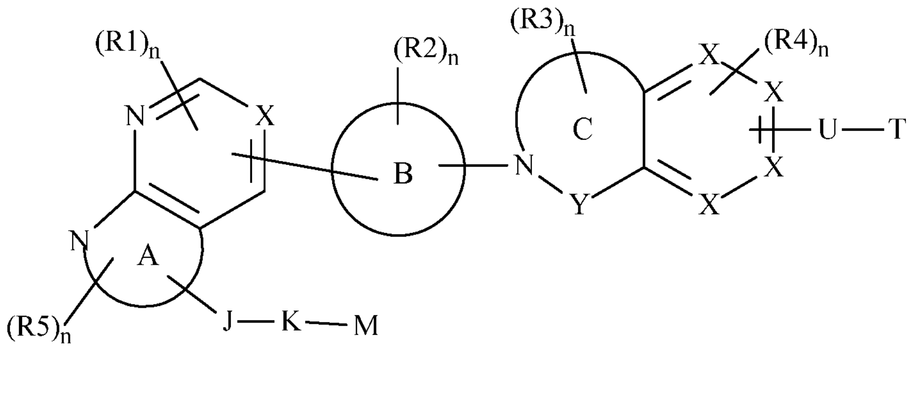

I

wherein,

- ring A represents a 5 membered ring which is unsaturated having upto three heteroatoms independently selected from O, N or S;
- ring B represents a 6 membered ring which is unsaturated optionally having upto three heteroatoms independently selected from O, N or S;
- ring C represents a 7 membered ring which is unsaturated or partially unsaturated optionally having upto three heteroatom independently selected from O, N or S;

each X independently represents $C(R^1)$;
Y represents -C(O);
J is absent or is selected from the group consisting of arylene, heterocyclylene, heteroarylene, $(C_{1-6})$alkylene, $(C_{1-6})$alkenylene or $(C_{1-6})$alkynylene;
K is a bond or $(C_{1-6})$alkylene wherein optionally one or more than one methylene groups of alkylene are independently replaced by hetero atoms or groups such as -O-, $-N(R^6)-$, -C(O)-; M is selected from hydrogen, cyano, halogen, haloalkyl, perhaloalkyl, alkyl, alkenyl, alkynyl, cyanoalkyl, acyl, $-(CR^aR^b)_mOR^6$, $-SR^6$, $-(CR^aR^b)_mCOOR^6$, $-(CR^aR^b)_mNR^7R^8$, $-(CR^aR^b)_mC(O)NR^7R^8$, $-(CR^aR^b)_mNR^6C(O)NR^7R^8$, thiocarbonyl, $-S(O)_pR^6$, $-SO_3H$, cycloalkyl, aryl, arylalkyl, heterocyclyl or heteroaryl;
J, K and M is optionally substituted with one or more substituents independently selected from cyano, nitro, keto, oxo, halogen, haloalkyl, perhaloalkyl, hydroxyamino, $-(CR^aR^b)_mOR^6$, $-(CR^aR^b)_mC(O)R^6$, $-OC(O)R^6$, $-SR^6$, $-(CR^aR^b)_mCOOR^6$, $-(CR^aR^b)_mNR^7R^8$, $-(CR^aR^b)_mC(O)NR^7R^8$, $-(CR^aR^b)_mNR^6C(O)NR^7R^8$, $-NR^6C(O)R^6$, thiocarbonyl, $-S(O)_2NR^7R^8$, $-NR^6S(O)_2R^6$, $-S(O)_pR^6$, $-SO_3H$, alkyl, alkenyl, alkynyl, cycloalkyl, cyclkenyl, cycloalkylalkyl, aryl, heterocyclyl or heteroaryl;
wherein alkyl, alkenyl, alkynyl, cycloalkyl, cyclkenyl, cycloalkylalkyl, aryl, heterocyclyl or heteroaryl are optionally substituted with one or more substituents selected from hydroxy, alkyl, alkoxy, alkoxyalkyl, halogen, haloalkyl, perhaloalkyl, cyano, cyanoalkyl, amino, carboxy, carboxyalkyl, $-OC(O)R^6$, $-(CR^aR^b)_mC(O)NR^7R^8$, $-NR^6C(O)R^6$, $-SR^6$, $-S(O)_pR^6$, $-S(O)_2NR^7R^8$ or $-NR^6S(O)_2R^6$;
U is a bond or is selected from the group consisting of cycloalkylene or $(C_{1-6})$alkylene;
T is selected from hydrogen, cyano, halogen, haloalkyl, perhaloalkyl, alkyl, alkenyl, alkynyl, cyanoalkyl, acyl, $-(CR^aR^b)_mOR^6$, $-SR^6$, $-(CR^aR^b)_mCOOR^6$, $-(CR^aR^b)_mNR^7R^8$, $-(CR^aR^b)_mC(O)NR^7R^8$, $-(CR^aR^b)_mNR^6C(O)NR^7R^8$, $-S(O)_pR^6$ or $-SO_3H$;
Each of $R^1$, $R^2$, $R^3$, $R^4$ and $R^5$ are independently selected from hydrogen, alkyl, alkoxy, acyl, acylamino, acyloxy, $-(CR^aR^b)_mC(O)R^6$, $-(CR^aR^b)_mNR^7R^8$, $-(CR^aR^b)_mO(CR^aR^b)_nSi(R^7)_3$, aminocarbonyl, alkoxycarbonylamino, hydroxyamino, alkoxyamino, azido, cyano, halogen, hydroxy, hydroxyalkyl, haloalkyl, perhaloalkyl, thiocarbonyl, carboxy, alkylcarboxy, carboxyalkyl, carboxyalkyloxy, alkylcarboxyalkyloxy $-SO_3H$, alkylthio, aminosulfonyl, alkylsulfonyl, or nitro;
$R^6$ is selected from the group consisting of hydrogen, $-(CR^aR^b)_mOR^6$, halogen, haloalkyl, $-(CR^aR^b)_mC(O)R^6$, alkyl or cycloalkyl;
$R^7$ and $R^8$ are independently selected from the group consisting of hydrogen, $-(CR^aR^b)_mOR^6$, haloalkyl, $-(CR^aR^b)_mC(O)R^6$, alkyl, or
$R^7$ and $R^8$ taken together form a monocyclic or a bicyclic ring system which is saturated or partially unsaturated and optionally have additional heteroatoms selected from O, N or S;
$R^a$ and $R^b$ are independently selected from the group consisting of hydrogen, $-OR^6$, halogen, haloalkyl, perhaloalkyl and alkyl; or
$R^a$ and $R^b$ taken together form a monocyclic or a bicyclic ring system which is saturated or partially unsaturated and optionally have additional heteroatoms selected from O, N or S;
m is 0-6;
n is 0 - 3;
p is 0, 1 or 2; and
q is 1 or 2.

The invention provides a compound selected from the group consisting of 8-cyclopropyl-6-fluoro-4-[5-fluoro-2-(hydroxymethyl)-3-[2-[5-[[4-(oxetan-3-yl)piperazin-1-yl]methyl]-2-pyridyl]-1H-pyrrolo[2,3-b]pyridin-4-yl]phenyl]-2,3-dihydro-1,4-benzoxazepin-5-one;
8-cyclopropyl-6-fluoro-4-[5-fluoro-2-(hydroxymethyl)-3-[2-[5-[[4-(2,2,2-trifluoroethyl)piperazin-1-yl]methyl]-2-pyridyl]-1H-pyrrolo[2,3-b]pyridin-4-yl]phenyl]-2,3-dihydro-1,4-benzoxazepin-5-one;
8-cyclopropyl-4-[3-[2-[5-[[4-(2,2-difluoroethyl)piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-6-fluoro-4-[5-fluoro-2-(hydroxymethyl)-3-[2-[5-(morpholinomethyl)-2-pyridyl]-1H-pyrrolo[2,3-b]pyridin-4-yl]phenyl]-2,3-dihydro-1,4-benzoxazepin-5-one;
8-cyclopropyl-4-[3-[2-[5-[[(3S)-4-(2,2-difluoroethyl)-3-methyl-piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-4-[3-[2-[5-[[(2S)-4-(2,2-difluoroethyl)-2-methyl-piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-4-[3-[2-[5-[[(3R)-4-(2,2-difluoroethyl)-3-methyl-piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-6-fluoro-4-[5-fluoro-2-(hydroxymethyl)-3-[2-[1-(oxetan-3-yl)-3,6-dihydro-2H-pyridin-4-yl]-1H-pyrrolo[2,3-

b]pyridin-4-yl]phenyl]-2,3-dihydro-1,4-benzoxazepin-5-one;
8-cyclopropyl-4-[5-fluoro-2-(hydroxymethyl)-3-[2-[1-(oxetan-3-yl)-3,6-dihydro-2H-pyridm-4-yl]-1H-pyrrolo[2,3-b]pyridin-4-yl]phenyl]-2,3-dihydro-1,4-benzoxazepin-5-one;
8-cyclopropyl-4-[5-fluoro-2-(hydroxymethyl)-3-[2-[1-(2-hydroxy-2-methyl-propyl)-3,6-dihydro-2H-pyridin-4-yl]-1H-pyrrolo[2,3-b]pyridin-4-yl]phenyl]-2,3-dihydro-1,4-benzoxazepin-5-one and
8-cyclopropyl-6-fluoro-4-[5-fluoro-2-(hydroxymethyl)-3-[2-[5-[[1-(oxetan-3-yl)-4-piperidyl]oxy]-2-pyridyl]-1H-pyrrolo[2,3-b]pyridin-4-yl]phenyl]-2,3-dihydro-1,4-benzoxazepin-5-one;
6-fluoro-4-[5-fluoro-2-(hydroxymethyl)-3-[2-[5-[[4-(oxetan-3-yl)piperazin-1-yl]methyl]-2-pyridyl]-1H-pyrrolo[2,3-b]pyridin-4-yl]phenyl]-8-(1-hydroxy-1-methyl-ethyl)-2,3-dihydro-1,4-benzoxazepin-5-one;
6-fluoro-4-[5-fluoro-2-(hydroxymethyl)-3-[2-[5-[[4-(2,2,2-trifluoroethyl)piperazin-1-yl]methyl]-2-pyridyl]-1H-pyrrolo[2,3-b]pyridin-4-yl]phenyl]-8-(1-hydroxy-1-methylethyl)-2,3-dihydro-1,4-benzoxazepin-5-one;
4-[3-[2-[5-[[4-(2,2-difluoroethyl)piperazin-1-yl]methyl]-2-pyridyl]-1H-pyrrolo[2,3-b]pyridin-4-yl]-5-fluoro-2-(hydroxymethyl)phenyl]-6-fluoro-8-(1-hydroxy-1-methylethyl)-2,3-dihydro-1,4-benzoxazepin-5-one;
4-[3-[2-[5-[[4-(2,2-difluoroethyl)piperazin-1-yl]methyl]-2-pyridyl]-1H-pyrrolo[2,3-b]pyridin-4-yl]-5-fluoro-2-(hydroxymethyl)phenyl]-6-fluoro-8-(1-fluorocyclopropyl)-2,3-dihydro-1,4-benzoxazepin-5-one;
6-fluoro-8-(1-fluorocyclopropyl)-4-[5-fluoro-2-(hydroxymethyl)-3-[2-[5-[[4-(2,2,2-trifluoroethyl)piperazin-1-yl]methyl]-2-pyridyl]-1H-pyrrolo[2,3-b]pyridin-4-yl]phenyl]-2,3-dihydro-1,4-benzoxazepin-5-one;
6-fluoro-8-(1-fluorocyclopropyl)-4-[5-fluoro-2-(hydroxymethyl)-3-[2-[5-[[4-(oxetan-3-yl)piperazin-1-yl]methyl]-2-pyridyl]-1H-pyrrolo[2,3-b]pyridin-4-yl]phenyl]-2,3-dihydro-1,4-benzoxazepin-5-one;
8-cyclopropyl-6-fluoro-4-[5-fluoro-2-(hydroxymethyl)-3-[6-[5-[[4-(oxetan-3-yl)piperazin-1-yl]methyl]-2-pyridyl]-7H-pyrrolo[2,3-d]pyrimidin-4-yl]phenyl]-2,3-dihydro-1,4-benzoxazepin-5-one;
8-cyclopropyl-6-fluoro-4-[5-fluoro-2-(hydroxymethyl)-3-[6-[5-[[4-(2,2,2-trifluoroethyl)piperazin-1-yl]methyl]-2-pyridyl]-7H-pyrrolo[2,3-d]pyrimidin-4-yl]phenyl]-2,3-dihydro-1,4-benzoxazepin-5-one;
8-cyclopropyl-4-[3-[6-[5-[[4-(2,2-difluoroethyl)piperazin-1-yl]methyl]-2-pyridyl]-7H-pyrrolo[2,3-d]pyrimidin-4-yl]-5-fluoro-2-(hydroxymethyl)phenyl]-6-fluoro-2,3-dihydro-1,4-benzoxazepin-5-one;
8-cyclopropyl-6-fluoro-4-[5-fluoro-2-(hydroxymethyl)-3-[6-[5-[(3-hydroxy-3-methyl-azetidin-1-yl)methyl]-2-pyridyl]-7H-pyrrolo[2,3-d]pyrimidin-4-yl]phenyl]-2,3-dihydro-1,4-benzoxazepin-5-one;
8-cyclopropyl-6-fluoro-4-[5-fluoro-2-(hydroxymethyl)-3-[2-[5-[(3-hydroxy-3-methyl-azetidin-1-yl)methyl]-2-pyridyl]-1H-pyrrolo[2,3-b]pyridin-4-yl]phenyl]-2,3-dihydro-1,4-benzoxazepin-5-one;
8-cyclopropyl-6-fluoro-4-[5-fluoro-2-(hydroxymethyl)-3-[2-[5-[(4-hydroxy-4-methyl-1-piperidyl)methyl]-2-pyridyl]-1H-pyrrolo[2,3-b]pyridin-4-yl]phenyl]-2,3-dihydro-1,4-benzoxazepin-5-one;
8-cyclopropyl-6-fluoro-4-[5-fluoro-2-(hydroxymethyl)-3-[6-[5-[(4-hydroxy-4-methyl-1-piperidyl)methyl]-2-pyridyl]-7H-pyrrolo[2,3-d]pyrimidin-4-yl]phenyl]-2,3-dihydro-1,4-benzoxazepin-5-one;
8-cyclopropyl-6-fluoro-4-[5-fluoro-2-(hydroxymethyl)-3-[6-[5-(morpholinomethyl)-2-pyridyl]-7H-pyrrolo[2,3-d]pyrimidin-4-yl]phenyl]-2,3-dihydro-1,4-benzoxazepin-5-one;
8-cyclopropyl-6-fluoro-4-[5-fluoro-2-(hydroxymethyl)-3-[2-[5-(morpholinomethyl)-2-pyridyl]-1H-pyrrolo[2,3-b]pyridin-4-yl]phenyl]-2,3-dihydro-1,4-benzoxazepin-5-one;
8-cyclopropyl-4-[3-[2-[5-[[3,5-dimethyl-4-(2,2,2-trifluoroethyl)piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-4-[3-[2-[5-[[3,5-dimethyl-4-(oxetan-3-yl)piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-4-[3-[2-[5-[[4-(2,2-difluoroethyl)-3,5-dimethyl-piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-4-[3-[2-[5-[[4-(2,2-difluoroethyl)-3-methyl-piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-6-fluoro-4-[5-fluoro-2-(hydroxymethyl)-3-[2-[5-[[3-methyl-4-(2,2,2-trifluoroethyl)piperazin-1-yl]methyl]-2-pyridyl]-1H-pyrrolo[2,3-b]pyridin-4-yl]phenyl]-2,3-dihydro-1,4-benzoxazepin-5-one;
8-cyclopropyl-6-fluoro-4-[5-fluoro-2-(hydroxymethyl)-3-[2-[5-[[3-methyl-4-(oxetan-3-yl)piperazin-1-yl]methyl]-2-pyridyl]-1H-pyrrolo[2,3-b]pyridin-4-yl]phenyl]-2,3-dihydro-1,4-benzoxazepin-5-one;
8-cyclopropyl-6-fluoro-4-[5-fluoro-2-(hydroxymethyl)-3-[2-[5-[1 -methyl-1 -[4-(oxetan-3-yl)piperazin-1-yl]ethyl]-2-pyridyl]-1H-pyrrolo[2,3-b]pyridin-4-yl]phenyl]-2,3-dihydro-1,4-benzoxazepin-5-one;
8-cyclopropyl-6-fluoro-4-[5-fluoro-2-(hydroxymethyl)-3-[2-[5-[1-methyl-1-[4-(2,2,2-trifluoroethyl)piperazin-1-yl]ethyl]-2-pyridyl]-1H-pyrrolo[2,3-b]pyridin-4-yl]phenyl]-2,3 - dihydro-1,4-benzoxazepin-5-one;
8-cyclopropyl-4-[3-[2-[5-[1-[4-(2,2-difluoroethyl)piperazin-1-yl]-1-methyl-ethyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-4-[3-[2-[5-[[4-(2,2-difluoroethyl)piperazin-1-yl]methyl]-4-fluoro-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-6-fluoro-4-[5-fluoro-3-[2-[4-fluoro-5-[[4-(2,2,2-trifluoroethyl)piperazin-1-yl]methyl]-2-pyridyl]-1H-pyrrolo[2,3-b]pyridin-4-yl]-2-(hydroxymethyl)phenyl]-2,3-dihydro-1,4-benzoxazepin-5-one;
8-cyclopropyl-6-fluoro-4-[5-fluoro-3-[2-[4-fluoro-5-[[4-(oxetan-3-yl)piperazin-1-yl]methyl]-2-pyridyl]-1H-pyrrolo[2,3-b]pyridin-4-yl]-2-(hydroxymethyl)phenyl]-2,3-dihydro-1,4-benzoxazepin-5-one;
8-cyclopropyl-6-fluoro-4-[5-fluoro-2-(hydroxymethyl)-3-[2-[5-[[4-(oxetan-3-ylmethyl)piperazin-1-yl]methyl]-2-pyridyl]-1H-pyrrolo[2,3-b]pyridin-4-yl]phenyl]-2,3-dihydro-1,4-benzoxazepin-5-one;
8-cyclopropyl-6-fluoro-4-[5-fluoro-2-(hydroxymethyl)-3-[6-[5-[[4-(oxetan-3-ylmethyl)piperazin-1-yl]methyl]-2-pyridyl]-7H-pyrrolo[2,3-d]pyrimidin-4-yl]phenyl]-2,3-dihydro-1,4-benzoxazepin-5-one
4-[4-[3-(8-cyclopropyl-6-fluoro-5-oxo-2,3-dihydropyrido[3,2-f][1,4]oxazepin-4-yl)-5-fluoro-2-(hydroxymethyl)phenyl]-7H-pyrrolo[2,3-d]pyrimidin-6-yl]-N,N-dimethyl-3,6-dihydro-2H-pyridine-1-carboxamide;
8-cyclopropyl-4-[3-[2-[5-[(2,4-dimethylpiperazin-1-yl)methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-4-[3-[2-[5-[1-(3,4-dimethylpiperazin-1-yl)ethyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
4-[3-[2-[1-(azetidine-1-carbonyl)-3,6-dihydro-2H-pyridin-4-yl]-1H-pyrrolo[2,3-b]pyridin-4-yl]-5-fluoro-2-(hydroxymethyl)phenyl]-8-cyclopropyl-6-fluoro-2,3-dihydro-1,4-benzoxazepin-5-one and
3-[4-[4-[3-(8-cyclopropyl-6-fluoro-5-oxo-2,3-dihydro-1,4-benzoxazepin-4-yl)-5-fluoro-2-(hydroxymethyl)phenyl]-1H-pyrrolo[2,3-b]pyridin-2-yl]-3,6-dihydro-2H-pyridin-1-yl]-3-oxo-propanenitrile;
or its tautomers, polymorphs, stereoisomers, solvates, co-crystals or a pharmaceutically acceptable salt thereof.

## Detailed description

### Definitions

In the structural formulae given herein and throughout the present disclosure, the following terms have the indicated meaning, unless specifically stated otherwise.

The term "optionally substituted" as used herein means that the group in question is either unsubstituted or substituted with one or more of the substituents specified. When the group in question is substituted with more than one substituent, the substituent may be same or different.

The term "alkyl" refers to a monoradical branched or unbranched saturated hydrocarbon chain having 1, 2, 3, 4, 5, 6, 7, 8, 9, 10, 11, 12, 13, 14, 15, 16, 17, 18, 19 or 20 carbon atoms, preferably 1, 2, 3, 4, 5, 6, 7, 8, 9 or 10 carbon atoms, more preferably 1, 2, 3, 4, 5 or 6 carbon atoms. This term is exemplified by groups such as methyl, ethyl, n-propyl, isopropyl, n-butyl, iso-butyl, t-butyl, n-hexyl, n-decyl, tetradecyl, and the like.

The term "alkylene" refers to a diradical of a branched or unbranched saturated hydrocarbon chain, having 1, 2, 3, 4, 5, 6, 7, 8, 9, 10, 11,12, 13, 14, 15, 16, 17, 18, 19 or 20 carbon atoms, preferably 1, 2, 3, 4, 5, 6, 7, 8, 9 or 10 carbon atoms, more preferably 1, 2, 3, 4, 5 or 6 carbon atoms. This term is exemplified by groups such as methylene ($-CH_2-$), ethylene ($-CH_2CH_2-$), the propylene isomers (e.g., $-CH_2CH_2CH_2-$ and $-CH(CH_3)CH_2-$) and the like. The term "substituted alkyl" or "substituted alkylene" refers to: 1) an alkyl group or alkylene group as defined above, having 1, 2, 3, 4 or 5 substituents, preferably 1, 2 or 3 substituents, selected from the group consisting of alkenyl, alkynyl, alkoxy, cycloalkyl, cycloalkenyl, acyl, acylamino, acyloxy, amino, monoalkylamino, dialkylamino, arylamino, heteroarylamino, aminocarbonyl, alkoxycarbonylamino, azido, cyano, halogen, hydroxy, hydroxyalkyl, keto, thiocarbonyl, carboxy, carboxyalkyl, $-SO_3H$, aryl, aryloxy, heteroaryl, aminocarbonylamino, heteroaryloxy, heterocyclyl, heterocyclyloxy, hydroxyamino, alkoxyamino, nitro, $-S(O)_2NR^aR^a$, $-NR^aS(O)_2R^a$ and $-S(O)_pR^b$, where each $R^a$ is independently selected from the group consisting of hydrogen, alkyl, cycloalkyl, cycloalkylalkyl, aryl, arylalkyl, heteroaryl heteroarylalkyl, heterocyclyl and heterocyclylalkyl; heterocyclyloxy where $R^b$ is hydrogen, alkyl, aryl, heteroaryl or heterocyclyl. Unless otherwise constrained by the definition, all substituents may optionally be further substituted by 1, 2, or 3 substituents selected from alkyl, carboxy, carboxyalkyl, aminocarbonyl, hydroxy, alkoxy, halogen, $CF_3$, amino, substituted amino, cyano, and $-S(O)_pR^c$, where $R^c$ is alkyl, aryl, or heteroaryl and p is 0,1 or 2;
or 2) an alkyl group or alkylene group as defined above that is interrupted by 1, 2, 3, 4, 5, 6, 7, 8, 9 or 10 atoms independently selected from oxygen, sulphur and $NR^d$, where $R^d$ is selected from hydrogen, alkyl, cycloalkyl, cycloalkenyl, aryl, heteroaryl and heterocyclyl, carbonylalkyl, carboxyester, carboxyamide and sulfonyl. All substituents may be optionally further substituted by alkyl, alkoxy, halogen, $CF_3$, amino, substituted amino, cyano, or $-S(O)_pR^c$, in which $R^c$ is alkyl, aryl, or heteroaryl and p is 0, 1, or 2;

or 3) an alkyl or alkylene as defined above that has 1, 2, 3, 4 or 5 substituents as defined above, as well as interrupted by 1, 2, 3, 4, 5, 6, 7, 8, 9 or 10 atoms as defined above.

The term "alkenyl" refers to a monoradical of a branched or unbranched unsaturated hydrocarbon group preferably having from 2, 3, 4, 5, 6, 7, 8, 9, 10, 11, 12, 13, 14, 15, 16, 17, 18, 19 or 20 carbon atoms, more preferably 2, 3, 4, 5, 6, 7, 8, 9 or 10 carbon atoms and even more preferably 2, 3, 4, 5 or 6 carbon atoms and having 1, 2, 3, 4, 5 or 6

double bond (vinyl), preferably 1 double bond. Preferred alkenyl groups include ethenyl or vinyl(-$CH=CH_2$), 1-propylene or allyl (-$CH_2CH=CH_2$), isopropylene (-$C(CH_3)=CH_2$), bicyclo [2.2. 1] heptene, and the like.

The term "alkenylene" refers to a diradical of a branched or unbranched unsaturated hydrocarbon group preferably having from 2, 3, 4, 5, 6, 7, 8, 9, 10, 11, 12, 13, 14, 15, 16, 17, 18, 19 or 20 carbon atoms, more preferably 2, 3, 4, 5, 6, 7, 8, 9 or 10 carbon atoms and even more preferably 2, 3, 4, 5 or 6 carbon atoms and having 1, 3, 4, 5 or 6 double bond (vinyl), preferably 1 double bond.

The term "substituted alkenyl" refers to an alkenyl group as defined above having 1, 2, 3, 4 or 5 substituents, and preferably 1, 2, or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, thiocarbonyl, carboxy, carboxyalkyl, $SO_3H$, aryl, aryloxy, heteroaryl, aminocarbonylamino, heteroaryloxy, heterocyclyl, heterocyclyloxy, hydroxyamino, alkoxyamino, nitro, -$S(O)_2NR^aR^a$, - $NR^aS(O)_2R^a$ and -$S(O)_pR^b$ where each $R^a$ is independently selected from the group consisting of hydrogen, alkyl, cycloalkyl, cycloalkylalkyl, aryl, arylalkyl, heteroaryl heteroarylalkyl, heterocyclyl, heterocyclylalkyl and heterocyclyloxy, where $R^b$ is alkyl, aryl, heteroaryl or heterocyclyl and p is 0, 1 or 2. Unless otherwise constrained by the definition, all substituents may optionally be further substituted by 1, 2, or 3 substituents selected from alkyl, carboxy, carboxyalkyl, aminocarbonyl, hydroxy, alkoxy, halogen, $CF_3$, amino, substituted amino, cyano, and -$S(O)_pR^c$, where $R^c$ is alkyl, aryl, or heteroaryl and p is 0, 1 or 2.

The term "alkynyl" refers to a monoradical of an unsaturated hydrocarbon, preferably having from 2, 3, 4, 5, 6, 7, 8, 9, 10, 11, 12, 13, 14, 15, 16, 17, 18, 19 or 20 carbon atoms, more preferably 2, 3, 4, 5, 6, 7, 8, 9 or 10 carbon atoms and even more preferably 2, 3, 4, 5 or 6 carbon atoms and having 1, 2, 3, 4, 5 or 6 sites of acetylene (triple bond) unsaturation, preferably 1 triple bond. Preferred alkynyl groups include ethynyl, (-$C\equiv CH$), propargyl (or prop-1-yn-3-yl,-$CH_2C\equiv CH$), homopropargyl (or but-1-yn-4-yl, -$CH_2CH_2C\equiv CH$) and the like.

The term "alkynylene" refers to a diradical of a branched or an unbranched unsaturated hydrocarbon group preferably having from 2, 3, 4, 5, 6, 7, 8, 9, 10, 11, 12, 13, 14, 15, 16, 17, 18, 19 or 20 carbon atoms, more preferably 2, 3, 4, 5, 6, 7, 8, 9 or 10 carbon atoms and even more preferably 2, 3, 4, 5 or 6 carbon atoms and having 1, 3, 4, 5 or 6 sites of acetylene (triple bond) unsaturation, preferably 1 triple bond.

The term "substituted alkynyl" refers to an alkynyl group as defined above having 1, 2, 3, 4 or 5 substituents, and preferably 1, 2, or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, -$SO_3H$, aryl, aryloxy, heteroaryl, aminocarbonylamino, heteroaryloxy, heterocyclyl, heterocyclyloxy, hydroxyamino, alkoxyamino, nitro, - $S(O)_2NR^aR^a$, -$NR^aS(O)_2R^a$ and -$S(O)_pR^b$, where each $R^a$ is independently selected from the group consisting of hydrogen, alkyl, cycloalkyl, cycloalkylalkyl, aryl, arylalkyl, heteroaryl heteroarylalkyl, heterocyclyl, heterocyclylalkyl and heterocyclyloxy, where $R^b$ is alkyl, aryl, heteroaryl or heterocyclyl and p is 0, 1 or 2. Unless otherwise constrained by the definition, all substituents may optionally be further substituted by 1, 2, or 3 substituents selected from alkyl, carboxy, carboxyalkyl, aminocarbonyl, hydroxy, alkoxy, halogen, $CF_3$, amino, substituted amino, cyano, and-$S(O)_pR^c$ where $R^c$ is alkyl, aryl, or heteroaryl and p is 0, 1 or 2.

The term "cycloalkyl" refers to unless otherwise mentioned, carbocyclic groups of from 3 to 20 carbon atoms having a single cyclic ring or multiple condensed rings or spirocyclic rings or bridged rings which are saturated. Such cycloalkyl groups include, by way of example, single ring structures such as cyclopropyl cyclobutyl, cyclopentyl, cyclopentenyl, cyclohexyl, cyclohexenyl, cyclooctyl, and the like, or multiple ring structures such as adamantanyl, bicyclo[2.2.1]heptane, bicyclo[2.2.2]octane, 1,3,3-trimethylbicyclo[2.2.1]hept-2-yl, (2,3,3-trimethylbicyclo[2.2.1]hept-2-yl), or carbocyclic groups to which is fused an aryl group, for example indane, and the like.

The term "cycloalkylene" refers to a divalent cycloalkyl group as defined above. This term is exemplified by groups such as 1,4-cyclohexylene, 1,3-cyclohexylene, 1,2-cyclohexylene, 1,4-cyclohexenyl and the like..

The term "substituted cycloalkyl" refers to cycloalkyl groups having 1, 2, 3, 4 or 5 substituents, and preferably 1, 2, or 3 substituents, selected from the group consisting of alkyl, alkoxy, cycloalkyl, cycloalkenyl, acyl, acylamino, acyloxy, amino, aminocarbonyl, alkoxycarbonylamino, azido, cyano, halogen, hydroxy, oxo, thiocarbonyl, aryl, aryloxy, heteroaryl, aminosulfonyl, aminocarbonylamino, heteroaryloxy, heterocyclyl, heterocyclyloxy, hydroxyamino, alkoxyamino, nitro, -C(O)R and -$S(O)_pR^b$, where R is hydrogen, hydroxyl, alkoxy, alkyl and cyclocalkyl, heterocyclyloxy where $R^b$ is alkyl, aryl, heteroaryl or heterocyclyl and p is 0, 1 or 2. Unless otherwise constrained by the definition, all substituents may optionally be further substituted by 1, 2, or 3 substituents selected from alkyl, carboxy, carboxyalkyl, aminocarbonyl, hydroxy, alkoxy, halogen, $CF_3$, amino, substituted amino, cyano, and-$S(O)_pR^c$, where $R^c$ is alkyl, aryl, or heteroaryl and p is 0, 1 or 2.

"Halo" or "Halogen", alone or in combination with any other term means halogens such as chloro (Cl), fluoro (F), bromo (Br) and iodo (I).

"Haloalkyl" refers to a straight chain or branched chain haloalkyl group with 1 to 6 carbon atoms. The alkyl group may be partly or totally halogenated. Representative examples of haloalkyl groups include but are not limited to fluoromethyl, chloromethyl, bromomethyl, difluoromethyl, dichloromethyl, dibromomethyl, trifluoromethyl, trichloromethyl, 2-fluoroethyl, 2-chloroethyl, 2-bromoethyl, 2,2,2-trifluoroethyl, 3-fluoropropyl, 3-chloropropyl, 3-bromopropyl and

the like.

The term "alkoxy" refers to the group R'''-O-, where R''' is optionally substituted alkyl or optionally substituted cycloalkyl, or optionally substituted alkenyl or optionally substituted alkynyl; or optionally substituted cycloalkenyl, where alkyl, alkenyl, alkynyl, cycloalkyl and cycloalkenyl are as defined herein. Representative examples of alkoxy groups include but are not limited to methoxy, ethoxy, n-propoxy, iso-propoxy, n-butoxy, tert-butoxy, sec-butoxy, n-pentoxy, n-hexoxy, 1,2-dimethylbutoxy, trifluoromethoxy, and the like.

The term "aminocarbonyl" refers to the group -C(O)NR'R' where each R' is independently hydrogen, alkyl, aryl, heteroaryl, heterocyclyl or both R' groups are joined to form a heterocyclic group (e. g. morpholino). Unless otherwise constrained by the definition, all substituents may optionally be further substituted by 1-3 substituents selected from alkyl, carboxy, carboxyalkyl, aminocarbonyl, hydroxy, alkoxy, halogen, $CF_3$, amino, substituted amino, cyano, and $-S(O)_pR^c$, where $R^c$ is alkyl, aryl, or heteroaryl and p is 0, 1 or 2.

The term "acylamino" refers to the group -NR"C(O)R" where each R" is independently hydrogen, alkyl, aryl, heteroaryl, or heterocyclyl. Unless otherwise constrained by the definition, all substituents may optionally be further substituted by 1-3 substituents selected from alkyl, carboxy, carboxyalkyl, aminocarbonyl, hydroxy, alkoxy, halogen, $CF_3$, amino, substituted amino, cyano, and$-S(O)_pR^c$, where $R^c$ is alkyl, aryl, or heteroaryl and p is 0, 1 or 2.

The term "acyloxy" refers to the groups -OC(O)-alkyl, -OC(O)-cycloalkyl, -OC(O)-aryl, -OC(O)-heteroaryl, and -OC(O)-heterocyclyl. Unless otherwise constrained by the definition, all substituents may be optionally further substituted by alkyl, carboxy, carboxyalkyl, aminocarbonyl, hydroxy, alkoxy, halogen, $CF_3$, amino, substituted amino, cyano, or $-S(O)_pR^c$, where $R^c$ is alkyl, aryl, or heteroaryl and p is 0, 1 or 2.

"Alkoxyalkyl" refers to alkyl groups as defined above wherein at least one of the hydrogen atoms of the alkyl group is replaced by an alkoxy group as defined above. Representative examples of alkoxyalkyl groups include but are not limited to methoxymethyl, methoxyethyl, ethoxymethyl and the like.

"Aryloxyalkyl" refers to the group -alkyl-O-aryl. Representative examples of aryloxyalkyl include but are not limited to phenoxymethyl, naphthyloxymethyl, phenoxyethyl, naphthyloxyethyl and the like.

"Di alkylamino" refers to an amino group, to which two same or different straight chain or branched chain alkyl groups with 1 to 6 carbon atoms are bound. Representative examples of di alkylamino include but are not limited to dimethylamino, diethylamino, methylethylamino, dipropylamino, dibutylamino and the like.

"Cycloalkylalkyl" refers to an alkyl radical as defined above which is substituted by a cycloalkyl radical as defined above. Representative examples of cycloalkylalkyl include but are not limited to cyclopropylmethyl, cyclobutylmethyl, cyclopentylmethyl, cyclohexylmethyl, 1-cyclopentylethyl, 1-cyclohexylethyl, 2-cyclopentylethyl, 2-cyclohexylethyl, cyclobutylpropyl, cyclopentylpropyl, cyclohexylbutyl and the like.

"Aminoalkyl" refers to an amino group that is attached to $(C_{1-6})$alkylene as defined herein. Representative examples of aminoalkyl include but are not limited to aminomethyl, aminoethyl, 1-aminopropyl, 2-aminopropyl, and the like. The amino moiety of aminoalkyl may be substituted once or twice with alkyl to provide alkylaminoalkyl and dialkylaminoalkyl respectively. Representative examples of alkylaminoalkyl include but are not limited to methylaminomethyl, methylaminoethyl, methylaminopropyl, ethylaminoethyl and the like. Representative examples of dialkylaminoalkyl include but are not limited to dimethylaminomethyl, dimethylaminoethyl, dimethylaminopropyl, N-methyl-N-ethylaminoethyl and the like.

The term "aryl" refers to an aromatic carbocyclic group of 6 to 20 carbon atoms having a single ring (e.g. phenyl) or multiple rings (e.g. biphenyl), or multiple condensed (fused) rings (e.g. naphthyl or anthranyl). Preferred aryls include phenyl, naphthyl and the like.

The term "arylene" refers to a diradical of an aryl group as defined above. This term is exemplified by groups such as 1,4-phenylene, 1,3-phenylene, 1,2-phenylene, 1,4'-biphenylene, and the like.

Unless otherwise constrained the aryl or arylene groups may optionally be substituted with 1, 2, 3, 4 or 5 substituents, preferably 1, 2 or 3 substituents, selected from the group consisting of alkyl, alkoxy, cycloalkyl, cycloalkenyl, acyl, acylamino, acyloxy, amino, aminocarbonyl, alkoxycarbonylamino, azido, cyano, halogen, hydroxy, carboxy, carboxyalkyl, $-SO_3H$, aryl, aryloxy, heteroaryl, aminosulfonyl, aminocarbonylamino, heteroaryloxy, heterocyclyl, heterocyclyloxy, hydroxyamino, alkoxyamino, nitro, $-S(O)_2NR^aR^a$, $-NR^aS(O)_2R^a$ and $-S(O)_pR^b$ where each $R^a$ is independently selected from the group consisting of hydrogen, alkyl, cycloalkyl, cycloalkylalkyl, aryl, arylalkyl, heteroaryl, heteroarylalkyl, heterocyclyl and heterocyclylalkyl; where $R^b$ is hydrogen, alkyl, aryl, heterocyclyl or heteroaryl and p is 0, 1 or 2. Unless otherwise constrained by the definition, all substituents may optionally be further substituted by 1, 2 or 3 substituents selected from alkyl, carboxy, carboxyalkyl, aminocarbonyl, hydroxy, alkoxy, halogen, $CF_3$, amino, substituted amino, cyano, and $-S(O)_pR^c$ where $R^c$ is hydrogen, alkyl, aryl, or heteroaryl and p is 0, 1 or 2.

The term "arylalkyl" refers to an aryl group covalently linked to an alkylene group, where aryl and alkylene are defined herein.

"Optionally substituted arylalkyl" refers to an optionally substituted aryl group covalently linked to an optionally substituted alkylene group. Such arylalkyl groups are exemplified by benzyl, phenethyl, naphthylmethyl, and the like.

The term "aryloxy" refers to the group aryl-O- wherein the aryl group is as defined above, and includes optionally

substituted aryl groups as also defined above.

The term "arylthio" refers to the group -S-aryl, where aryl is as defined herein including optionally substituted aryl groups as also defined above.

The term "substituted amino" refers to the group -NR'R' where each R' is independently selected from the group consisting of hydrogen, alkyl, cycloalkyl, carboxyalkyl, alkoxycarbonyl, aryl, heteroaryl and heterocyclyl. Unless otherwise constrained by the definition, all substituents may optionally be further substituted by 1, 2 or 3 substituents selected from alkyl, carboxy, carboxyalkyl, aminocarbonyl, hydroxy, alkoxy, halogen, $CF_3$, amino, substituted amino, cyano, and $-S(O)_pR^c$, where $R^c$ is alkyl, aryl, or heteroaryl and p is 0, 1 or 2.

The term "carboxyalkyl" refers to the groups -alkylene-C(O)OH.

The term "alkylcarboxyalkyl" refers to the groups -alkylene-C(O)OR$^d$ where $R^d$ is alkyl, cycloalkyl, where alkyl, cycloalkyl are as defined herein, and may be optionally further substituted by alkyl, halogen, $CF_3$, amino, substituted amino, cyano, or $-S(O)_pR^c$, in which $R^c$ is alkyl, aryl, or heteroaryl and p is 0, 1 or 2.

The term "heteroaryl" refers to an aromatic cyclic group having 1, 2, 3, 4, 5, 6, 7, 8, 9, 10, 11, 12, 13, 14, or 15 carbon atoms and 1, 2, 3 or 4 heteroatoms selected from oxygen, nitrogen and sulphur within at least one ring. Such heteroaryl groups can have a single ring (e.g. pyridyl or furyl) or multiple condensed rings (e.g. indolizinyl, benzothiazolyl, or benzothienyl). Examples of heteroaryls include, but are not limited to, [1,2,4] oxadiazole, [1,3,4] oxadiazole, [1,2,4] thiadiazole, [1,3,4] thiadiazole, pyrrole, imidazole, pyrazole, pyridine, pyrazine, pyrimidine, pyridazine, indolizine, isoindole, indole, indazole, purine, quinolizine, isoquinoline, quinoline, phthalazine, quinoxaline, quinazoline, cinnoline, pteridine, carbazole, carboline, phenanthridine, acridine, phenanthroline, isothiazole, phenazine, isoxazole, phenoxazine, phenothiazine, furan, thiophene, oxazole, thiazole, triazole, triazine and the like.

The term "heteroarylene" refers to a diradical of a heteroaryl group as defined above.

Unless otherwise constrained the heteroaryl or heterarylene groups can be optionally substituted with 1, 2, 3, 4 or 5 substituents, preferably 1, 2 or 3 substituents selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, thiocarbonyl, carboxy, carboxyalkyl, $-SO_3H$, aryl, aryloxy, heteroaryl, aminocarbonylamino, heteroaryloxy, heterocyclyl, heterocyclyloxy, hydroxyamino, alkoxyamino, nitro, - $S(O)_2NR^aR^a$, $-NR^aS(O)_2R^a$ and $-S(O)_pR^b$, where each $R^a$ is independently selected from the group consisting of hydrogen, alkyl, cycloalkyl, cycloalkylalkyl, aryl, arylalkyl, heteroaryl heteroarylalkyl, heterocyclyl and heterocyclylalkyl; where $R^b$ is hydrogen, alkyl, aryl, heterocyclyl or heteroaryl, and p is 0, 1 or 2. Unless otherwise constrained by the definition, all substituents may optionally be further substituted by 1-3 substituents selected from alkyl, carboxy, carboxyalkyl, aminocarbonyl, hydroxy, alkoxy, halogen, $CF_3$, amino, substituted amino, cyano, and-$S(O)_nR^c$, where $R^c$ is alkyl, aryl, or heteroaryl and n is 0,1 or 2.

The term "heteroarylalkyl" refers to a heteroaryl group covalently linked to an alkylene group, where heteroaryl and alkylene are defined herein.

"Optionally substituted heteroarylalkyl" refers to an optionally substituted heteroaryl group covalently linked to an optionally substituted alkylene group. Such heteroarylalkyl groups are exemplified by 3-pyridylmethyl, quinolin-8-ylethyl, 4-methoxythiazol-2-ylpropyl, and the like.

The term "heterocyclyl" refers to a saturated or partially unsaturated group having a single ring or multiple condensed rings or spirocyclic rings, or bridged rings unless otherwise mentioned, having from 1 to 40 carbon atoms and from 1 to 10 hetero atoms, preferably 1, 2, 3 or 4 heteroatoms, selected from nitrogen, sulphur, phosphorus, and/or oxygen within the ring. Heterocyclic groups can have a single ring or multiple condensed rings, and include tetrahydrofuranyl, morpholinyl, piperidinyl, piperazinyl, dihydropyridinyl, tetrahydroquinolinyl and the like. Unless otherwise constrained by the definition for the heterocyclic substituent, such heterocyclic groups can be optionally substituted with 1, 2, 3, 4 or 5, and preferably 1, 2 or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, oxo, -C(O)R where R is hydrogen, hydroxyl, alkoxy, alkyl and cyclocalkyl, thiocarbonyl, carboxy, carboxyalkyl, aryl, aryloxy, heteroaryl, aminosulfonyl, aminocarbonylamino, heteroaryloxy, heterocyclyl, heterocyclyloxy, hydroxyamino, alkoxyamino, nitro, and $-S(O)_pR^b$, where $R^b$ is hydrogen, alkyl, aryl, heterocyclyl or heteroaryl and p is 0, 1 or 2. Unless otherwise constrained by the definition, all substituents may optionally be further substituted by 1-3 substituents selected from alkyl, carboxy, carboxyalkyl, aminocarbonyl, hydroxy, alkoxy, halogen, $CF_3$, amino, substituted amino, cyano, and $-S(O)_nR^c$, where $R^c$ is alkyl, aryl, or heteroaryl and n is 0, 1 or 2.

The term "heterocyclylalkyl" refers to a heterocyclyl group covalently linked to an alkylene group, where heterocyclyl and alkylene are defined herein.

"Optionally substituted heterocyclylalkyl" refers to an optionally substituted heterocyclyl group covalently linked to an optionally substituted alkylene group.

The term "heteroaryloxy" refers to the group heteroaryl-O-.

The term "thiol" refers to the group -SH.

The term "substituted alkylthio" refers to the group -S-substituted alkyl.

The term "heteroarylthio" refers to the group -S-heteroaryl wherein the heteroaryl group is as defined above

including optionally substituted heteroaryl groups as also defined above.

The term "sulfoxide" refers to a group -S(O).

"Substituted sulfoxide" refers to a group -S(O)R, in which R is substituted alkyl, substituted aryl, or substituted heteroaryl, as defined herein.

The term "sulfone" refers to a group $-S(O)_2R$.

The term "substituted sulfone" refers to a group $-S(O)_2R$, in which R is alkyl, aryl, or heteroaryl.

The compounds of the present disclosure may have the ability to crystallize in more than one form, a characteristic known as polymorphism, and all such polymorphic forms ("polymorphs") are encompassed within the scope of the disclosure. Polymorphism generally can occur as a response to changes in temperature or pressure or both, and can also result from variations in the crystallization process. Polymorphs can be distinguished by various physical characteristics, and typically the x-ray diffraction patterns, solubility behaviour, and melting point of the compound are used to distinguish polymorphs.

The compounds described herein may contain one or more chiral centers and/or double bonds and therefore, may exist as stereoisomers, such as double-bond isomers (i.e., geometric isomers), regioisomers, enantiomers or diastereomers. Accordingly, the chemical structures depicted herein encompass all possible enantiomers and stereoisomers of the illustrated or identified compounds including the stereoisomerically pure form (e.g., geometrically pure, enantiomerically pure or diastereomerically pure) and enantiomeric and stereoisomeric mixtures. Enantiomeric and stereoisomeric mixtures can be resolved into their component enantiomers or stereoisomers using separation techniques or chiral synthesis techniques well known to the person skilled in the art. The compounds may also exist in several tautomeric forms including the enol form, the keto form and mixtures thereof. Accordingly, the chemical structures depicted herein encompass all possible tautomeric forms of the illustrated or identified compounds.

Compounds may exist in unsolvated forms as well as solvated forms, including hydrated forms and as N-oxides. In general, compounds may be hydrated, solvated or N-oxides. Certain compounds may exist in multiple crystalline or amorphous forms. Also contemplated within the scope of the disclosure are congeners, analogs, hydrolysis products, metabolites and precursor or prodrugs of the compound. In general, unless otherwise indicated, all physical forms are equivalent for the uses contemplated herein and are intended to be within the scope of the present disclosure.

"Promoiety" refers to a group bonded to a drug, typically to a functional group of the drug, via bond(s) that are cleavable under specified conditions of use. The bond(s) between the drug and promoiety may be cleaved by enzymatic or non-enzymatic means. Under the conditions of use, for example following administration to a patient, the bond(s) between the drug and promoiety may be cleaved to release the parent drug. The cleavage of the promoiety may proceed spontaneously, such as via a hydrolysis reaction, or it may be catalyzed or induced by another agent, such as by an enzyme, by light, by acid, or by a change of or exposure to a physical or environmental parameter, such as a change of temperature, pH, etc. The agent may be endogenous to the conditions of use, such as an enzyme present in the systemic circulation to which the prodrug is administered or the acidic conditions of the stomach or the agent may be supplied exogenously.

"Pharmaceutically acceptable salt" embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the disclosure are quaternary ammonium compounds wherein an equivalent of an anion (M-) is associated with the positive charge of the N atom. M- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. M- is preferably an anion selected from chloride, bromide, iodide, sulphate, nitrate, acetate, maleate, oxalate, succinate or trifluoroacetate. More preferably M- is chloride, bromide, trifluoroacetate or methanesulphonate.

"Co-crystal" refers to a crystalline material comprising two or more compounds at ambient temperature (20 to 25[deg.]C, preferably 20[deg.]C), of which at least two are held together by weak interaction, wherein at least one of the compounds is a co-crystal former. Weak interaction is being defined as an interaction which is neither ionic nor covalent and includes for example: hydrogen bonds, van der Waals forces, and interactions.

"Pharmaceutical composition" refers to one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present disclosure encompass any composition comprising a compound of the present disclosure and a pharmaceutically

acceptable carrier.

"Carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the therapeutic, preferably in purified form, together with a suitable amount of carrier so as to provide the form for proper administration to the patient. The formulation should suit the mode of administration.

"Drug or pharmaceutically active agent" includes a drug or pharmaceutical agent that will elicit the biological or medical response of a tissue, system, animal or human that is being sought, for instance, by a researcher or clinician.

"Combined" or "in combination" or "combination" should be understood as a functional coadministration, wherein some or all compounds may be administered separately, in different formulations, different modes of administration (for example subcutaneous, intravenous or oral) and different times of administration. The individual compounds of such combinations may be administered either sequentially in separate pharmaceutical compositions as well as simultaneously in combined pharmaceutical compositions.

"Therapeutically effective amount" is an amount of a compound of Formula (I) or a combination of two or more such compounds, which inhibits, totally or partially, the progression of the condition or alleviates, at least partially, one or more symptoms of the condition. A therapeutically effective amount can also be an amount which is prophylactically effective. The amount which is therapeutically effective will depend upon the patient's size and gender, the condition to be treated, the severity of the condition and the result sought. For a given patient, a therapeutically effective amount can be determined by methods known to those of skill in the art.

The present disclosure provides hetero-bicyclic compounds of formula (I), their tautomers, polymorphs, stereoisomers, solvates, hydrates, N-oxides, co-crystals, pharmaceutically acceptable salts, pharmaceutical compositions containing them and compounds for use in treating conditions and diseases that are mediated by Bruton's tyrosine kinase (Btk) activity, such as those mentioned herein above,

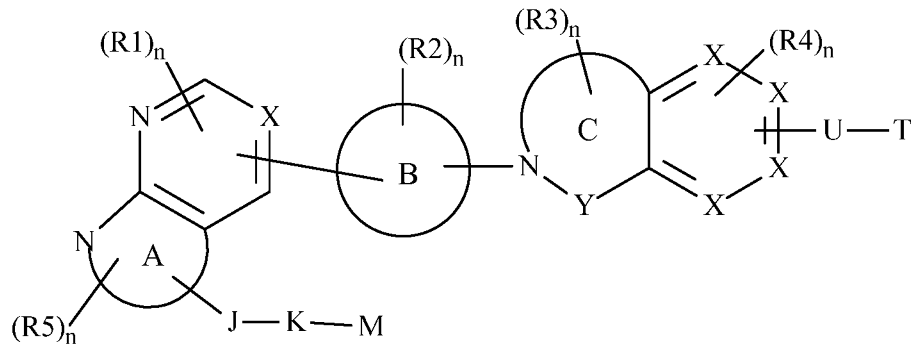

**I**

wherein,

- ring A represents a 5 membered ring which is unsaturated having upto three heteroatoms independently selected from O, N or S;
- ring B represents a 6 membered ring which is unsaturated optionally having upto three heteroatoms independently selected from O, N or S;
- ring C represents a 7 membered ring which is unsaturated or partially unsaturated optionally having upto three heteroatom independently selected from O, N or S;
- each X independently represents $C(R^1)$;
- Y represents -C(O);

J is absent or is selected from the group consisting of arylene, heterocyclylene, heteroarylene, $(C_{1-6})$alkylene, $(C_{1-6})$alkenylene or $(C_{1-6})$alkynylene;

K is a bond or $(C_{1-6})$alkylene wherein optionally one or more than one methylene groups of alkylene are independently replaced by hetero atoms or groups such as -O-, -N($R^6$)-, -C(O)-; M is selected from hydrogen, cyano, halogen, haloalkyl, perhaloalkyl, alkyl, alkenyl, alkynyl, cyanoalkyl, acyl, $-(CR^aR^b)_mOR^6$, $-SR^6$, $-(CR^aR^b)_mCOOR^6$, $-(CR^aR^b)_mNR^7R^8$, $-(CR^aR^b)_mC(O)NR^7R^8$, $-(CR^aR^b)_mNR^6C(O)NR^7R^8$, thiocarbonyl, $-S(O)_pR^6$, $-SO_3H$, cycloalkyl, aryl, arylalkyl, heterocyclyl or heteroaryl;

J, K and M is optionally substituted with one or more substituents independently selected from cyano, nitro, keto, oxo, halogen, haloalkyl, perhaloalkyl, hydroxyamino, $-(CR^aR^b)_mOR^6$, $-(CR^aR^b)_mC(O)R^6$, $-OC(O)R^6$, $-SR^6$, $-(CR^aR^b)_mCOOR^6$, $-(CR^aR^b)_mNR^7R^8$, $-(CR^aR^b)_mC(O)NR^7R^8$, $-(CR^aR^b)_mNR^6C(O)NR^7R^8$, $-NR^6C(O)R^6$, thiocarbonyl, $-S(O)_2NR^7R^8$, $-NR^6S(O)_2R^6$, $-S(O)_pR^6$, $-SO_3H$, alkyl, alkenyl, alkynyl, cycloalkyl, cyclkenyl, cycloalkylalkyl, aryl, heterocyclyl or heteroaryl;

wherein alkyl, alkenyl, alkynyl, cycloalkyl, cyclkenyl, cycloalkylalkyl, aryl, heterocyclyl or heteroaryl are optionally substituted with one or more substituents selected from hydroxy, alkyl, alkoxy, alkoxyalkyl, halogen, haloalkyl, perhaloalkyl, cyano, cyanoalkyl, amino, carboxy, carboxyalkyl, $-OC(O)R^6$, $-(CR^aR^b)_mC(O)NR^7R^8$, $-NR^6C(O)R^6$, $-SR^6$, $-S(O)_pR^6$, $-S(O)_2NR^7R^8$ or $-NR^6S(O)_2R^6$;

U is a bond or is selected from the group consisting of cycloalkylene or $(C_{1-6})$alkylene;

T is selected from hydrogen, cyano, halogen, haloalkyl, perhaloalkyl, alkyl, alkenyl, alkynyl, cyanoalkyl, acyl, $-(CR^aR^b)_mOR^6$, $-SR^6$, $-(CR^aR^b)_mCOOR^6$, $-(CR^aR^b)_mNR^7R^8$, $-(CR^aR^b)_mC(O)NR^7R^8$, $-(CR^aR^b)_mNR^6C(O)NR^7R^8$, $-S(O)_pR^6$ or $-SO_3H$;

Each of $R^1$, $R^2$, $R^3$, $R^4$ and $R^5$ are independently selected from hydrogen, alkyl, alkoxy, acyl, acylamino, acyloxy, $-(CR^aR^b)_mC(O)R^6$, $-(CR^aR^b)_mNR^7R^8$, $-(CR^aR^b)_mO(CR^aR^b)_nSi(R^7)_3$, aminocarbonyl, alkoxycarbonylamino, hydroxyamino, alkoxyamino, azido, cyano, halogen, hydroxy, hydroxyalkyl, haloalkyl, perhaloalkyl, thiocarbonyl, carboxy, alkylcarboxy, carboxyalkyl, carboxyalkyloxy, alkylcarboxyalkyloxy $-SO_3H$, alkylthio, aminosulfonyl, alkylsulfonyl, or nitro;

$R^6$ is selected from the group consisting of hydrogen, $-(CR^aR^b)_mOR^6$, halogen, haloalkyl, $-(CR^aR^b)_mC(O)R^6$, alkyl or cycloalkyl;

$R^7$ and $R^8$ are independently selected from the group consisting of hydrogen, $-(CR^aR^b)_mOR^6$, haloalkyl, $-(CR^aR^b)_mC(O)R^6$, alkyl, or

$R^7$ and $R^8$ taken together form a monocyclic or a bicyclic ring system which is saturated or partially unsaturated and optionally have additional heteroatoms selected from O, N or S;

$R^a$ and $R^b$ are independently selected from the group consisting of hydrogen, $-OR^6$, halogen, haloalkyl, perhaloalkyl and alkyl; or

$R^a$ and $R^b$ taken together form a monocyclic or a bicyclic ring system which is saturated or partially unsaturated and optionally have additional heteroatoms selected from O, N or S;

m is 0-6;

n is 0 - 3;

p is 0, 1 or 2; and

q is 1 or 2.

According to another embodiment, the present disclosure relates to compounds of formula (Ia) or its tautomers, polymorphs, stereoisomers, solvate, co-crystals or a pharmaceutically acceptable salt thereof,

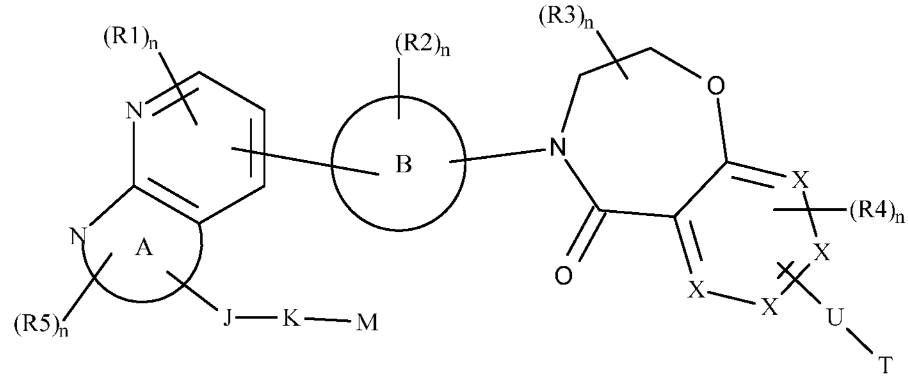

(Ia)

wherein,

ring A is selected from pyrrole, imidazole, pyrazole, oxazole, isoxazole, thiazole, isothiazole, triazole, oxadiazole, thiadiazole, dithiazole;
ring B is selected from phenyl, pyridine, pyridazine, pyrimidine or pyrazine;
each X is $C(R^1)$;
J is absent or is selected from the group consisting of $(C_{1-6})$alkylene, phenylene, pyrazolylene, imidazolylene, thienylene, furanylene, thiazolylene, oxazolylene, triazolylenene, pyridylene, pyridazinylene, pyrazinylene, dihydropyrazolylene, dihydroimidazolylene, dihydropyridylene or tetrahydropyridylene;
K is a bond or $(C_{1-6})$alkylene wherein optionally one or more than one methylene groups of alkylene are independently replaced by hetero atoms or groups such as -O-, $-N(R^6)$-, -C(O)-;
M is selected from hydrogen, cyano, halogen, haloalkyl, perhaloalkyl, alkyl, cyanoalkyl, acyl, $-(CR^aR^b)_mOR^6$, $-SR^6$, $-(CR^aR^b)_mCOOR^6$, $-(CR^aR^b)_mNR^7R^8$, $-(CR^aR^b)_mC(O)NR^7R^8$, thiocarbonyl, $-S(O)_pR^6$, $-SO_3H$, cyclopropyl, cyclohexyl, phenyl, azetidinyl, oxetanyl, pyrrolidinyl, dihydroimidazolyl, tetrahydrofuranyl, piperidinyl, piperazinyl, morpholinyl, thiomorpholinyl, thiazinyl-dioxide, tetrahydropyranyl, tetrahydropyridinyl, pyrrolyl, imidazolyl, furanyl, thiazolyl, oxazolyl, pyranyl, pyridyl or pyrimidinyl;
J, K and M is optionally substituted with one or more substituents independently selected from cyano, nitro, keto, oxo, halogen, haloalkyl, perhaloalkyl, hydroxyamino, $-(CR^aR^b)_mOR^6$, $-(CR^aR^b)_mC(O)R^6$, $-OC(O)R^6$, $-SR^6$, $-(CR^aR^b)_mCOOR^6$, $-(CR^aR^b)_mNR^7R^8$, - $(CR^aR^b)_mC(O)NR^7R^8$, $-(CR^aR^b)_mNR^6C(O)NR^7R^8$, $-NR^6C(O)R^6$, thiocarbonyl, $-S(O)_2NR^7R^8$, - $NR^6S(O)_2R^6$, $-S(O)_pR^6$, $-SO_3H$, alkyl, alkenyl or alkynyl, cycloalkyl, cyclkenyl, cycloalkylalkyl, aryl, heterocyclyl or heteroaryl;
wherein alkyl, alkenyl or alkynyl are optionally substituted with one or more substituents selected from hydroxy, alkyl, alkoxy, alkoxyalkyl, halogen, haloalkyl, perhaloalkyl, cyano, cyanoalkyl, amino, carboxy, carboxyalkyl, $-OC(O)R^6$, $-(CR^aR^b)_mC(O)NR^7R^8$, - $NR^6C(O)R^6$, $-SR^6$, $-S(O)_pR^6$, $-S(O)_2NR^7R^8$ or $-NR^6S(O)_2R^6$;
U is a bond or is selected from the group consisting of cyclopropylene, cyclobutylene, cyclohexylene or $(C_{1-6})$alkylene;
T is selected from hydrogen, cyano, halogen, haloalkyl, perhaloalkyl or alkyl $-(CR^aR^b)_mOR^6$; Each of $R^1$, $R^2$, $R^3$, $R^4$ and $R^5$ are independently selected from hydrogen, alkyl, alkoxy, acyl, acylamino, acyloxy, $-(CR^aR^b)_mNR^7R^8$, $-(CR^aR^b)_mO(CR^aR^b)_nSi(R^7)_3$, azido, cyano, halogen, hydroxy, hydroxyalkyl, haloalkyl, perhaloalkyl, carboxy, alkylcarboxy, carboxyalkyl, carboxyalkyloxy, $-SO_3H$, alkylthio, alkylsulfonyl, or nitro;
$R^6$ is selected from the group consisting of hydrogen, $-(CR^aR^b)_mOR^6$, halogen, haloalkyl, - $(CR^aR^b)_mC(O)R^6$ or alkyl, cycloalkyl;
$R^7$ and $R^8$ are independently selected from the group consisting of hydrogen, $-(CR^aR^b)_mOR^6$, haloalkyl, $-(CR^aR^b)_mC(O)R^6$, alkyl;
$R^a$ and $R^b$ are independently selected from the group consisting of hydrogen, $-OR^6$, halogen, haloalkyl, perhaloalkyl and alkyl;
m is 0-3;
n is 0 - 3;
p is 0, 1 or 2; and
q is 1 or 2.

According to another embodiment, the present disclosure relates to compounds of formula (Ia) or its tautomers, polymorphs, stereoisomers, solvate, co-crystals or a pharmaceutically acceptable salt thereof,
wherein,
ring A is pyrrole;
ring B is phenyl;
each X independently represents $C(R^1)$;
J is absent or is selected from the group consisting of $(C_{1-6})$alkylene, phenylene, pyridylene, dihydropyridylene or tetrahydropyridylene;
K is a bond or $(C_{1-6})$alkylene wherein optionally one or more than one methylene groups of alkylene are independently replaced by hetero atoms or groups such as -O-, $-N(R^6)$-, -C(O)-; M is selected from hydrogen, cyano, halogen, haloalkyl, perhaloalkyl, alkyl, cyanoalkyl, acyl, $-(CR^aR^b)_mOR^6$, $-SR^6$, $-(CR^aR^b)_mCOOR^6$, $-(CR^aR^b)_mNR^7R^8$, $-(CR^aR^b)_mC(O)NR^7R^8$, thiocarbonyl, $-S(O)_pR^6$, $-SO_3H$, cyclopropyl, cyclohexyl, phenyl, azetidinyl, oxetanyl, pyrrolidinyl, dihydroimidazolyl, tetrahydrofuranyl, piperidinyl, piperazinyl, morpholinyl, thiomorpholinyl, thiazinyl-dioxide, tetrahydropyranyl, tetrahydropyridinyl, pyrrolyl, imidazolyl, furanyl, thiazolyl, oxazolyl, pyranyl, pyridyl or pyrimidinyl;
J, K and M is optionally substituted with one or more substituents independently selected from cyano, nitro, keto, oxo, halogen, haloalkyl, perhaloalkyl, hydroxyamino, $-(CR^aR^b)_mOR^6$, $-(CR^aR^b)_mC(O)R^6$, $-OC(O)R^6$, $-SR^6$, $-(CR^aR^b)_mCOOR^6$, $-(CR^aR^b)_mNR^7R^8$, - $(CR^aR^b)_mC(O)NR^7R^8$, $-(CR^aR^b)_mNR^6C(O)NR^7R^8$, $-NR^6C(O)R^6$, thiocarbonyl, $-S(O)_2NR^7R^8$, - $NR^6S(O)_2R^6$, $-S(O)_pR^6$, $-SO_3H$, alkyl, alkenyl or alkynyl, cycloalkyl, cyclkenyl, cycloalkylalkyl, aryl, heterocyclyl or heteroaryl;
wherein alkyl, alkenyl or alkynyl are optionally substituted with one or more substituents selected from hydroxy, alkyl,

alkoxy, alkoxyalkyl, halogen, haloalkyl, perhaloalkyl, cyano, cyanoalkyl, amino, carboxy, carboxyalkyl, -OC(O)$R^6$, -$(CR^aR^b)_m$C(O)$NR^7R^8$, - $NR^6$C(O)$R^6$, -$SR^6$, -$S(O)_pR^6$, -$S(O)_2NR^7R^8$ or -$NR^6S(O)_2R^6$;

U is a bond or is selected from the group consisting of cyclopropylene, cyclobutylene, cyclohexylene or $(C_{1-6})$alkylene;

T is selected from hydrogen, cyano, halogen, haloalkyl, perhaloalkyl or alkyl -$(CR^aR^b)_mOR^6$; Each of $R^1$, $R^2$, $R^3$, $R^4$ and $R^5$ are independently selected from hydrogen, alkyl, alkoxy, acyl, acylamino, acyloxy, -$(CR^aR^b)_mNR^7R^8$, -$(CR^aR^b)_mO(CR^aR^b)_nSi(R^7)_3$, azido, cyano, halogen, hydroxy, hydroxyalkyl, haloalkyl, perhaloalkyl, carboxy, alkylcarboxy, carboxyalkyl, carboxyalkyloxy, -$SO_3H$, alkylthio, alkylsulfonyl, or nitro;

$R^6$ is selected from the group consisting of hydrogen, -$(CR^aR^b)_mOR^6$, halogen, haloalkyl, - $(CR^aR^b)_m$C(O)$R^6$ or alkyl, cycloalkyl;

$R^7$ and $R^8$ are independently selected from the group consisting of hydrogen, -$(CR^aR^b)_mOR^6$, haloalkyl, -$(CR^aR^b)_m$C(O)$R^6$, alkyl;

$R^a$ and $R^b$ are independently selected from the group consisting of hydrogen, -$OR^6$, halogen, haloalkyl, perhaloalkyl and alkyl;

m is 0-3;

n is 0 - 3;

p is 0, 1 or 2; and

q is 1 or 2.

The present disclosure also relates to the process of preparation of compounds of formula (I), or pharmaceutically acceptable salts thereof.

The compounds of formula (I) may be prepared as outlined in Schemes below:

**Scheme 1:** Preparation of compounds of formula (I)

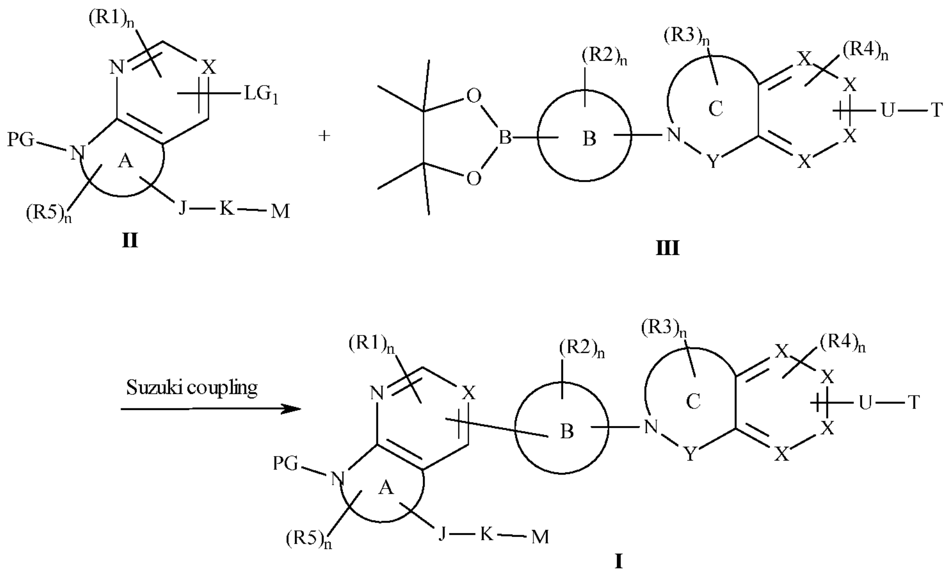


II

III

I

Compounds of formula (I) may be prepared from intermediates of formulae (II) and (III) using Suzuki coupling as shown in Scheme 1 wherein PG is H or a protecting group selected from Tosyl, SEM, MEM, and like, and $LG_1$ is leaving groups selected from halogens, triflate and the like. Deprotection of protecting group (PG) also leads to compounds of formula (I).

Intermediates of formula (II) and (III) used herein in Scheme 1 may be prepared as outlined in Schemes 2 and 3 below:

**Scheme 2:** Preparation of compounds of formula (II)

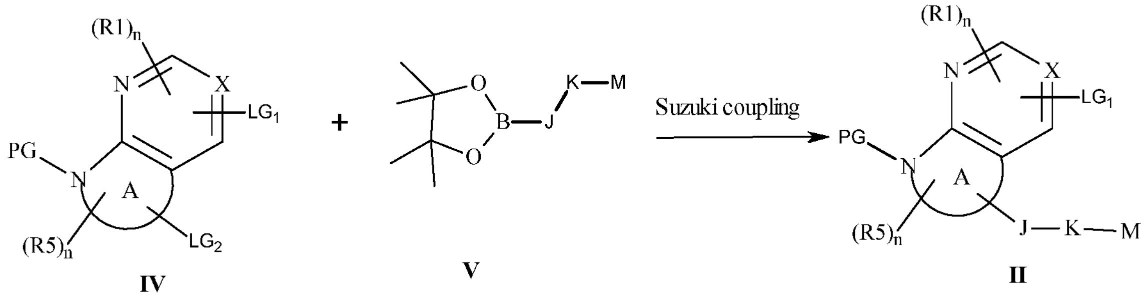


IV V II

Intermediates of general formula (II) may be prepared from reactions of intermediates of general formulae (IV) and (V) using Suzuki coupling reaction as shown in Scheme 2 wherein PG is H or a protecting group selected from Tosyl, SEM, MEM, and like and $LG_1$ and $LG_2$ are leaving groups selected from halogens, triflate and the like.

**Scheme 3:** Preparation of compounds of formula (III)

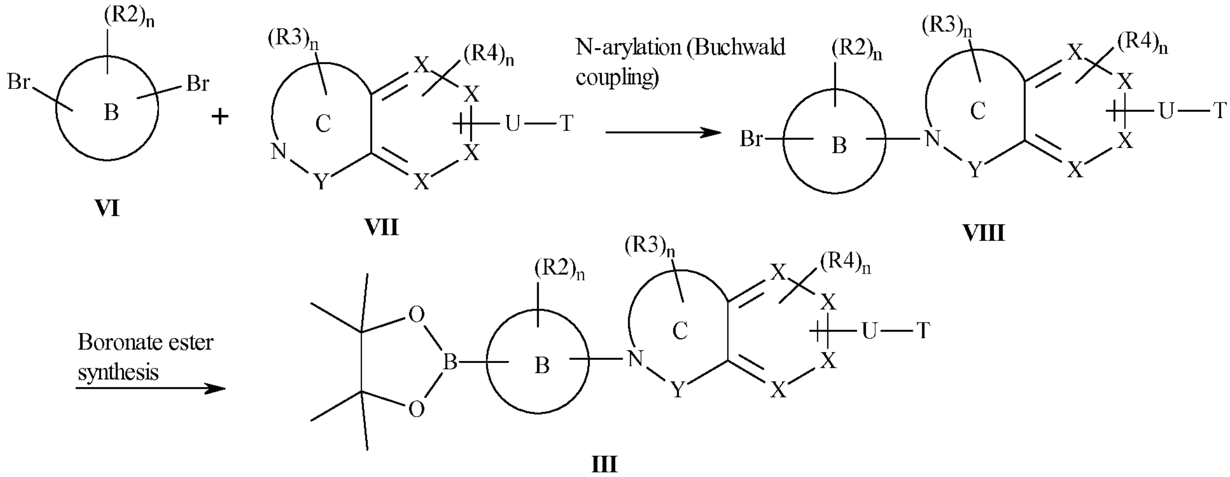


VI VII VIII

III

Intermediates of general formula (III) may be prepared from intermediates of general formulae (VI) and (VII) using N-arylation or Buchwald coupling conditions to furnish intermediate of general formula (VIII) which may be converted to the boronic esters of general formula (III) as shown in Scheme 3.

**Scheme 4:** Preparation of compounds of formula (I) wherein K is -CH2-

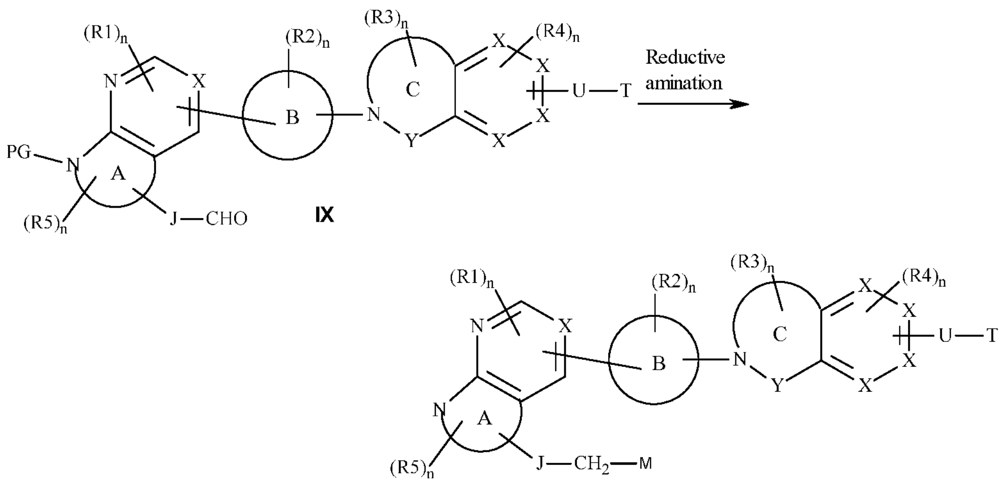


IX

I

Alternatively, compounds of formula (I) may also be prepared from intermediate of formula (IX) via reductive amination as shown in Scheme 4 wherein PG is H or a protecting group selected from Tosyl, SEM, MEM, and the like. Deprotection of protecting group (PG) also leads to compounds of formula (I).

**Scheme 5:** Preparation of compounds of formula (I) wherein K is -CO

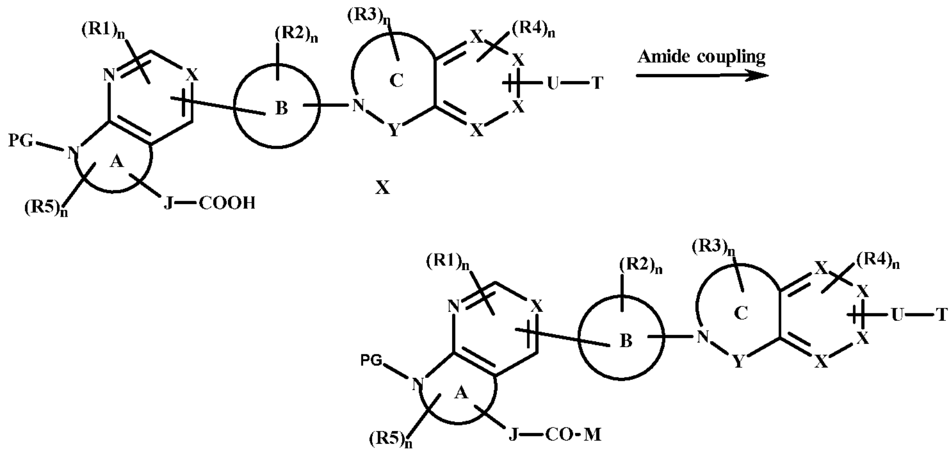


X

I

Alternatively, compounds of formula (I) may also be prepared from the corresponding intermediate of the general formula (X) using amide coupling reaction conditions as shown in Scheme 5 wherein PG is H or protecting group selected from Tosyl, SEM, MEM and the like. Deprotection of protecting group (PG) also leads to compounds of formula (I).

**Scheme 6:** Preparation of compounds of formula (IX) and (X)

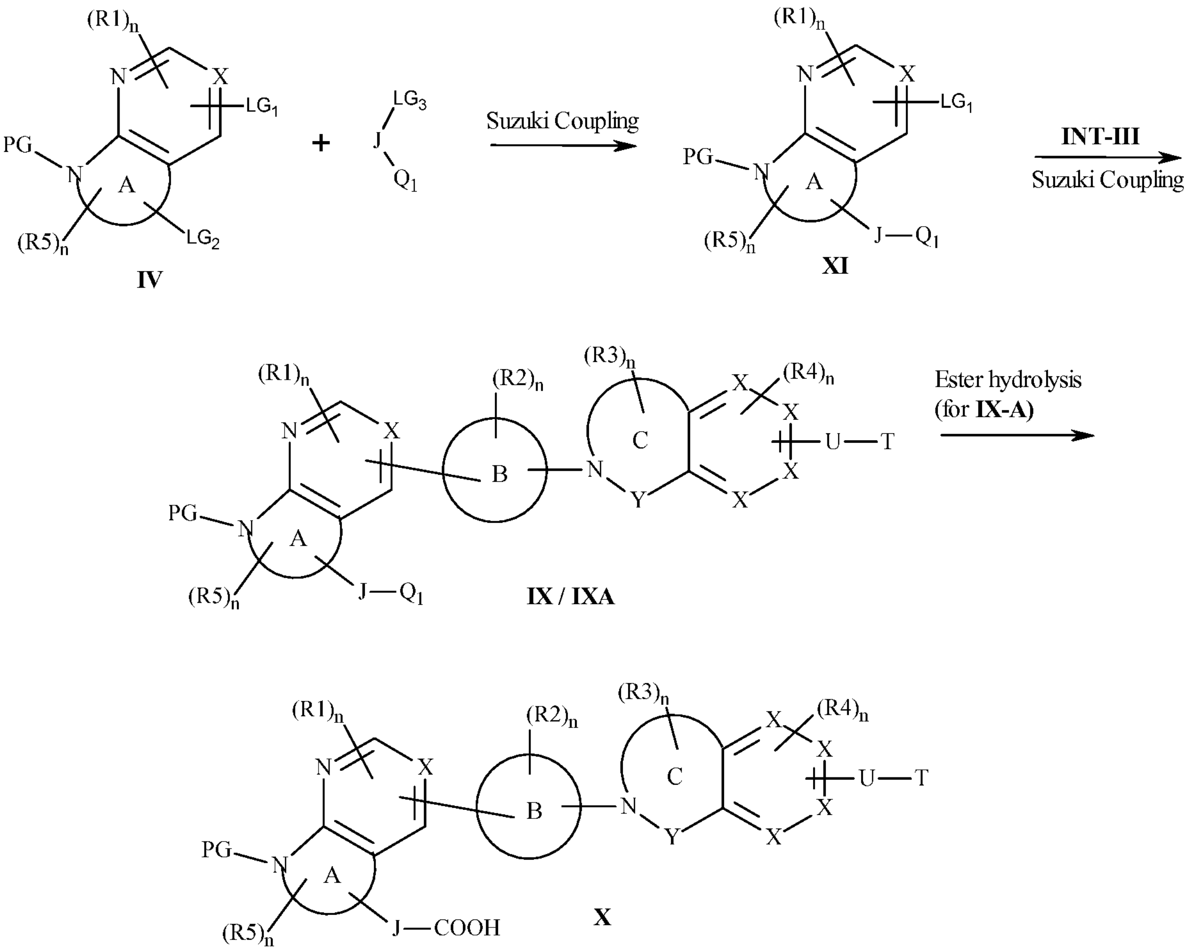


IV XI IX / IXA X

Intermediates of general formulae (IX) and (X) can be prepared from intermediates of general formulae **(XI)** and **(III)** via Suzuki coupling reaction conditions as shown in Scheme 6. Further, intermediates of general formula (XI) can be prepared from intermediates of general formula IV and $Q_1$-J-$LG_3$ *via* Suzuki coupling reaction conditions as shown in Scheme-6. In these general structures, PG is H or protecting group selected from Tosyl, SEM, MEM; $Q_1$ is -CR(O) (Compound IX) or -$CO_2$R (Compound IXA); R is H or lower alkyl group, $LG_1$, $LG_2$ and $LG_3$ are leaving groups or groups suitable for conducting Suzuki like cross coupling reaction, selected from halogens, triflate, boronic acid, boronate ester and the like.

## **Scheme 7:** Preparation of compounds of formula (II)

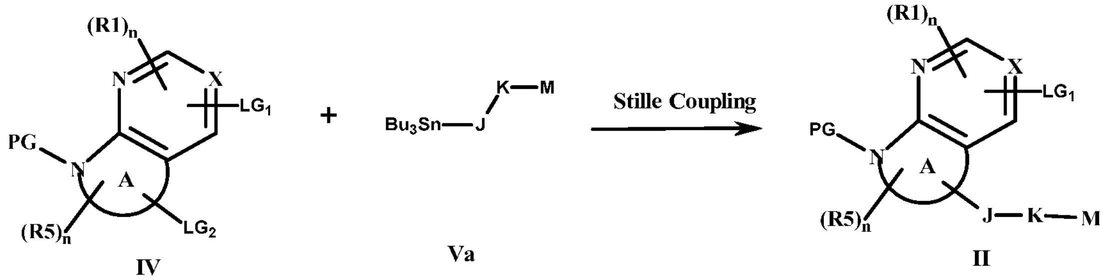


IV Va II

Intermediates of general formula (II) may also be prepared from reactions of intermediates of general formulae (IV) and (Va) using Stille coupling reaction as shown in Scheme 7 wherein PG is H or a protecting group selected from Tosyl, benzenesulfonyl, SEM, MEM and $LG_1$ and $LG_2$ are leaving groups selected from halogens, triflate and the like.

**Suzuki coupling:** Suzuki coupling is the organic reaction of an aryl- or vinyl-boronic acid with an aryl- or vinyl-halide catalyzed by a palladium(0) complex such as tetrakis(triphenylphosphine)palladium [Chemical Reviews 95 (7):

2457-2483].

**Stille coupling:** Stille coupling is the C-C bond formation reaction between an aryl- or vinyl stannanes and an aryl- or heteroaryl- or vinyl halide (or pseudo halide), which uses various Pd(0) complexes as the catalyst, *e. g.* tetrakis(triphenylphosphine)palladium [J. Org. Chem., 2009, 74, 5599-5602].

**N-arylation reaction:** N-Arylation is a chemical reaction used in organic chemistry for the formation of carbon-nitrogen bonds, which may include Ullmann coupling conditions or Buchwald-Hartwig amination reaction conditions. This can be palladium-catalyzed cross-coupling of amines or amides with aryl halides in presence of ligands like Xantphos or Cu(I) catalyzed cross-coupling of amines or amides with aryl halides in presence of ligands like *trans*-N,N'-dimethylcyclohexane-1,2-diamine. Palladium catalysts used in this reaction may include $PdCl_2$ [P (o-tolyl) 3]$_2$, Pd $(PPh_3)_4$ [Chem. Sci. 2: 27-50; Pure Appl. Chem, 71 (8): 1416-1423; J. Am. Chem. Soc. 2001, 123 (31), 7727-7729].

**Reductive Amination:** Reductive amination is a form of reaction that involves the conversion of a carbonyl group to an amine via an intermediate imine. The carbonyl group is most commonly a ketone or an aldehyde. The reaction is carried out with reducing agents that are more reactive toward protonated imines than ketones, and that are stable under moderately acidic conditions. These include sodium cyanoborohydride ($NaBH_3CN$) and sodium triacetoxyborohydride ($NaBH(OCOCH_3)_3$. [Organic Reactions, 1, 59, 2002]

**Amide Coupling:** Amide coupling may be carried out using any suitable amide coupling regents such as oxalyl chloride, thionyl chloride, BOP-Cl, DCC, HOBt, HOAt, HATU, EDCI, alkylchloroformate and the like in the presence of organic non-nucleophillic bases such as triethyl amine, di-isopropylethyl amine, pyridine, N-methyl pyrrolidine, N,N-dimethylaminopyridine, DBU, DABCO, other hindered amines and pyridines. The amide coupling reaction may be carried out in the presence of solvents such as dichloromethane, dichloroethane, DMF, dimethylacetamide, THF, acetonitrile or mixture of them may be used at a temperature ranging from -5 to 150 °C. The reaction may be carried out optionally in presence of catalytic amount of DMF. Amide coupling may also be carried out by heating ester and amine either in the absence of solvent or in presence of high boiling solvent like toluene, xylene, DMSO. Amide coupling may be carried out in presence of trialkyl aluminium (Chem. Commun., 2008, 1100-1102).

Wherever desired or necessary, in any of the above mentioned processes, any of the compounds of the invention may be converted into a pharmaceutically acceptable salt or vice versa or converting one salt form into another pharmaceutically acceptable salt form.

According to another embodiment the present invention provides co-crystals comprising a compound of the invention wherein compounds of formula (I) that contain groups capable of acting as donors and/or acceptors for hydrogen bonds may be capable of forming co-crystals with suitable co-crystal formers. These co-crystals may be prepared from compounds of the invention by known co-crystal forming procedures. Such procedures include grinding, heating, co-subliming, co-melting, or contacting in solution compounds of the invention with the co-crystal former under crystallization conditions and isolating co-crystals thereby formed.

According to another embodiment the present invention provides pharmaceutical compositions comprising a compound of the invention or a pharmaceutically acceptable salt thereof as active ingredient together with a pharmaceutically acceptable carrier, optionally in combination with one or more other pharmaceutical compositions.

In another embodiment the present invention provides a compound of the invention or a pharmaceutically acceptable salt thereof for use as a medicament.

In another embodiment the present invention provides a compound of the invention or a pharmaceutically acceptable salt thereof for use in the treatment of a disease or disorder mediated by Btk such as autoimmune disorders, inflammatory diseases, allergic diseases, airway diseases, such as asthma and chronic obstructive pulmonary disease (COPD), transplant rejection; diseases in which antibody production, antigen presentation, cytokine production or lymphoid organogenesis are abnormal or are undesirable; such as rheumatoid arthritis, systemic onset juvenile idiopathic arthritis (SOJIA), gout, pemphigus vulgaris, idiopathic thrombocytopenic purpura, systemic lupus erythematosus, multiple sclerosis, myasthenia gravis, Sjogren's syndrome, autoimmune hemolytic anemia, anti-neutrophil cytoplasmic antibodies (ANCA)-associated vasculitides, cryoglobulinemia, thrombotic thrombocytopenic purpura, chronic autoimmune urticaria, allergy (atopic dermatitis, contact dermatitis, allergic rhinitis), atherosclerosis, type 1 diabetes, type 2 diabetes, inflammatory bowel disease, ulcerative colitis, morbus Crohn, pancreatitis, glomerolunephritis, Goodpasture's syndrome, Hashimoto's thyroiditis, Grave's disease, antibody-mediated transplant rejection (AMR), graft versus host disease, B cell-mediated hyperacute, acute and chronic transplant rejection; thromboembolic disorders, myocardial infarct, angina pectoris, stroke, ischemic disorders, pulmonary embolism; cancers of haematopoietic origin such as multiple myeloma; leukemia; acute myelogenous leukemia; chronic myelogenous leukemia; lymphocytic leukemia; myeloid leukemia; non-Hodgkin lymphoma; lymphomas; polycythemia vera; chronic lymphocytic leukemia; Mantle cell lymphoma, essential thrombocythemia; myelofibrosis with myeloid metaplasia; and Waldenstroem disease.

According to another embodiment compositions can be prepared by mixing one or more compounds described herein, or pharmaceutically acceptable salts or tautomers thereof, with pharmaceutically acceptable carriers or the like, for use in treating or ameliorating a variety of Btk related conditions. The pharmaceutical compositions of the present disclosure can be manufactured by methods well known in the art such as conventional granulating, mixing, dissolving,

encapsulating, lyophilizing, emulsifying or levigating processes, among others. The compositions can be in the form of, for example, granules, powders, tablets, capsule syrup, suppositories, injections, emulsions, elixirs, suspensions or solutions. The instant compositions can be formulated for various routes of administration, for example, by oral administration, transmucosal administration, rectal administration, topical administration or subcutaneous administration as well as intrathecal, intravenous, intramuscular, intraperitoneal, intranasal, intraocular or intraventricular injection. The compound or compounds of the instant invention can also be administered in a local rather than a systemic fashion, such as injection as a sustained release formulation.

Besides those representative dosage forms described above, pharmaceutically acceptable excipients and carries are generally known to those skilled in the art and are thus included in the instant invention. Such excipients and carriers are described, for example, in "Remington's Pharmaceutical Sciences" Mack Pub. Co., New Jersey (1991).

The formulations of the invention can be designed for to be short-acting, fast-releasing, long-acting, and sustained-releasing. Thus, the pharmaceutical formulations can also be formulated for controlled release or for slow release.

The pharmaceutical compositions of the present disclosure can also comprise, for example, micelles or liposomes, or some other encapsulated form, or can be administered in an extended release form to provide a prolonged storage and/or delivery effect. Therefore, the pharmaceutical formulations can be compressed into pellets or cylinders and implanted intramuscularly or subcutaneously as depot injections or as implants such as stents. Such implants can employ known materials such as silicones and biodegradable polymers.

The pharmaceutical compositions may contain, for example, from about 0.1 % by weight, to about 90% or more by weight, of the active material, depending on the method of administration. Where the compositions comprise dosage units, each unit can contain, for example, from about 0.1 to 500 mg or more of the active ingredient. The dosage as employed for adult human treatment can range, for example, from about 0.1 to 1000 mg per day, depending on the route and frequency of administration.

Specific dosages can be adjusted depending on conditions of the BTK related condition, the age, body weight, general health conditions, sex, and diet of the subject, dose intervals, administration routes, excretion rate, and combinations of drugs. Any of the above dosage forms containing effective amounts are well within the bounds of routine experimentation and therefore, well within the scope of the instant invention. Generally, the total daily dose can typically range from about 1 mg/kg/day to about 500 mg/kg/day in single or in divided doses. Typically, dosages for humans can range from about 5 mg to about 100 mg per day, in a single or multiple doses.

A therapeutically effective dose or amount can vary depending upon the route of administration and dosage form. Some compositions of the instant invention is a formulation that exhibits a high therapeutic index. The therapeutic index is the dose ratio between toxic and therapeutic effects which can be expressed as the ratio between $LD_{50}$ and $ED_{50}$. The $LD_{50}$ is the dose lethal to 50% of the population and the $ED_{50}$ is the minimal efficatious dose to achieve 50% of the desired response. The $LD_{50}$ and $ED_{50}$ can be determined by standard pharmaceutical procedures in animal cell cultures or experimental models.

In another aspect, the present invention provides a pharmaceutical composition comprising a compound of the present invention and a pharmaceutically acceptable carrier. The pharmaceutical composition can be formulated for particular routes of administration such as oral administration, parenteral administration, and rectal administration, etc.

Compounds of the invention may be useful in the treatment of an indication selected from: Autoimmune disorders, inflammatory diseases, allergic diseases, airway diseases, such as asthma and chronic obstructive pulmonary disease (COPD), transplant rejection; diseases in which antibody production, antigen presentation, cytokine production or lymphoid organogenesis are abnormal or are undesirable; including rheumatoid arthritis, systemic onset juvenile idiopathic arthritis (SOJIA), gout, pemphigus vulgaris, idiopathic thrombocytopenic purpura, systemic lupus erythematosus, multiple sclerosis, myasthenia gravis, Sjogren's syndrome, autoimmune hemolytic anemia, anti-neutrophil cytoplasmic antibodies (ANCA)-associated vasculitides, cryoglobulinemia, thrombotic thrombocytopenic purpura, chronic autoimmune urticaria, allergy (atopic dermatitis, contact dermatitis, allergic rhinitis), atherosclerosis, type 1 diabetes, type 2 diabetes, inflammatory bowel disease, ulcerative colitis, morbus Crohn, pancreatitis, glomerolunephritis, Goodpasture's syndrome, Hashimoto's thyroiditis, Grave's disease, antibody-mediated transplant rejection (AMR), graft versus host disease, B cell-mediated hyperacute, acute and chronic transplant rejection; thromboembolic disorders, myocardial infarct, angina pectoris, stroke, ischemic disorders, pulmonary embolism; cancers of haematopoietic origin including but not limited to multiple myeloma; leukemia; acute myelogenous leukemia; chronic myelogenous leukemia; lymphocytic leukemia; myeloid leukemia; non-Hodgkin lymphoma; lymphomas; polycythemia vera; chronic lymphocytic leukemia; Mantle cell lymphoma, essential thrombocythemia; myelofibrosis with myeloid metaplasia; and Waldenstroem disease.

In another embodiment, the invention provides a compound of the invention for use in modulatiing Btk activity comprising administration of a therapeutically acceptable amount of a compound of formula (I) or a salt thereof. In a further embodiment, the disease is selected from the afore-mentioned list.

The compound of the present invention may be administered either simultaneously with, or before or after, one or more other therapeutic agent. The compound of the present invention may be administered separately, by the same or different route of administration, or together in the same pharmaceutical composition as the other agents.

According to another embodiment, compounds of the invention can be used alone or in combination with one or more additional therapeutic agent selected from the group consisting of a BTK inhibitors such as Ibrutinib, AVL-292, ONO-4059; B-cell depleting protein based therapeutics such as Rituxan, a CD20 antibody; calcineurin inhibitor such as cyclosporin A or FK 506; a mTOR inhibitor such as rapamycin, 40-O-(2-hydroxyethyl)-rapamycin, CCI779, ABT578, AP23573, AP23464, AP23675, AP23841 , TAFA-93, biolimus-7 or biolimus-9; an ascomycin having immunosuppressive properties such as ABT-281, ASM981; corticosteroids such as cortisone, dexamethasone, methylprednisolone, prednisolone, prednisone; cyclophosphamide; azathioprene; methotrexate; leflunomide; teriflunomide; mizoribine; etanercept; infliximab; a chemotherapeutic agent such as paclitaxel, gemcitabine, cisplatine, doxorubicin, 5-fluorouracil, imatinib, dasatinib, sunitinib, gefitinib, sorafenib, erlotinib, camptothecin, topotecan, daunomycin, etoposide, taxol, vinblastine, bortezomib, carfilzomib; mycophenolic acid or salt; mycophenolate mofetil; 15-deoxyspergualine or an immunosuppressive homologue, analogue or derivative thereof; NSAIDs such as Ibuprofen, flurbiprofen, naproxen, diclofenac, misoprostol, sulindac, oxaprozin, diflunisal, piroxicam, indomethacin, etodolac, fenoprofen, ketoprofen, sodium nabumetone, sulfasalazine, tolmetin sodium, hydroxychloroquine, celecoxib, valdecoxib, lumiracoxib, etoricoxib; JAK kinase inhibitors such as Tofacitinib, LY-3009104, VX-509, GLPG-0634, ASP-015K , N-benzyl-3,4-dihydroxy-benzylidene-cyanoacetamide $\alpha$-cyano-(3,4-dihydroxy)-]N-benzylcinnamamide (Tyrphostin AG 490), prodigiosin 25-C (PNU156804), [4-(4'-hydroxyphenyl)-amino-6,7-dimethoxyquinazoline] (WHI-P131), [4-(3'-bromo-4'-hydroxylphenyl)-amino-6,7-dimethoxyquinazoline] (WHI-P154), [4-(3',5'-dibromo-4'-hydroxylphenyl)-amino-6,7-dimethoxyquinazoline] WHI-P97, KRX-21 1,3-{(3R,4R)-4-methyl-3-[methyl-(7H-pyrrolo[2,3-d]pyrimidin-4-yl)-amino]-piperidin-1-yl}-3-oxo-propionitrile, in free form or in a pharmaceutically acceptable salt form, e.g. mono-citrate (also called CP-690,550); SYK inhibitor such as fostamatinib; sphingosine-1-phosphate receptor modulators such as FTY720 (fingolimod); immunosuppressive monoclonal antibodies such as monoclonal antibodies to leukocyte receptors like MHC, CD2, CD3, CD4, CD7, CD8, CD25, CD28, CD40, CD45, CD52, CD58, CD80, CD86 or their ligands; other immunomodulatory compounds such as a recombinant binding molecule having at least a portion of the extracellular domain of CTLA4 or a mutant thereof, e.g. an at least extracellular portion of CTLA4 or a mutant thereof joined to a non-CTLA4 protein sequence, e.g. CTLA4lg (for ex. designated ATCC 68629) or a mutant thereof, e.g. LEA29Y; adhesion molecule inhibitors, e.g. LFA-1 antagonists, ICAM-1 or -3 antagonists, VCAM-4 antagonists or VLA-4 antagonists; or an anti-infectious agent. Further combination partners to a compound of formula (I) may be selected from a PI3K inhibitor (e.g. pan, or alpha, beta, gamma, delta selectives), TNF inhibitors, ILI beta inhibitors, IL17 inhibitors, and inhibitors of IL6 or IL receptor.

In one embodiment, the invention provides a compound of the invention for use in treating or preventing a condition associated with BTK such as those mentioned herein above in a subject, such as a mammal, i.e., a human or non-human mammal, comprising administering an effective amount of one or more compounds described herein to the subject. Suitable non-human subjects that can be treated include domestic or wild animals, companion animals, such as dogs, cats and the like; livestock, including horses, cows and other ruminants, pigs, poultry, rabbits and the like; primates, for example monkeys, such as macaques including rhesus monkeys and cynomolgus monkeys, marmosets, tamarins and the like, apes, including chimpanzees and orangutans; and rodents, such as rats, mice, gerbils, guinea pigs and the like.

In one embodiment, the invention provides a product comprising a compound of the invention and at least one other therapeutic agent as a combined preparation for simultaneous, separate or sequential use in therapy. In one embodiment, the therapy is the treatment of a disease or condition mediated by Btk kinases such as those mentioned herein above. Products provided as a combined preparation include a composition comprising a compound of the invention and the other therapeutic agent(s) together in the same pharmaceutical composition, or a compound of the invention and the other therapeutic agent(s) in separate form, e.g. in the form of a kit.

In one embodiment, the invention provides a pharmaceutical composition comprising a compound of the invention and another therapeutic agent(s).

Optionally, the pharmaceutical composition may comprise a pharmaceutically acceptable excipient, as described above.

The invention also provides a compound of the invention for use in a method of treating a disease or condition mediated by Btk, wherein the compound of formula (I) is prepared for administration with another therapeutic agent. The invention also provides another therapeutic agent for use in a method of treating a disease or condition mediated by Btk such as those mentioned herein above, wherein the other therapeutic agent is prepared for administration with a compound of the invention . The invention also provides a compound of the invention for use in a method of treating a disease or condition mediated by Btk such as those mentioned herein above, wherein the compound of the invention is administered with another therapeutic agent. The invention also provides another therapeutic agent for use in a method of treating a disease or condition mediated by Btk such as those mentioned herein above, wherein the other therapeutic agent is administered with a compound of the invention.

## Examples

The invention is further illustrated by the following examples which in no way should be construed as being further limiting. One skilled in the art will readily appreciate that the specific methods and results described are merely illustrative. Structures of the intermediates as well as the final compounds were confirmed by nuclear magnetic resonance spectra for proton ($^1$H NMR) and LCMS.

Following intermediates were synthesized as described in the references mentioned in the following table (Table-1)

**Table-1**

| Number | Structure/IUPAC name | Reference | LCMS $(M+1)^+$ |
|---|---|---|---|
| IV-1 | 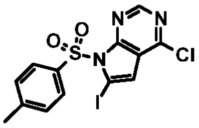 4-chloro-6-iodo-7-(p-tolylsulfonyl)pyrrolo[2,3-d]pyrimidine | WO03000695 | 434 |
| IV-2 | 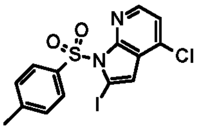 4-chloro-2-iodo-1-(p-tolylsulfonyl)pyrrolo[2,3-b]pyridine | WO2003000688 | 433 |
| IV-5 | 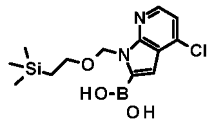 [4-chloro-1-(2-trimethylsilylethoxymethyl)pyrrolo[2,3-b]pyridin-2-yl] boronic acid | WO2010003133 | 327 |

**Intermediate VII-1: 8-cyclopropyl-3,4-dihydro-2H-1,4-benzoxazepin-5-one**

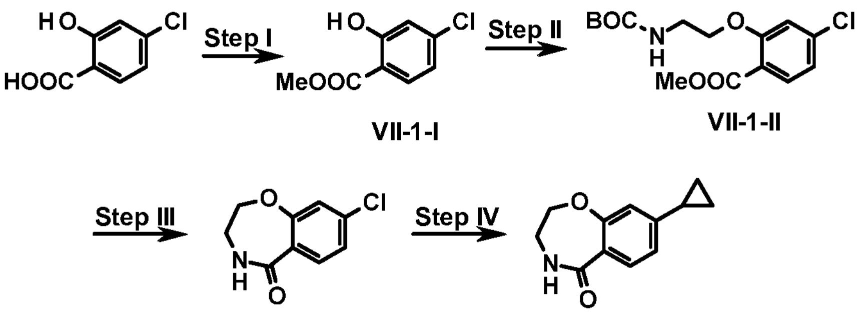

VII-1-I
VII-1-II
VII-1-III
VII-1

**Step-I: Methyl 4-chloro-2-hydroxy-benzoate (VII-1-I):** To a solution of 4-chlorosalicylic acid (75 g, 435.6 mmol) in anhydrous DMF (871 mL) was added $Cs_2CO_3$ (70.7 g, 217.8 mmol) and MeI (27.5 mL, 439.9 mmol) sequentially. The reaction mixture was stirred at room temperature overnight. Ice cold water (3 L) was added to the reaction mixture and the precipitated solid was filtered and dried to provide VII-1-I (75 g, 92% yield).

**Step-II: Methyl 2-[2-(tert-butoxycarbonylamino)ethoxy]-4-chloro-benzoate (VII-1-11):** To a solution of VII-1-I (126.8 g, 679.6 mmol) in anhydrous DMF (1 L) was added anhydrous $K_2CO_3$ (187.5 g, 1.36 mol) and stirred at room temperature for 30 min. Finally, a solution of tert-butyl N-(2-bromoethyl)carbamate (228.4 g, 1.02 mol) in THF (360 mL) was added to it and the reaction mixture was stirred at 65 °C for 16 h. The reaction mixture was, then, filtered

through celite and the filtrate was concentrated under reduced pressure. Diethyl ether (1.5 L) was added to the residue and was washed with water (1 L); brine (1 L), dried over anhydrous $Na_2SO_4$ filtered and concentrated under reduced pressure to provide crude intermediate VII-1-II (242 g); which was used without further purification.

**Step-III: 8-chloro-3,4-dihydro-2H-1,4-benzoxazepin-5-one (VII-1-III)**

To a solution of crude VII-1-II (242 g) in $CH_2Cl_2$ (500 mL) was added TFA (250 mL, 3.68 mol) at 0 °C and the reaction mixture was stirred at room temperature for 16 h. Solvent was removed under reduced pressure and the residue was dried. To this was added toluene (500 mL) and $Et_3N$ (512 mL, 3.68 mol) and the reaction mixture was refluxed for 16 h. Finally, solvents were removed under reduced pressure and ice cold water (500 mL) was added to the residue. The precipitated solid was filtered and dried. It was, then, stirred in diethyl ether (200 mL) and filtered to provide VII-1-III (40 g, 29% yield for two steps). LCMS: *m/z* 198 $(M+1)^+$.

**Step-IV: 8-cyclopropyl-3,4-dihydro-2H-1,4-benzoxazepin-5-one (VII-1):** Argon was purged through a suspension of VII-1-III (10 g, 50.7 mmol), cyclopropylboronic acid (13.1 g, 152 mmol), anhydrous $K_3PO_4$ (32 g, 152.3 mmol) and tricyclohexylphosphine (5.7 g, 20.3 mmol) in toluene:water (150 mL + 20 mL) for 30 min, followed by addition of $Pd(OAc)_2$ (2.3 g, 10.1 mmol). The reaction mixture was then stirred at 110 °C for 16 h. After completion of reaction, the reaction mixture was filtered through celite and the residue was washed with EtOAc (50 mL x 3). The combined organic layers were concentrated under reduced pressure and the residue was purified by silica gel column chromatography (25% acetone in hexane) to provide INT VII-1 (8 g, 77% yield). LCMS; *m/z*: 204 $(M+1)^+$. $^1H$ NMR ($CDCl_3$, 400 MHz) $\delta$ 0.73-0.77 (m, 2H); 0.99-1.04 (m, 2H); 1.86-1.90 (m, 1H); 3.50 (q, *J* = 4.8 Hz, 2H); 4.37 (two d, *J* = 4.4 Hz, 2H); 6.80 (d, *J* = 1.6 Hz, 1H); 6.81 (dd, $J_1$ = 1.2 Hz, $J_2$ = 8.8 Hz, 1H); 7.35 (bs, 1H); 7.89 (d, *J* = 8.8 Hz, 1H).

**Intermediate VII-5: 8-cyclopropyl-6-fluoro-3,4-dihydro-2H-1,4-benzoxazepin-5-one**

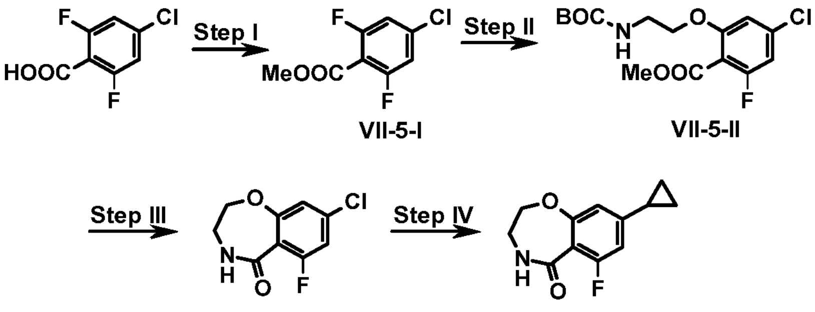


VII-5-I VII-5-II

VII-5-III VII-5

**Step-I: Methyl 4-chloro-2,6-difluoro-benzoate (VII-5-I):** Intermediate VII-5-I was synthesized as described for the synthesis of VII-1-1; using 4-chloro-2,6-difluorobenzoic acid (yield: 93%).

**Step-II: Methyl 2- [2-(tert-butoxycarbonylamino)ethoxy] -4-chloro-6-fluoro-benzoate (VII-5-II):** A solution of VII-5-I (5 g, 24.2 mmol) and tert-butyl N-(2-hydroxyethyl)carbamate (4.7 g, 29.0 mmol) in anhydrous THF (50 mL) was cooled to -40 °C, and NaH (washed with hexane) (0.76 g, 31.5 mmol) was added to it portion wise over 30 min. The reaction mixture was warmed to -20 °C over 1 h and quenched using saturated $NH_4Cl$ solution (100 mL). Extraction was carried out using EtOAc (50 mL); the combined organic layers were washed with brine (100 mL), dried over anhydrous $Na_2SO_4$ filtered and concentrated under reduced pressure. The residue was purified by silica gel column chromatography (12% EA in hexane) to provide VII-5-II (5.2 g, 61% yield). LCMS; *m/z*: 348 $(M+1)^+$.

**Step-III: 8-chloro-6-fluoro-3,4-dihydro-2H-1,4-benzoxazepin-5-one (VII-5-III):** To a solution of VII-5-II (4.8 g, 13.8 mmol) in MeOH (50 mL) was added aqueous NaOH solution (2.8 g in 30 mL $H_2O$, 69.2 mmol) and the reaction mixture was stirred at 60 °C for 1 h. MeOH was removed under reduced pressure and 10% citric acid solution was added to the reaction mixture to make it acidic (pH < 4). Extraction was carried out using EtOAc (50 mL x 3); the combined organic layers were washed with water (100 mL), brine (100 mL), dried over anhydrous $Na_2SO_4$ filtered and concentrated under reduced pressure. The residue was, then, stirred in 1M HCl in dioxane (50 mL) at room temperature for 3 h. Finally, dioxane was removed under reduced pressure; the residue was washed with ether (30 mL x 2) and dried. The solid obtained was, then, dissolved in DMF (30 mL), to which N-methylmorpholine (4.2 mL; 38.7 mmol) and HATU (7.4 g, 19.4 mmol) were added sequentially. The reaction mixture was stirred at room temperature for 16 h. Finally, ice-cold water was added to the reaction mixture and the resultant solid was filtered and dried to provide VII-5-III (1.8 g, 60% yield). LCMS; *m/z*: 216 $(M+1)^+$.

**Step-IV: 8-cyclopropyl-6-fluoro-3,4-dihydro-2H-1,4-benzoxazepin-5-one (VII-5):** Intermediate VII-5 was synthesized as described for the synthesis of VII-1; using intermediate VII-5-III (yield: 80%). LCMS; *m/z*: 222 $(M+1)^+$. $^1H$ NMR ($CDCl_3$, 400 MHz) $\delta$ 0.65-0.68 (m, 2H); 1.01-1.04 (m, 2H); 1.81-1.86 (m, 1H); 3.40 (q, *J* = 5.6 Hz, 2H); 4.27 (t, *J* = 5.6 Hz, 2H); 6.57 (s, 1H); 6.62 (d, *J* = 11.2 Hz, 1H); 7.28 (bs, 1H).

Alternatively, intermediate VII-5 can also be synthesized using 4-bromo-2,6-difluorobenzoic acid as described below.

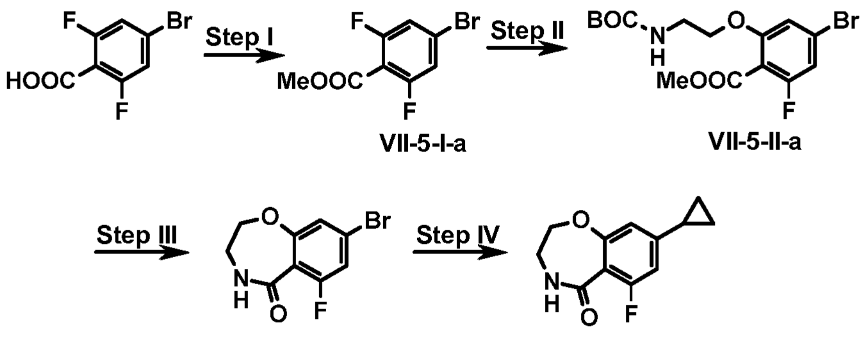


VII-5-I-a VII-5-II-a

VII-5-III-a VII-5

**Step-I: methyl 4-bromo-2,6-difluoro-benzoate (VII-5-I-a):** As described for the synthesis of VII-5-I.
**Step-II: methyl 4-bromo-2-[2-(tert-butoxycarbonylamino)ethoxy]-6-fluoro-benzoate (VII-5-II-a):** As described for the synthesis of VII-5-II.
**Step-III: 8-bromo-6-fluoro-3,4-dihydro-2H-1,4-benzoxazepin-5-one (VII-5-III-a):** As described for the synthesis of VII-5-III-a.
**Step-IV: 8-cyclopropyl-6-fluoro-3,4-dihydro-2H-1,4-benzoxazepin-5-one (VII-5):** As described above (VII-5-III to VII-5).

Alternate synthesis of VII-5:

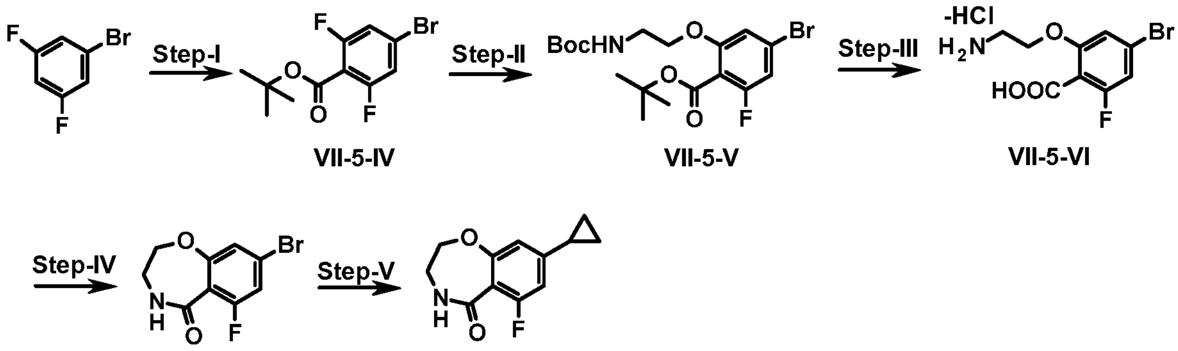


VII-5-IV VII-5-V VII-5-VI

VII-5-III-a VII-5

**Step-I: tert-butyl 4-bromo-2,6-difluoro-benzoate (VII-5-IV):** To a solution of diisopropylamine (17.9 mL, 0.12 mol) in anhydrous THF at -78 °C was added n-BuLi (45.5 mL, 0.11 mol, 2.5 M in hexane) dropwise, over period of 15 min and the resulting solution was stirred at -78 °C for 30 mins. To this was added 1-bromo-3,5-difluoro benzene (20.0 g, 0.10 mol) in THF (30 mL) dropwise and it was stirred for 1 h. Finally, Boc-anhydride (26.0 mL, 0.11 mol) was added to it and the reaction mixture was slowly allowed to come to room temperature over a period of 2 h. After completion of the reaction, it was diluted with water (100 mL) and extraction was carried out using diethyl ether (300 x 2). The combine organic layers were washed with water (250 mL), brine (250 mL) and dried over anhydrous $Na_2SO_4$ filtered and concentrated under reduced pressure to provide crude VII-5-IV (26 g) as a brown liquid, which was used for next step with out any purification. $^1H$ NMR (DMSO-$d_6$, 400 MHz) $\delta$ 1.42 (s, 9H), 7.63 (d, *J* = 8.0 Hz, 2H).
**Step-II: tert-butyl 4-bromo-2-[2-(tert-butoxycarbonylamino)ethoxy]-6-fluoro-benzoate (VII-5-V):** A solution of VII-5-IV (26 g, 88.7 mmol) and tert-butyl *N*-(2-hydroxyethyl)carbamate (15.7 g, 97.6 mmol) in anhydrous THF (400 mL) was cooled to -10 °C; and NaH (60% suspension in oil) (5.3 g, 133 mmol) was added to it portion wise over 30

min. The reaction mixture was stirred for 1 h and quenched using saturated $NH_4Cl$ solution (300 mL). Extraction was carried out using EtOAc (300 mL x 2); the combined organic layers were washed with brine (350 mL), dried over anhydrous $Na_2SO_4$ filtered and concentrated under reduced pressure to provide desired product VII-5-V (32 g) as a brown thick oil, which was used for next step with out any purification. LCMS: m/z: 278 [M-(2 x BOC)]$^+$ (67% pure). $^1$H NMR ($CDCl_3$, 400 MHz) $\delta$ 1.44 (s, 9H); 1.56 (s, 9H); 3.51 (q, *J* = 5.4 Hz, 2H); 4.07 (t, *J* = 5.3 Hz, 2H); 5.07 (bs, 1H); 6.84 (bs, 1H); 6.93 (dd, $J_1$ = 1.4 Hz, $J_2$ = 8.1 Hz, 1H).

**Step-III: 2-(2-aminoethoxy)-4-bromo-6-fluoro-benzoic acid (hydrochloride salt) (VII-5-VI):** To a solution of compound VII-5-V (32 g) in anhydrous $CH_2Cl_2$ (400 mL) was added a solution of 4M HCl in dioxane (55 mL); and the reaction mixture was stirred at room temperature for 16 h. Finally, solvents were removed under reduced pressure; the residue was washed with diethyl ether (50 mL x 2) and dried to provide desired compound VII-5-VI (17 g) as white solid. LCMS: m/z: 278 (M + 1)$^+$. $^1$H NMR (DMSO-$d_6$, 400 MHz) $\delta$ 3.10-3.16 (m, 2H); 4.33 (t, *J*= 6.0 Hz, 2H); 7.30-7.35 (aromatics, 2H); 8.11 (bs, 3H); 13.60 (bs, 1H). **Step-IV: 8-bromo-6-fluoro-3,4-dihydro-2H-1,4-benzoxazepin-5-one (VII-5-III-a):** Intermediate VII-5-VI was subjected to intramolecular amide coupling cyclization reaction conditions as described in step-III of synthesis of VII-5-III; to provide VII-5-III-a.

**Step-V:** As described in step-IV for the synthesis of VII-5 (from VII-5-III).

**Intermediate VIII-5: [2-bromo-6-(8-cyclopropyl-5-oxo-2,3-dihydro-1,4-benzoxazepin-4-yl)phenyl]methyl acetate**

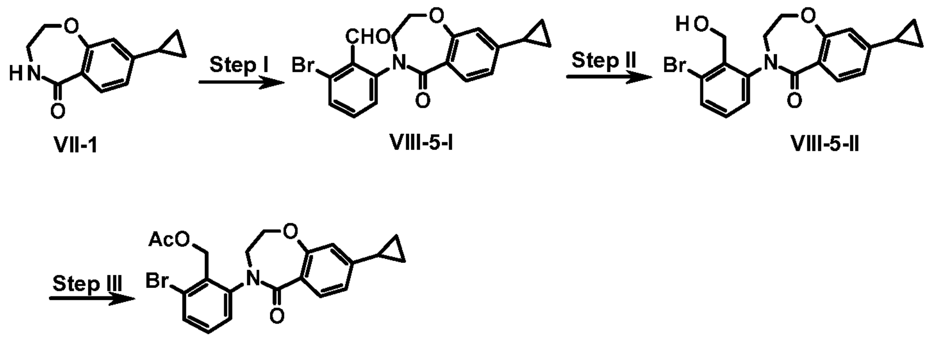


VII-1 VIII-5-I VIII-5-II VIII-5

**Step-I: 2-bromo-6-(8-cyclopropyl-5-oxo-2,3-dihydro-1,4-benzoxazepin-4-yl)benzaldehyde (VIII-5-I):** To a stirred solution of 8-cyclopropyl-3, 4-dihydro-2H-1, 4-benzoxazepin-5-one (VII-1) (2.0 g, 9.85 mmol) in 1, 4-dioxane (10 ml) was added 2, 6-dibromobenzaldehyde (5.14 g, 19.7 mmol), xanthphos (0.171 g, 0.29 mmol) and cesium carbonate (4.50 g, 13.79 mmol, 1.4 equiv.). The reaction mixture was purged using Argon for 45 minutes and then $Pd(dba)_2$ (0.113 g, 1.97 mmol) was added. The resulting reaction mixture was stirred at 100 °C for 18 h. Reaction mixture was cooled to room temperature and filtered through celite and washed with ethyl acetate (20 mL x 2). The combined organic layers were washed with water (50 mL); brine (50 mL), dried over anhydrous $Na_2SO_4$ filtered and concentrated under reduced pressure to provide crude product, which was further purified using silica gel column chromatography (20-25% ethyl acetate in hexanes) to provide the title compound VIII-5-I (1.60 g, 43%). LCMS: *m/z* 386.0 (M+1)$^+$.

**Step-II: 4-[3-bromo-2-(hydroxymethyl) phenyl]-8-cyclopropyl-2, 3-dihydro-1, 4-benzoxazepin-5-one (VIII-5-II):** To a stirred solution of VIII-5-I (1.6 g, 4.14 mmol) in THF (12 mL) was added superhydride (1M solution in THF, 8.3 mL, 8.29 mmol) at 20 °C. After 15 min, the reaction mixture was quenched using saturated $NH_4Cl$ solution (30 mL) and extracted using EtOAc (30 mL x 2). The combined organic layers were washed with brine (30 mL), dried over anhydrous $Na_2SO_4$ filtered and evaporated under reduced pressure to give crude product, which was further purified using silica gel column chromatography (25-30% EtOAc in hexanes) to give the title compound VIII-5-II (1.35 g, 85%). LCMS: *m/z* 388 (M+1)$^+$.

**Step-III: [2-bromo-6-(8-cyclopropyl-5-oxo-2, 3-dihydro-1, 4-benzoxazepin-4-yl) phenyl] methyl acetate (VIII-5):** To a stirred solution of VIII-5-11 (1.30 g, 3.35 mmol) in $CH_2Cl_2$ (15 mL) was added TEA (1.0 ml, 5.02 mmol), acetic anhydride (0.62 ml, 6.71 mmol) and DMAP (50 mg, 0.35 mmol) at room temperature. After stirring for 2 h, volatiles were evaporated under reduced pressure and the residue was purified using silica gel column chromatography (15-25% ethyl acetate in hexanes) to provide title compound VIII-5 (1.37 g, 95%). LCMS: *m/z* 430 (M+1)$^+$. $^1$H NMR ($CDCl_3$, 400 MHz) $\delta$ 0.74-0.78 (m, 2H); 1.02-1.07 (m, 2H); 1.86-1.93 (m, 1H); 2.05 (s, 3H); 3.73-3.79 (m, 1H); 3.87-3.94 (m, 1H); 4.40-4.50 (m, 2H); 5.11 (d, *J* = 12.0 Hz, 1H); 5.33 (d, *J* = 12.0 Hz, 1H); 6.74 (d, *J* = 1.6 Hz, 1H); 6.89 (dd, $J_1$ = 1.6 Hz, $J_2$ = 8.4 Hz, 1H); 7.24-7.28 (aromatics, 1H); 7.32 (t, *J* = 8.0 Hz, 1H); 7.63 (td, $J_1$ = 1.2 Hz, $J_2$

= 8.0 Hz, 1H); 7.75 (d, *J* = 8.0 Hz, 1H).

Following intermediates were synthesized using similar reaction sequence and procedure as described for the synthesis of intermediate VIII-5 (Table-2).

**Table-2**

| No | Structure/IUPAC name | Characterization data | INT used |
|---|---|---|---|
| VIII-11 | 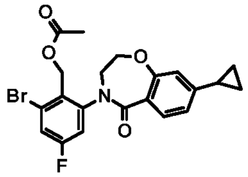 [2-bromo-6-(8-cyclopropyl-5-oxo-2,3-dihydro-1,4-benzoxazepin-4-yl)-4-fluorophenyl]methyl acetate | LCMS: *m/z*; 448 $(M+1)^+$ | VII-1 (2,6-dibromo-4-fluoro benzal dehyde) |

**Synthesis of intermediate VIII-12: [2-bromo-6-(8-cyclopropyl-6-fluoro-5-oxo-2,3-dihydro-1,4-benzoxazepin-4-yl)-4-fluoro-phenyl]methyl acetate**

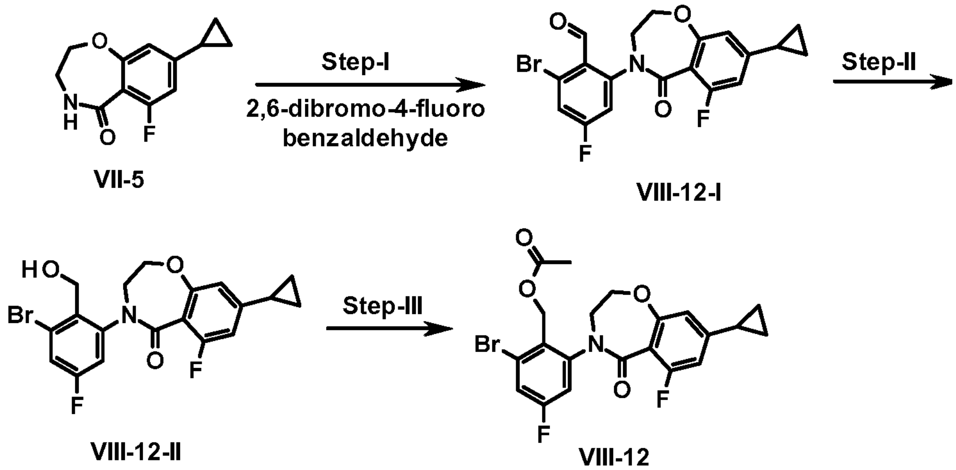


VII-5 VIII-12-I VIII-12-II VIII-12

**Step-I: 2-bromo-6-(8-cyclopropyl-6-fluoro-5-oxo-2,3-dihydro-1,4-benzoxazepin-4-yl)-4-fluoro-benzaldehyde (VIII-12-I):** To a stirred solution of VII-5 (22 g, 99.5 mmol) in 1, 4-dioxane (140 ml) was added 2,6-dibromo-4-fluorobenzaldehyde (56 g, 199 mmol) and sodium carbonate (21 g, 199 mmol). The reaction mixture was purged using Argon for 30 minutes and then CuI (19 g, 99. 5 mmol) was added to it. Ar purging was continued for additional 15 min and then the resulting reaction mixture was stirred at 110 °C for 24 h. It was cooled to room temperature and the solvent was removed under reduced pressure. EtOAc (220 mL) was added to the residue, stirred for 15 min and filtered through celite. The residue was washed additionally using EtOAc (150 mL x 2). Finally, the combined organic layers were washed with water (250 mL); brine (250 mL), dried over anhydrous $Na_2SO_4$ filtered and concentrated under reduced pressure. The residue was then subjected to silica gel column chromatography (initially column was washed using 5-15% EtOAc in hexane, and then product was eluted using $CH_2Cl_2$) and then the solid obtained was washed using diethyl ether to provide VIII-12-I. Additional quantity of VIII-12-I was recovered by column purification of residue obtained after filtrate evaporation. Isolated yield: 25 g (60% yield). LCMS: *m/z* 422.0 $(M+1)^+$. $^1H$ NMR (DMSO-$d_6$; 400 MHz) $\delta$ 0.77-0.81 (m, 2H); 1.01-1.05 (m, 2H); 1.96-2.02 (m, 1H); 3.91 (t, *J* = 5.2 Hz, 2H); 4.42 (t, *J* = 5.2 Hz, 2H); 6.76 (bs, 1H); 6.83 (dd, $J_1$ = 1.2 Hz, $J_2$ = 11.6 Hz, 1H); 7.58 (dd, $J_1$ = 2.4 Hz, $J_2$ = 9.2 Hz, 1H); 7.83 (dd, $J_1$ = 2.4 Hz, $J_2$ = 8.4 Hz, 1H); 10.0 (s, 1H).

**Step-II: 4-[3-bromo-5-fluoro-2-(hydroxymethyl)phenyl]-8-cyclopropyl-6-fluoro-2,3-dihydro-1,4-benzoxazepin-5-one (VIII-12-II):** To a stirred solution of VIII-12-I (10 g, 23.7 mmol) in dry MeOH-THF (200 mL + 100 mL) was added $NaBH_4$ (0.9 g, 23.7 mmol) portion wise at -20 °C. The reaction was monitored by TLC (complete in 30

min). It was, then, quenched using saturated $NH_4Cl$ solution (100 mL) and organic solvents were removed under reduced pressure. The aqueous layer was extracted using $CH_2Cl_2$ (100 mL x 3). The combined organic layers were washed with brine (150 mL), dried over anhydrous $Na_2SO_4$ filtered and evaporated under reduced pressure to give crude product, which was further purified by silica gel column chromatography (neat $CH_2Cl_2$) (column purification done twice) to give title compound VIII-12-II (8.5 g, 85% yield). LCMS: *m/z* 424 $(M+1)^+$. $^1$H NMR (DMSO-$d_6$; 400 MHz) $\delta$ 0.77-0.81 (m, 2H); 1.01-1.05 (m, 2H); 1.96-2.02 (m, 1H); 3.81 (t, $J$ = 5.2 Hz, 2H); 4.42 (t, $J$ = 5.2 Hz, 2H); 4.46-4.50 (m, 2H); 5.01 (t, $J$ = 4.8 Hz, 1H); 6.76 (bs, 1H); 6.84 (dd, $J_1$ = 1.6 Hz, $J_2$ = 11.6 Hz, 1H); 7.35 (dd, $J_1$ = 2.8 Hz, $J_2$ = 9.2 Hz, 1H); 7.69 (dd, $J_1$ = 2.8 Hz, $J_2$ = 8.8 Hz, 1H).

**Step-III: [2-bromo-6-(8-cyclopropyl-6-fluoro-5-oxo-2,3-dihydro-1,4-benzoxazepin-4-yl)-4-fluoro-phenyl]methyl acetate (VIII-12):** To a stirred solution of VIII-12-II (54 g, 127.4 mmol) in $CH_2Cl_2$ (540 mL) was added TEA (53 ml, 382 mmol) and DMAP (3.1 g, 25.5 mmol); and it was cooled to 0 °C. Finally, acetic anhydride (24 mL, 254.7 mmol) was added to it and the reaction mixture was stirred at room temperature for 2-3 h. After completion of the reaction, the organic layer was washed with saturated $NaHCO_3$ (300 mL), brine (300 mL); dried over anhydrous $Na_2SO_4$ filtered and concentrated under reduced pressure. The residue was purified using silica gel column chromatography (initially column was washed using 10% EtOAc in hexane, then product eluted using $CH_2Cl_2$) to provide title compound VIII-12 (54 g, 94%). LCMS: *m/z* 466.1 $(M+1)^+$. $^1$H NMR (DMSO-$d_6$; 400 MHz) $\delta$ 0.77-0.81 (m, 2H); 1.01-1.06 (m, 2H); 1.96-2.02 (m, 1H); 2.00 (s, 3H); 3.75-3.90 (m, 2H); 4.20-4.30 (m, 1H); 4.38-4.48 (m, 1H); 4.98 (d, $J$ = 12.4 Hz, 1H); 5.11 (d, $J$ = 12.4 Hz, 1H); 6.76 (bs, 1H); 6.85 (dd, $J_1$ = 1.2 Hz, $J_2$ = 11.2 Hz, 1H); 7.46 (dd, $J_1$ = 2.8 Hz, $J_2$ = 9.2 Hz, 1H); 7.78 (dd, $J_1$ = 2.8 Hz, $J_2$ = 8.8 Hz, 1H).

**Intermediate III-12: [2-(8-cyclopropyl-6-fluoro-5-oxo-2,3-dihydro-1,4-benzoxazepin-4-yl)-4-fluoro-6-(4,4,5,5-tetramethyl-1,3,2-dioxaborolan-2-yl)phenyl]methyl acetate**

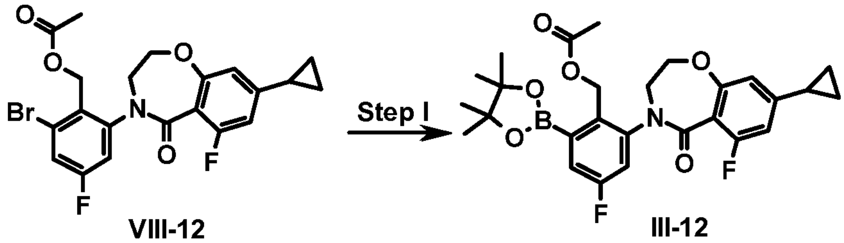


VIII-12 III-12

**Step-I:** Argon was purged through a solution of **VIII-12** (15 g, 32.11 mmol), bispinacolato diboron (12.3 g, 48.2 mmol) and potassium acetate (6.3 g, 64.2 mmol) in 1,4-dioxane (300 mL), for 30 minutes and then Pd(dppf)$Cl_2$-$CH_2Cl_2$ (1.3 g, 1.61 mmol) was added. Purging was continued for 15 min more and then the reaction mixture was refluxed for 16 h. After completion of the reaction, dioxane was removed under reduced pressure and the residue was dissolved in EtOAc (300 mL). It was filtered through celite, which was washed with additional EtOAc (100 mL) and then the combined organic layers were washed with water (300 mL); brine (300 mL); dried over anhydrous $Na_2SO_4$ filtered and concentrated under reduced pressure. The residue was purified using silica gel column chromatography (5-30% EtOAc in hexane) to provide title compound **III-12** (16 g, quantitative yield). LCMS: *m/z* 514 $(M+1)^+$ (55% boronate ester mass; 27% corresponding boronic acid, 12% des-halo byproduct)

Following intermediates were synthesized using similar procedure as described for III-12 (Table-3)

**Table-3**

| No | Structure/IUPAC name | Characterization data | INT used |
|---|---|---|---|
| III-11 | 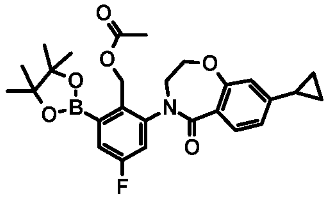 [2-(8-cyclopropyl-5-oxo-2,3-dihydro-1,4-benzoxazepin-4-yl)-4-fluoro-6-(4,4,5,5-tetramethyl-1,3,2-dioxaborolan-2-yl)phenyl]methyl acetate | LCMS: *m/z*; 496 $(M+1)^+$ | VIII-11 |

**Intermediate XI-3: 6-[4-chloro-1-(2-trimethylsilylethoxymethyl)pyrrolo[2,3-b]pyridine-2-yl]pyridine-3-carbaldehyde**

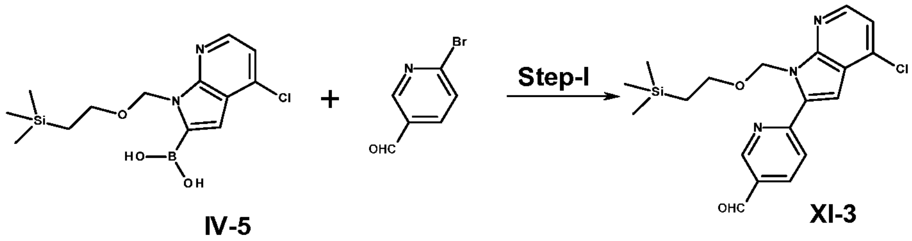


IV-5 XI-3

**Step-I:** Argon was purged through a solution of IV-5 (1.05 g, 3.22 mmol), 6-bromo-3-pyridinecarboxaldehyde (CAS: 149806-06-4) (0.5 g, 2.7 mmol) and KF (0.47 g, 8.06 mmol) in acetonitrile: water (7 mL + 3.5 mL), for 30 min. Finally, $Pd(PPh_3)_4$ (0.93 g, 0.81 mmol) was added to the reaction mixture and purging was continued for 10 min more. It was, then, stirred at 90 °C for 4 h. After cooling the reaction mixture, it was diluted using EtOAc (60 mL) and the organic layer was washed with water (50 mL x 2), brine (50 mL), dried over anhydrous $Na_2SO_4$ filtered and concentrated under reduced pressure. The residue was then purified using silica gel column chromatography (12% EtOAc in hexanes) to provide XI-3 (0.56 g, 53% yield). LCMS: *m/z*; 388.1 $(M+1)^+$. $^1H$ NMR ($CDCl_3$; 400 MHz) $\delta$ -0.16 (s, 9H); 0.82 (t, *J* = 8.4 Hz, 2H); 3.52 (t, *J* = 8.4 Hz, 2H); 6.29 (s, 2H); 7.18 (d, *J* = 5.2 Hz, 1H); 7.19 (s, 1H); 8.08 (d, *J* = 8.4 Hz, 1H); 8.26 (dd, $J_1$ = 2.4 Hz, $J_2$ = 8.4 Hz, 1H); 8.31 (d, *J* = 5.2 Hz, 1H); 9.15 (d, *J* = 1.2 Hz, 1H); 10.15 (s, 1H).

**Example 1: Synthesis of 8-cyclopropyl-6-fluoro-4-[5-fluoro-2-(hydroxymethyl)-3-[2-[5-[[4-(oxetan-3-yl)piperazin-1-yl]methyl]-2-pyridyl]-1H-pyrrolo[2,3-b]pyridin-4-yl]phenyl]-2,3-dihydro-1,4-benzoxazepin-5-one**

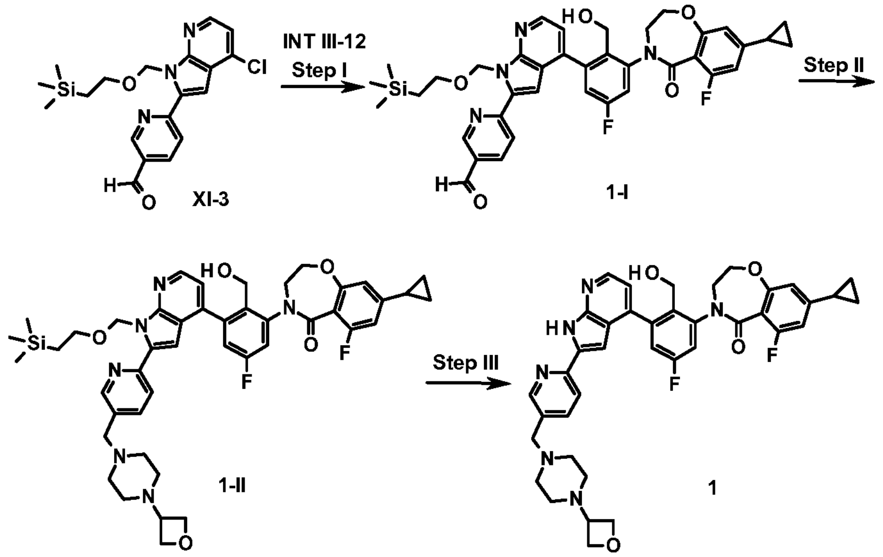


XI-3 1-I 1-II 1

**Step-I: Synthesis of 6-[4-[3-(8-cyclopropyl-6-fluoro-5-oxo-2,3-dihydro-1,4-benzoxazepin-4-yl)-5-fluoro-2-(hydroxymethyl)phenyl]-1-(2-trimethylsilylethoxy methyl)pyrrolo[2,3-b]pyridin-2-yl]pyridine-3-carbaldehyde (1-I):** To a solution of XI-3 (20 g, 51.67 mmol) and III-12 (80% pure by LCMS) (37.1 g, 57.74 mmol) in 1,4-dioxane (400 mL) was added a solution of $Na_2CO_3$ (5.9 g, 155 mmol) in water (80 mL). Argon was purged through this for 20 min. Finally, $Pd(PPh_3)_4$ (5.9 g, 5.16 mmol) was added to it and purging was continued for 20 min more. The reaction mixture was then stirred at 100 °C (oil-bath temperature) for 9 h. After completion of the reaction, dioxane was removed under reduced pressure and the residue was diluted using water (250 mL). Extraction was carried out using EtOAc (200 mL x 3); the combined organic layers were washed with brine (300 mL); dried over anhydrous $Na_2SO_4$ filtered and concentrated under reduced pressure. The residue was purified using silica gel column chromatography to provide **1-I** (28 g, 78% yield). LCMS: *m/z* 697 (M + 1)$^+$. (60% pure, it contains TPPO as

impurity).

**Step-II: Synthesis of 8-cyclopropyl-6-fluoro-4-[5-fluoro-2-(hydroxymethyl)-3-[2-[5-[[4-(oxetan-3-yl)piperazin-1-yl] methyl]-2-pyridyl]-1-(2-trimethylsilylethoxymethyl) pyrrolo[2,3-b]pyridin-4-yl]phenyl]-2,3-dihydro-1,4-benzoxazepin-5-one (1-II):** To a solution of **1-I** (mixture of OH and OAc, ~25% OAc) (3.7 g, 5.3 mmol) in $CH_2Cl_2$ (40 mL) was added 1-(oxetan-3-yl)piperazine (7.5 g, 53 mmol) (CAS: 1254115-23-5), HOAc (5 drops) and 4 A° molecular sieves (~500 mg). After stirring for 2 h, sodium triacetoxyborohydride (2.2 g, 10.6 mmol) was added to it and the reaction mixture was stirred for 16 h. After complete consumption of starting material (indicated by TLC), saturated $NaHCO_3$ solution (60 mL) was added to it and extraction was carried out using $CH_2Cl_2$ (50 mL x 2). The combined organic layers were washed with water (100 mL), brine (100 mL); dried over anhydrous $Na_2SO_4$ filtered and concentrated under reduced pressure. The residue was purified using silica gel column chromatography (2-3% MeOH in $CH_2Cl_2$) to provide desired intermediate **1-II.** Isolated yield: 3.3 g (mixture of OH and OAc); LCMS: *m/z* 823 $(M+1)^+$.

**Step-III:** To a solution of **1-II** (4.1 g, 4.98 mmol) in $CH_2Cl_2$ (40 mL) was added TFA (20 mL, excess) and the reaction mixture was stirred at room temperature for 2 h. After completion of the reaction, solvents were removed under reduced pressure and saturated $NaHCO_3$ solution (50 mL) was added to the residue (pH: ~8). Extraction was carried out using $CH_2Cl_2$ (70 mL x 3); the combined organic layers were washed with brine (100 mL); dried over anhydrous $Na_2SO_4$ filtered and concentrated under reduced pressure. The residue obtained was dissolved in THF (50 mL), to which aqueous $LiOH-H_2O$ (1 g, 24.9 mmol, 10 mL water) was added and it was stirred at room temperature for 6-8 h (to convert OAc to OH). It was then diluted using water (30 mL) and extraction was carried out using EtOAc (50 mL x 3). The combined organic layers were washed with brine (100 mL); dried over anhydrous $Na_2SO_4$ filtered and concentrated under reduced pressure. The residue obtained was subjected to silica gel column chromatography (4-5% MeOH in $CH_2Cl_2$); followed by crystallization of the residue using acetonitrile (20 mL) to provide final product 1 (1.3 g, 37% yield). LCMS: *m/z* 693.3 $(M+1)^+$. $^1H$ NMR (DMSO-$d_6$, 400 MHz) $\delta$ 0.77-0.82 (m, 2H); 1.00-1.10 (m, 2H); 1.95-2.05 (m, 1H); 2.30-2.50 (m, 8H); 3.34-3.41 (m, 1H); 3.53 (s, 2H); 3.80-4.00 (m, 2H); 4.15-4.30 (m, 1H); 4.30-4.40 (m, 2H); 4.40 (t, $J$ = 6.0 Hz, 2H); 4.51 (t, $J$ = 6.0 Hz, 2H); 4.50-4.60 (m, 1H); 4.79 (bs, 1H); 6.77 (s, 1H); 6.84 (d, $J$ = 11.2 Hz, 1H); 6.93 (d, $J$ = 2.0 Hz, 1H); 7.22 (d, $J$ = 3.6 Hz, 1H); 7.34 (dd, $J_1$ = 2.8 Hz, $J_2$ = 9.2 Hz, 1H); 7.40 (dd, $J_1$ = 3.2 Hz, $J_2$ = 9.2 Hz, 1H); 7.76 (dd, $J_1$ = 2.0 Hz, $J_2$ = 8.4 Hz, 1H); 8.04 (d, $J$ = 8.0 Hz, 1H); 8.34 (d, $J$ = 4.8 Hz, 1H); 8.53 (s, 1H); 12.38 (s, 1H)

**Alternate route for the synthesis of 1:**

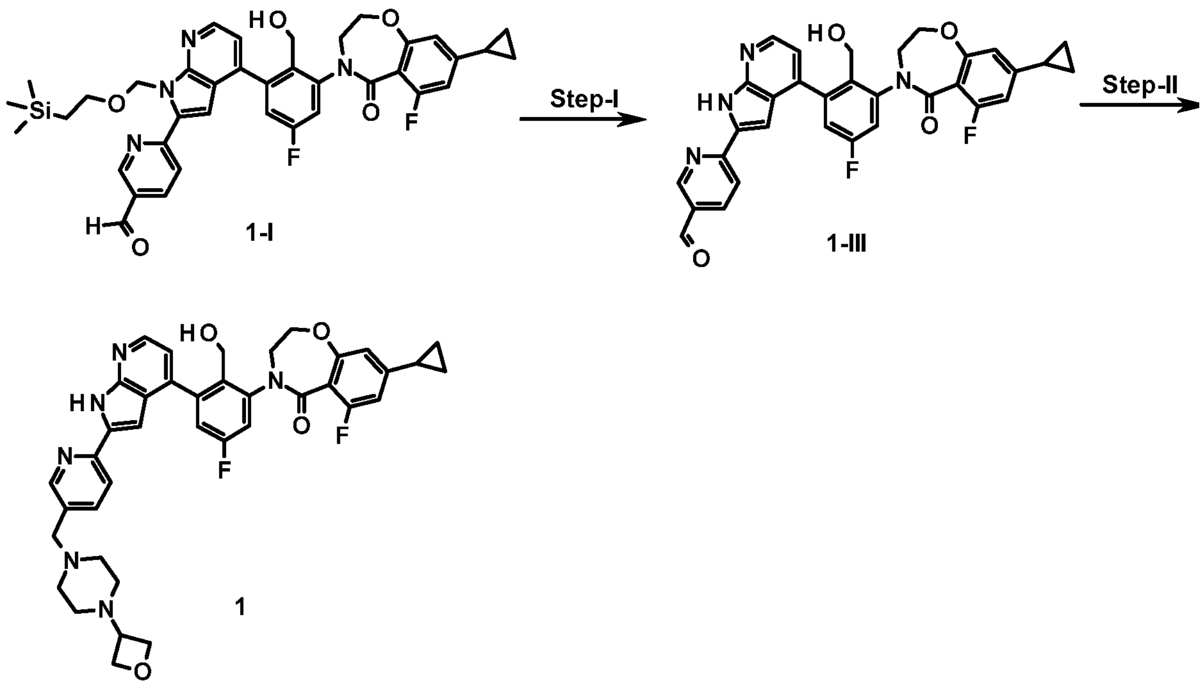


1-I

1-III

1

**Step-I: Synthesis of 6-[4-[3-(8-cyclopropyl-6-fluoro-5-oxo-2,3-dihydro-1,4-benzoxazepin-4-yl)-5-fluoro-2-(hydroxymethyl)phenyl]-1H-pyrrolo[2,3-b]pyridin-2-yl]pyridine-3-carbaldehyde (1-III):** To a solution of **1-I**

(60% by LCMS, TPPO as impurity) (20 g, 28.7 mmol) in anhydrous $CH_2Cl_2$ (200 mL) was added TFA (220 mL, excess) and it was stirred at room temperature for 3-4 h. After completion of the reaction, solvents were removed under reduced pressure (<40 °C) and saturated $NaHCO_3$ solution (~300 mL) was added to the residue (pH: ~8). Extraction was carried out using $CH_2Cl_2$ (200 mL x 3); the combined organic layers were washed with brine (300 mL); dried over anhydrous $Na_2SO_4$, filtered and concentrated under reduced pressure. The residue was purified using silica gel column chromatography (2% MeOH in $CH_2Cl_2$) to provide **1-III** (8.5 g, 52% yield). LCMS: 567 $(M+1)^+$.

**Step-II:** To a solution of **1-III** (8.5 g, 15 mmol) in $CH_2Cl_2$ (250 mL) was added 1-(oxetan-3-yl)piperazine (10.7 g, 75 mmol), HOAc (0.84 g, 15 mmol) and 4 A° molecular sieves (~2 g). After stirring for 2 h, sodium triacetoxyborohydride (6.36 g, 30 mmol) was added to it and the reaction mixture was stirred for 3 h. After complete consumption of starting material (indicated by TLC), saturated $NaHCO_3$ solution (100 mL) was added to it and extraction was carried out using $CH_2Cl_2$ (100 mL x 3). The combined organic layers were washed with water (150 mL), brine (150 mL); dried over anhydrous $Na_2SO_4$, filtered and concentrated under reduced pressure. The residue was purified using silica gel column chromatography (2-5% MeOH in $CH_2Cl_2$) to provide desired compound **1** (9.1 g, 87% yield).

Following compounds were synthesized using similar sequence of reactions and procedures as described for the synthesis of **1**, and using intermediates XI-3, III-12 and the respective amines for reductive amination (Table-4).

**Table-4**

| No | Structure/IUPAC name | Characterization | amine used for reductive amination |
|---|---|---|---|
| 2 | 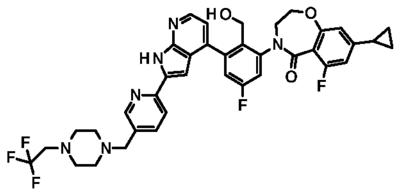 8-cyclopropyl-6-fluoro-4-[5-fluoro-2-(hydroxymethyl)-3-[2-[5-[[4-(2,2,2-trifluoroethyl)piperazin-1-yl]methyl]-2-pyridyl]-1H-pyrrolo[2,3-b]pyridin-4-ylphenyl]-2,3-dihydro-1,4-benzoxazepin-5-one | LCMS: $m/z$ 719.3 $(M+1)^+$. $^1H$ NMR (DMSO-$d_6$, 400 MHz) $\delta$ 0.78-0.84 (m, 2H); 1.00-1.06 (m, 2H); 1.96-2.02 (m, 1H); 2.35-2.45 (m, 4H); 2.60-2.65 (m, 4H); 3.15 (q, $J$ = 10.8 Hz, 2H); 3.53 (s, 2H); 3.83-4.00 (m, 2H); 4.15-4.30 (m, 1H); 4.30-4.45 (m, 2H); 4.50-4.60 (m, 1H); 4.82 (bs, 1H); 6.76 (s, 1H); 6.83 (dd, $J_1$ = 1.6 Hz, $J_2$ = 11.2 Hz, 1H); 6.93 (d, $J$ = 2.0 Hz, 1H); 7.21 (d, $J$ = 3.2 Hz, 1H); 7.34 (dd, $J_1$ = 2.8 Hz, $J_2$ = 9.2 Hz, 1H); 7.40 (dd, $J_1$ = 2.8 Hz, $J_2$ = 9.2 Hz, 1H); 7.77 (dd, $J_1$ = 2.4 Hz, $J_2$ = 8.0 Hz, 1H); 8.04 (d, $J$ = 8.0 Hz, 1H); 8.34 (d, $J$ = 4.8 Hz, 1H); 8.53 (d, $J$ = 1.2 Hz, 1H); 12.36 (s, 1H) | N-(trifluoro ethyl) piperazine; CAS: 13349-90-1 |
| 3 | 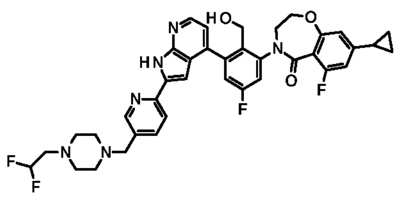 8-cyclopropyl-4-[3-[2-[5-[[4-(2,2-difluoroethyl)piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one | LCMS: $m/z$ 701.3 $(M+1)^+$. $^1H$ NMR (DMSO-$d_6$, 400 MHz) $\delta$ 0.78-0.85 (m, 2H); 1.00-1.08 (m, 2H); 1.96-2.02 (m, 1H); 2.35-2.45 (m, 4H); 2.50-2.60 (m, 4H); 2.70 (td, $J_1$ = 4.4 Hz, $J_2$ = 15.6 Hz, 2H); 3.52 (s, 2H); 3.80-4.00 (m, 2H); 4.15-4.30 (m, 1H); 4.30-4.45 (m, 2H); 4.50-4.60 (m, 1H); 4.82 (bs, 1H); 6.11 (tt, $J_1$ = 4.4 Hz, $J_2$ = 56 Hz, 1H); 6.77 (s, 1H); 6.84 (d, $J$ = 11.2 Hz, 1H); 6.93 (d, $J$ = 1.2 Hz, 1H); 7.21 (d, $J$ = 4.0 Hz, 1H); 7.34 (dd, $J_1$ = 2.8 Hz, $J_2$ = 9.2 Hz, 1H); 7.40 (dd, $J_1$ = 2.8 Hz, $J_2$ = 9.2 Hz, 1H); 7.77 (dd, $J_1$ = 2.0 Hz, $J_2$ = 8.4 Hz, 1H); 8.03 (d, $J$ = 8.4 Hz, 1H); 8.34 (d, $J$ = 4.4 Hz, 1H); 8.53 (d, $J$ = 1.6 Hz, 1H); 12.36 (s, 1H) | N-(di-fluoro ethyl) piperazine (CAS: 767609-14-3) |

(continued)

| No | Structure/IUPAC name | Characterization | amine used for reductive amination |
|---|---|---|---|
| 4 | 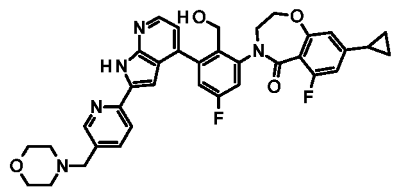 8-cyclopropyl-6-fluoro-4-[5-fluoro-2-(hydroxymethyl)-3-[2-[5-(morpholinomethyl)-2-pyridyl]-1H-pyrrolo[2,3-b]pyridin-4-yl]phenyl]-2,3-dihydro-1,4-benzoxazepin-5-one | LCMS: $m/z$ 638.1 $(M+1)^+$. $^1H$ NMR (DMSO-$d_6$, 400 MHz) $\delta$ 0.78-0.82 (m, 2H); 1.00-1.06 (m, 2H); 1.96-2.04 (m, 1H); 2.30-2.44 (m, 4H); 3.53 (s, 2H); 3.56-3.60 (m, 4H); 3.80-4.00 (m, 2H); 4.15-4.28 (m, 1H); 4.30-4.45 (m, 2H); 4.50-4.60 (m, 1H); 4.82 (bs, 1H); 6.77 (s, 1H); 6.84 (dd, $J_1$ = 0.9 Hz, $J_2$ = 11.3 Hz, 1H); 6.93 (d, $J$ = 1.5 Hz, 1H); 7.20-7.24 (aromatics, 1H); 7.34 (dd, $J_1$ = 3.0 Hz, $J_2$ = 9.3 Hz, 1H); 7.40 (dd, $J_1$ = 2.5 Hz, $J_2$ = 9.3 Hz, 1H); 7.79 (dd, $J_1$ = 1.9 Hz, $J_2$ = 7.8 Hz, 1H); 8.04 (d, $J$ = 8.3 Hz, 1H); 8.34 (d, $J$ = 4.9 Hz, 1H); 8.55 (d, $J$ = 1.5 Hz, 1H); 12.40 (s, 1H) | morpholine |
| 5 | 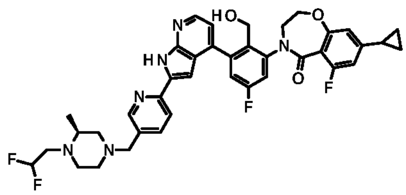 8-cyclopropyl-4-[3-[2-[5-[[(3S)-4-(2,2-difluoroethyl)-3-methyl-piperazin-1-yl] methyl]-2-pyridyl]-1H-pyrrolo[2,3-b] pyridin-4-yl]-5-fluoro-2-(hydroxymethyl) phenyl]-6-fluoro-2,3-dihydro-1,4-benzoxazepin-5-one | LCMS: $m/z$ 715.3 $(M+1)^+$. $^1H$ NMR (DMSO-$d_6$, 400 MHz) $\delta$ 0.76-0.80 (m, 2H); 0.90-0.98 (m, 2H); 0.98-1.04 (m, 3H); 1.96-2.02 (m, 1H); 2.40-2.65 (m, 7H); 2.78-3.00 (m, 2H); 3.47 (s, 2H); 3.80-4.00 (m, 2H); 4.15-4.25 (m, 1H); 4.30-4.42 (m, 2H); 4.48-4.58 (m, 1H); 4.75-4.85 (m, 1H); 6.04 (t, $J$ = 56 Hz, 1H); 6.75 (s, 1H); 6.82 (d, $J$ = 12.8 Hz, 1H); 6.90-6.95 (aromatics, 1H); 7.18-7.22 (aromatics, 1H); 7.33 (dd, $J_1$ = 2.8 Hz, $J_2$ = 9.2 Hz, 1H); 7.39 (dd, $J_1$ = 2.4 Hz, $J_2$ = 9.2 Hz, 1H); 7.72-7.78 (aromatics, 1H); 8.02 (d, $J$ = 6.8 Hz, 1H); 8.33 (d, $J$ = 4.8 Hz, 1H); 8.52 (bs, 1H); 12.36 (s, 1H) | (2S)-1-(2,2-difluoroethyl)-2-methyl-piperazine |
| 6 | 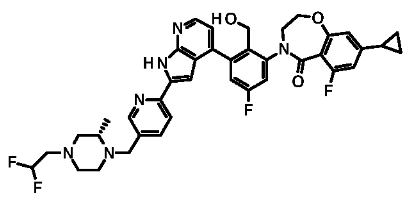 8-cyclopropyl-4-[3-[2-[5-[[(2S)-4-(2,2-difluoroethyl)-2-methyl-piperazin-1-yl] methyl]-2-pyridyl]-1H-pyrrolo[2,3-b] pyridin-4-yl]-5-fluoro-2-(hydroxymethyl) phenyl]-6-fluoro-2,3-dihydro-1,4-benzoxazepin-5-one | LCMS: $m/z$ 715.4 $(M+1)^+$. $^1H$ NMR (DMSO-$d_6$, 400 MHz) $\delta$ 0.76-0.82 (m, 2H); 1.00-1.05 (m, 2H); 1.05-1.11 (m, 3H); 1.96-2.04 (m, 1H); 2.08-2.32 (m, 3H); 2.40-2.68 (m, 6H); 3.35 (s, 2H); 3.80-4.02 (m, 2H); 4.16-4.25 (m, 1H); 4.32-4.44 (m, 2H); 4.50-4.60 (m, 1H); 4.78-4.88 (m, 1H); 6.10 (tt, $J_1$ = 4.4 Hz, $J_2$ = 56 Hz, 1H); 6.77 (s, 1H); 6.83 (d, $J$ = 11.6 Hz, 1H); 6.92 (bs, 1H); 7.20-7.25 (aromatics, 1H); 7.35 (d, $J$ = 9.2 Hz, 1H); 7.40 (dd, $J_1$ = 2.8 Hz, $J_2$ = 9.2 Hz, 1H); 7.76 (d, $J$ = 8 Hz, 1H); 8.03 (d, $J$ = 8.0 Hz, 1H); 8.34 (d, $J$ = 4.8 Hz, 1H); 8.53 (bs, 1H); 12.36 (s, 1H) | (3S)-1-(2,2-difluoroethyl)-3-methyl-piperazine |
| 7 | 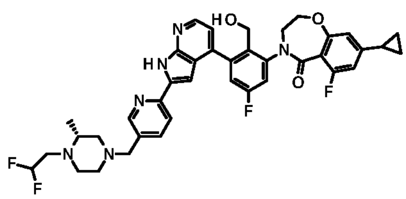 8-cyclopropyl-4-[3-[2-[5-[[(3R)-4-(2,2-difluoroethyl)-3-methyl-piperazin-1-yl] methyl]-2-pyridyl]-1H-pyrrolo[2,3-b] pyridin-4-yl]-5-fluoro-2-(hydroxymethyl) phenyl]-6-fluoro-2,3-dihydro-1,4-benzoxazepin-5-one | LCMS: $m/z$ 715.2 $(M+1)^+$. $^1H$ NMR (DMSO-$d_6$, 400 MHz) $\delta$ 0.76-0.82 (m, 2H); 0.92-1.05 (m, 5H); 1.86-1.94 (m, 1H); 1.96-2.04 (m, 1H); 2.15-2.22 (m, 1H); 2.42-2.64 (m, 5H); 2.70-3.02 (m, 2H); 3.49 (s, 2H); 3.70-4.02 (m, 2H); 4.18-4.30 (m, 1H); 4.32-4.44 (m, 2H); 4.50-4.60 (m, 1H); 4.76-4.88 (m, 1H); 6.05 (tt, $J_1$ = 4.4 Hz, $J_2$ = 56 Hz, 1H); 6.77 (s, 1H); 6.84 (d, $J$ = 11.2 Hz, 1H); 6.93 (bs, 1H); 7.20-7.24 (aromatics, 1H); 7.35 (d, $J$ = 8.8 Hz, 1H); 7.40 (d, $J$ = 8.4 Hz, 1H); 7.76 (d, $J$ = 7.6 Hz, 1H); 8.04 (d, $J$ = 8.0 Hz, 1H); 8.34 (d, $J$ = 4.8 Hz, 1H); 8.53 (bs, 1H); 12.36 (s, 1H) | (2R)-1-(2,2-difluoroethyl)-2-methyl-piperazine |

**Reference Example 8: Synthesis of 8-cyclopropyl-6-fluoro-4-[5-fluoro-2-(hydroxymethyl)-3-[2-[1-(oxetan-3-yl)-3,6-dihydro-2H-pyridin-4-yl]-1H-pyrrolo[2,3-b]pyridin-4-yl]phenyl]-2,3-dihydro-1,4-benzoxazepin-5-one**

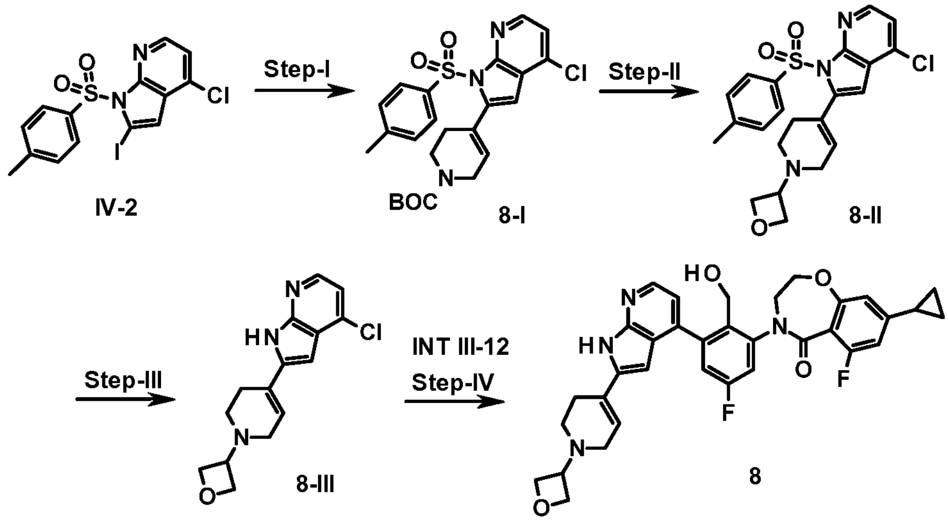


**Step-I: Synthesis of tert-butyl 4-[4-chloro-1-(p-tolylsulfonyl)pyrrolo[2,3-b]pyridin-2-yl]-3,6-dihydro-2H-pyridine-1-carboxylate (8-1):** To a solution of **IV-2** (13.4 g, 25.4 mmol) and tert-butyl 4-(4,4,5,5-tetramethyl-1,3,2-dioxaborolan-2-yl)-3,6-dihydro-2H-pyridine-1-carboxylate (7.9 g, 25.4 mmol) in 1,4-dioxane (150 mL) was added an aqueous solution of $K_2CO_3$ (7 g in 30 mL water, 50.8 mmol) and Argon was purged through it for 15 min. Finally, $PdCl_2$(dppf)-DCM adduct (2 g, 2.54 mmol) was added to the reaction mixture and purging was continued for 10 min more. It was, and then stirred at 100 °C for 5-8 h and after completion; it was cooled to room temperature. Solvent was then removed under reduced pressure and the residue was diluted with water (100 mL). Extraction was carried out using EtOAc (150 mL x 2); the combined organic layers were washed with brine (100 mL), dried over anhydrous $Na_2SO_4$, filtered and evaporated under reduced pressure to give crude product, which was further purified by silica gel column chromatography (10% acetone in hexanes) to give title compound **8-I** (11.4 g, 75%). LCMS: *m/z* 488 $(M+1)^+$.

**Step-II: Synthesis of 4-chloro-2-[1-(oxetan-3-yl)-3,6-dihydro-2H-pyridin-4-yl]-1-(p-tolylsulfonyl)pyrrolo[2,3-b]pyridine (8-II):** Intermediate **8-I** (2.5 g, 5.12 mmol) was dissolved in $CH_2Cl_2$ (25 mL), to which trifluoroacetic acid (10 mL, excess) was added at 0 °C and the reaction mixture was stirred at room temperature for 2 h. Solvents were removed under reduced pressure (at 35 °C) and saturated $NaHCO_3$ solution (70 mL) was added to it (pH should be alkaline). Extraction was carried out using $CH_2Cl_2$ (50 mL x 2); the combined organic layers were washed with water (60 mL), brine (60 mL), dried over anhydrous $Na_2SO_4$, filtered and concentrated under reduced pressure to provide corresponding Boc-deprotected intermediate (1.3 g), which was carried forward for the next step without any purification. To the solution of this intermediate (1.3 g, 3.35 mmol) and 3-oxetanone (1.2 g, 16.75 mmol) in MeOH (30 mL) was added $ZnCl_2$ (2.28 g, 16.7 mmol) at 10 °C and it was stirred for 1 h. Finally, $NaBH_3CN$ (0.63 g, 10 mmol) was added to it and the reaction mixture was stirred for 3-4 h (TLC monitoring). After completion of the reaction, MeOH was removed under reduced pressure and the residue was diluted using EtOAc (70 mL). It was washed with water (50 mL), brine (50 mL), dried over anhydrous $Na_2SO_4$, filtered and concentrated under reduced pressure. The residue obtained was washed with hexane (20 mL x 2) to provide desired compound **8-II** (1 g, 44% yield for two steps), which was used without any purification. LCMS: *m/z* 444 $(M+1)^+$.

**Step-III: Synthesis of 4-chloro-2-[1-(oxetan-3-yl)-3,6-dihydro-2H-pyridin-4-yl]-1H-pyrrolo[2,3-b]pyridine (8-III):** To a solution of **8-II** (1 g, 2.25 mmol) in acetone (12 mL) was added an aqueous solution of NaOH (0.45 g in 8 mL $H_2O$, 11.26 mmol) and the reaction mixture was stirred at 70 °C for 16 h. After completion of the reaction, solvent was removed under reduced pressure and the residue was diluted using water (50 mL). Extraction was carried out using $CH_2Cl_2$ (30 mL x 3) and the combined organic layers were dried over anhydrous $Na_2SO_4$, filtered and concentrated under reduced pressure. The residue obtained was purified using silica gel column chromatography (2-4% MeOH in $CH_2Cl_2$) to provide desired compound **8-III** (0.55 g, 84% yield). LCMS: *m/z* 290 $(M+1)^+$.

**Step-IV: Synthesis of 8-cyclopropyl-6-fluoro-4-[5-fluoro-2-(hydroxymethyl)-3-[2-[1-(oxetan-3-yl)-3,6-dihydro-2H-pyridin-4-yl]-1H-pyrrolo[2,3-b]pyridin-4-yl]phenyl]-2,3-dihydro-1,4-benzoxazepin-5-one:** To a solution of **8-III** (0.7 g, 2.42 mmol) and **III-12** (1.6 g, 3.15 mmol) in dioxane (15 mL) was added an aqueous solution of

2.5 M $Na_2CO_3$ (4.85 mL, 12.1 mmol) and Argon was purged through it for 15 min. Finally, $Pd(PPh_3)_4$ (0.28 g, 0.242 mmol) was added to the reaction mixture and purging was continued for 10 min more. It was, and then stirred at 100 °C for 16 h and then it was cooled to room temperature. Solvent was then removed under reduced pressure and the residue was diluted with water (50 mL). Extraction was carried out using EtOAc (50 mL x 2); the combined organic layers were washed with brine (50 mL), dried over anhydrous $Na_2SO_4$, filtered and evaporated under reduced pressure to give crude product, which was further purified by column chromatography (2-4% MeOH in $CH_2Cl_2$) followed by acetonitrile washing to provide the title compound **8** (0.465 g, 32%). LCMS: *m/z* 599.2 $(M+1)^+$. $^1H$ NMR (DMSO-$d_6$,,400 MHz) $\delta$ 0.77-0.82 (m, 2H); 1.00-1.09 (m, 2H); 1.96-2.06 (m, 1H); 2.45-2.50 (m, 4H); 3.03 (bs, 2H); 3.50-3.60 (m, 1H); 3.70-4.00 (m, 2H); 4.10-4.40 (m, 3H); 4.51 (t, $J$ = 6.4 Hz, 2H); 4.57 (t, $J$ = 6.4 Hz, 2H); 4.50-4.60 (m, 1H); 4.76-4.90 (m, 1H); 6.21 (s, 1H); 6.48-6.54 (aromatics, 1H); 6.77 (s, 1H); 6.83 (d, $J$ = 11.2 Hz, 1H); 7.12-7.18 (aromatics, 1H); 7.29 (dd, $J_1$ = 2.8 Hz, $J_2$ = 8.8 Hz, 1H); 7.36 (dd, $J_1$ = 2.8 Hz, $J_2$ = 9.2 Hz, 1H); 8.24 (d, $J$ = 5.2 Hz, 1H); 11.90 (bs, 1H) Using similar reaction sequence and conditions as described for the synthesis of 5, following compounds were also synthesized (Table-5).

**Table-5** (Contains Reference Examples 9 and 10)

| No | Structure/IUPAC name | Characterization | INT used |
|---|---|---|---|
| **9** | 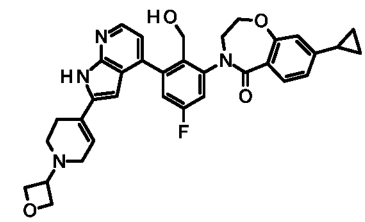 8-cyclopropyl-4-[5-fluoro-2-(hydroxymethyl)-3-[2-[1-(oxetan-3-yl)-3,6-dihydro-2H-pyridin-4-yl]-1H-pyrrolo[2,3-b]pyridin-4-yl]phenyl]-2,3-dihydro-1,4-benzoxazepin-5-one | LCMS: *m/z* 581.2 $(M+1)^+$. $^1H$ NMR (DMSO-$d_6$,, 400 MHz) $\delta$ 0.70-0.80 (m, 2H); 0.98-1.05 (m, 2H); 1.92-2.02 (m, 1H); 2.45-2.50 (m, 4H); 3.03 (bs, 2H); 3.50-3.60 (m, 1H); 3.88-4.00 (m, 2H); 4.10-4.35 (m, 2H); 4.40-4.65 (m, 6H); 4.70-4.80 (m, 1H); 6.21 (s, 1H); 6.51 (bs, 1H); 6.80 (s, 1H); 6.87 (d, $J$ = 8.0 Hz, 1H); 7.12-7.18 (aromatics, 1H); 7.26 (d, $J$ = 8.0 Hz, 1H); 7.38 (d, $J$ = 8.8 Hz, 1H); 7.68 (d, $J$ = 8.4 Hz, 1H); 8.20-8.24 (aromatics, 1H); 11.90 (bs, 1H) | III-11 |
| **10** | 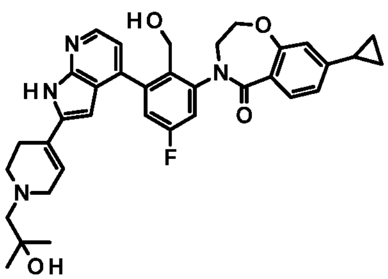 8-cyclopropyl-4-[5-fluoro-2-(hydroxymethyl)-3-[2-[1-(2-hydroxy-2-methyl-propyl)-3,6-dihydro-2H-pyridin-4-yl]-1H-pyrrolo[2,3-b]pyridin-4-yl]phenyl]-2,3-dihydro-1,4-benzoxazepin-5-one | LCMS: *m/z* 597.3 $(M+1)^+$. $^1H$ NMR (DMSO-$d_6$,,400 MHz) $\delta$ 0.70-0.77 (m, 2H); 0.99-1.03 (m, 2H); 1.10 (s, 6H); 1.92-2.00 (m, 1H); 2.30 (bs, 2H); 2.40-2.50 (m, 2H); 2.74 (t, $J$ = 5.6 Hz, 2H); 3.26 (bs, 2H); 3.92 (t, $J$ = 4.4 Hz, 2H); 4.10-4.30 (m, 3H); 4.50-4.60 (m, 2H); 4.70-4.80 (m, 1H); 6.19 (s, 1H); 6.46-6.50 (aromatics, 1H); 6.80 (d, $J$ = 1.6 Hz, 1H); 6.88 (dd, $J_1$ = 1.2 Hz, $J_2$ = 8.4 Hz, 1H); 7.12-7.18 (aromatics, 1H); 7.26 (dd, $J_1$ 2.8 Hz, $J_2$ = 8.8 Hz, 1H); 7.38 (dd, $J_1$ = 2.8 Hz, $J_2$ = 8.8 Hz, 1H); 7.68 (d, $J$ = 8.4 Hz, 1H); 8.23 (d, $J$ = 4.8 Hz 1H); 11.82 (bs, 1H) | III-11 |

**Example 11: Synthesis of 8-cyclopropyl-6-fluoro-4-[5-fluoro-2-(hydroxymethyl)-3-[2-[5-[[1-(oxetan-3-yl)-4-piperidyl]oxy]-2-pyridyl]-1H-pyrrolo[2,3-b]pyridin-4-yl]phenyl]-2,3-dihydro-1,4-benzoxazepin-5-one**

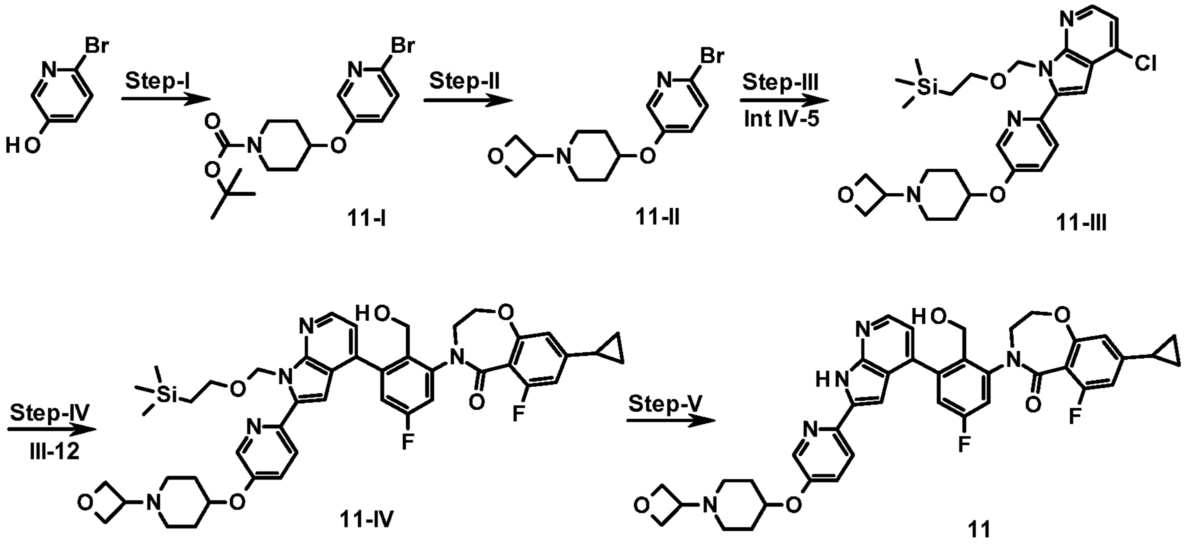


**Step-I: tert-butyl 4-[(6-bromo-3-pyridyl)oxy]piperidine-1-carboxylate (11-I):** To a solution of 6-bromo-3-hydroxypyridine (3 g, 17.2 mmol) and 1-Boc-4-hydroxypiperidine (3.5 g, 17.24 mmol) in anhydrous THF (40 mL) was added triphenylphosphine (6.8 g, 25.86 mmol) under Ar atmosphere. Finally, DEAD (4 mL, 25.86 mmol) was added to it at 0 °C and the reaction mixture was warmed to room temperature over 16 h. After completion of the reaction, saturated $NaHCO_3$ solution (70 mL) was added to it and extraction was carried out using EtOAc (30 mL x 2). The combined organic layers were washed with water (100 mL), brine (100 mL), dried over anhydrous $Na_2SO_4$, filtered and concentrated under reduced pressure. The residue was purified using silica gel column chromatography (15-20% EtOAc in hexane as eluent) to provide **11-I** (3 g, 47% yield). LCMS: *m/z*; 301 $[(M\text{-tert-Bu})+1]^+$

**Step-II: 2-bromo-5-[[1-(oxetan-3-yl)-4-piperidyl]oxy]pyridine (11-II):** As described for the synthesis of **11-II.** (40% overall yield) LCMS: *m/z*; 313 $(M+1)^+$.

**Step-III: 2-[[4-chloro-2-[5-[[1-(oxetan-3-yl)-4-piperidyl]oxy]-2-pyridyl]pyrrolo[2,3-b]pyridin-1-yl]methoxy]ethyl-trimethyl-silane (11-III):** This intermediate was synthesisized using intermediates **11-II** and **IV-5**, following procedure described for the synthesis of **XI-3.** (69% yield). LCMS: *m/z*; 515.2 $(M+1)^+$.

**Step-IV: 8-cyclopropyl-6-fluoro-4-[5-fluoro-2-(hydroxymethyl)-3-[2-[5-[[1-(oxetan-3-yl)-4-piperidyl]oxy]-2-pyridyl]-1-(2-trimethylsilylethoxymethyl)pyrrolo[2,3-b]pyridin-4-yl]phenyl]-2,3-dihydro-1,4-benzoxazepin-5-one (11-IV):** This intermediate was synthesized using intermediates **11-III** and **III-12**, following procedure described for the synthesis of **1-I** (step-I). (23% yield) LCMS: *m/z*; 824 $(M+1)^+$.

**Step-V: 8-cyclopropyl-6-fluoro-4-[5-fluoro-2-(hydroxymethyl)-3-[2-[5-[[1-(oxetan-3-yl)-4-piperidyl]oxy]-2-pyridyl]-1H-pyrrolo[2,3-b]pyridin-4-yl]phenyl]-2,3-dihydro-1,4-benzoxazepin-5-one (11):** As described for the synthesis of **1** (step-III) (16% yield). LCMS: *m/z;* 694.0 $(M+1)^+$. $^1H$ NMR (DMSO-$d_6$, 400 MHz) $\delta$ 0.74-0.82 (m, 2H); 1.00-1.10 (m, 2H); 1.60-1.72 (m, 2H); 1.94-2.04 (m, 3H); 2.06-2.14 (m, 2H); 2.50-2.60 (m, 2H); 3.40-3.44 (m, 1H); 3.80-4.00 (m, 2H); 4.16-4.30 (m, 1H); 4.30-4.45 (m, 4H); 4.50-4.60 (m, 4H); 4.75-4.85 (m, 1H); 6.77-6.82 (aromatics, 2H); 6.84 (d, *J* = 11.7 Hz, 1H); 7.18-7.22 (aromatics, 1H); 7.33 (d, *J* = 9.3 Hz, 1H); 7.38 (dd, $J_1$ = 2.5 Hz, $J_2$ = 9.3 Hz, 1H); 7.53 (dd, $J_1$ = 2.4 Hz, $J_2$ = 8.8 Hz, 1H); 8.01 (d, *J* = 8.8 Hz, 1H); 8.30 (d, *J* = 4.9 Hz, 1H); 8.33 (d, *J* = 2.4 Hz, 1H); 12.20 (bs, 1H).

The below list of examples, but not to be limited to these numbers, can also be synthesized by following the general synthesis described above:

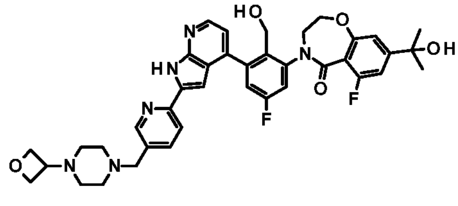

6-fluoro-4-[5-fluoro-2-(hydroxymethyl)-3-[2-[5-[[4-(oxetan-3-yl)piperazin-1-yl]methyl]-2-pyridyl]-1H-pyrrolo[2,3-b]pyridin-4-yl]phenyl]-8-(1-hydroxy-1-methyl-ethyl)-2,3-dihydro-1,4-benzoxazepin-5-one;

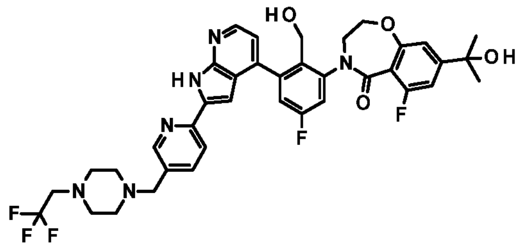

6-fluoro-4-[5-fluoro-2-(hydroxymethyl)-3-[2-[5-[[4-(2,2,2-trifluoroethyl)piperazin-1-yl]methyl]-2-pyridyl]-1H-pyrrolo[2,3-b]pyridin-4-yl]phenyl]-8-(1-hydroxy-1-methyl-ethyl)-2,3-dihydro-1,4-benzoxazepin-5-one;

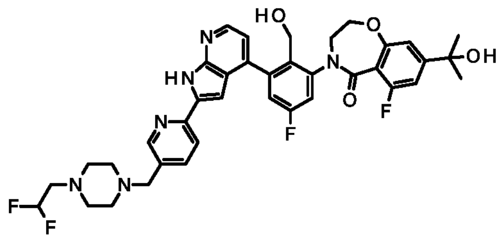

4-[3-[2-[5-[[4-(2,2-difluoroethyl)piperazin-1-yl]methyl]-2-pyridyl]-1H-pyrrolo[2,3-b]pyridin-4-yl]-5-fluoro-2-(hydroxymethyl)phenyl]-6-fluoro-8-(1-hydroxy-1-methyl-ethyl)-2,3-dihydro-1,4-benzoxazepin-5-one;

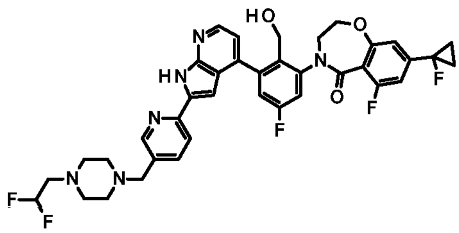

4-[3-[2-[5-[[4-(2,2-difluoroethyl)piperazin-1-yl]methyl]-2-pyridyl]-1H-pyrrolo[2,3-b]pyridin-4-yl]-5-fluoro-2-(hydroxymethyl)phenyl]-6-fluoro-8-(1-fluorocyclopropyl)-2,3-dihydro-1,4-benzoxazepin-5-one;

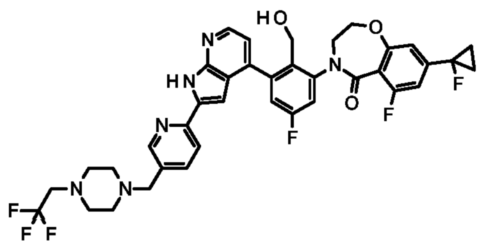

6-fluoro-8-(1-fluorocyclopropyl)-4-[5-fluoro-2-(hydroxymethyl)-3-[2-[5-[[4-(2,2,2-trifluoroethyl)piperazin-1-yl]methyl]-2-pyridyl]-1H-pyrrolo[2,3-b]pyridin-4-yl]phenyl]-2,3-dihydro-1,4-benzoxazepin-5-one;

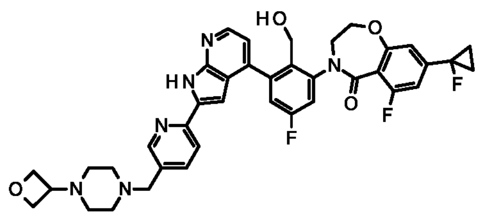

6-fluoro-8-(1-fluorocyclopropyl)-4-[5-fluoro-2-(hydroxymethyl)-3-[2-[5-[[4-(oxetan-3-yl)piperazin-1-yl]methyl]-2-pyridyl]-1H-pyrrolo[2,3-b]pyridin-4-yl]phenyl]-2,3-dihydro-1,4-benzoxazepin-5-one;

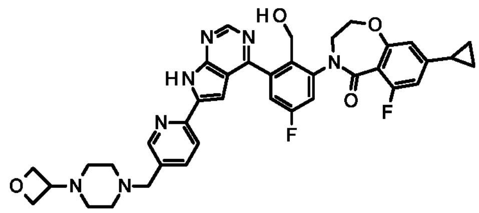

8-cyclopropyl-6-fluoro-4-[5-fluoro-2-(hydroxymethyl)-3-[6-[5-[[4-(oxetan-3-yl)piperazin-1-yl]methyl]-2-pyridyl]-7H-pyrrolo[2,3-d]pyrimidin-4-yl]phenyl]-2,3-dihydro-1,4-benzoxazepin-5-one; (Reference Example)

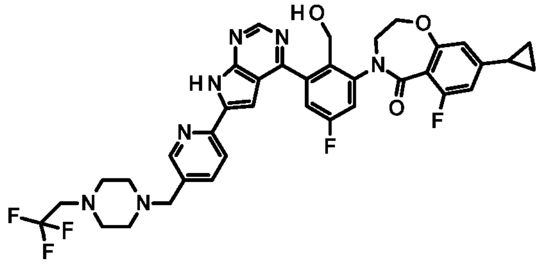

8-cyclopropyl-6-fluoro-4-[5-fluoro-2-(hydroxymethyl)-3-[6-[5-[[4-(2,2,2-trifluoroethyl)piperazin-1-yl]methyl]-2-pyridyl]-7H-pyrrolo[2,3-d]pyrimidin-4-yl]phenyl]-2,3-dihydro-1,4-benzoxazepin-5-one; (Reference Example)

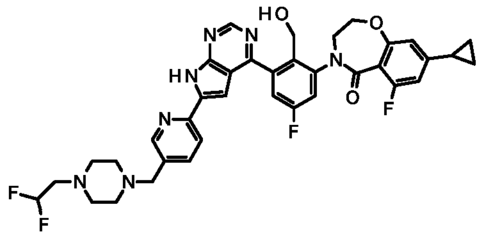

8-cyclopropyl-4-[3-[6-[5-[[4-(2,2-difluoroethyl)piperazin-1-yl]methyl]-2-pyridyl]-7H-pyrrolo[2,3-d]pyrimidin-4-yl]-5-fluoro-2-(hydroxymethyl)phenyl]-6-fluoro-2,3-dihydro-1,4-benzoxazepin-5-one; (Reference Example)

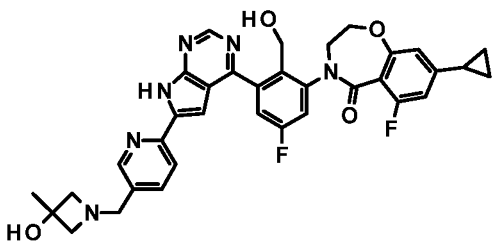

8-cyclopropyl-6-fluoro-4-[5-fluoro-2-(hydroxymethyl)-3-[6-[5-[(3-hydroxy-3-methyl-azetidin-1-yl)methyl]-2-pyridyl]-7H-pyrrolo[2,3-d]pyrimidin-4-yl]phenyl]-2,3-dihydro-1,4-benzoxazepin-5-one; (Reference Example)

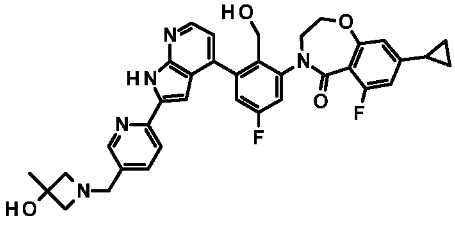

8-cyclopropyl-6-fluoro-4-[5-fluoro-2-(hydroxymethyl)-3-[2-[5-[(3-hydroxy-3-methyl-azetidin-1-yl)methyl]-2-pyridyl]-1H-pyrrolo[2,3-b]pyridin-4-yl]phenyl]-2,3-dihydro-1,4-benzoxazepin-5-one;

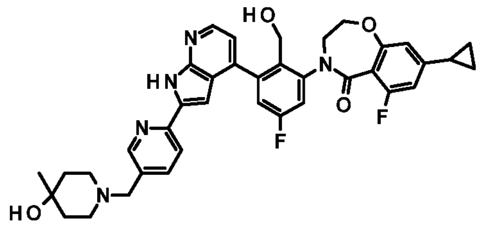

8-cyclopropyl-6-fluoro-4-[5-fluoro-2-(hydroxymethyl)-3-[2-[5-[(4-hydroxy-4-methyl-1-piperidyl)methyl]-2-pyridyl]-1H-pyrrolo[2,3-b]pyridin-4-yl]phenyl]-2,3-dihydro-1,4-benzoxazepin-5-one;

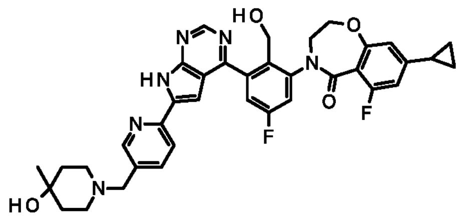

8-cyclopropyl-6-fluoro-4-[5-fluoro-2-(hydroxymethyl)-3-[6-[5-[(4-hydroxy-4-methyl-1-piperidyl)methyl]-2-pyridyl]-7H-pyrrolo[2,3-d]pyrimidin-4-yl]phenyl]-2,3-dihydro-1,4-benzoxazepin-5-one; (Reference Example)

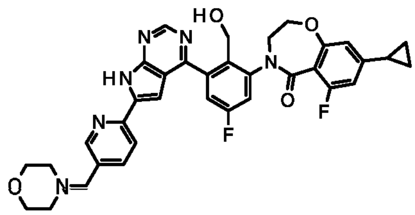

8-cyclopropyl-6-fluoro-4-[5-fluoro-2-(hydroxymethyl)-3-[6-[5-(morpholinomethyl)-2-pyridyl]-7H-pyrrolo[2,3-d]pyrimidin-4-yl]phenyl]-2,3-dihydro-1,4-benzoxazepin-5-one; (Reference Example)

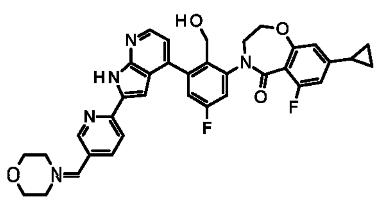

8-cyclopropyl-6-fluoro-4-[5-fluoro-2-(hydroxymethyl)-3-[2-[5-(morpholinomethyl)-2-pyridyl]-1H-pyrrolo[2,3-b]pyridin-4-yl]phenyl]-2,3-dihydro-1,4-benzoxazepin-5-one; (Reference Example)

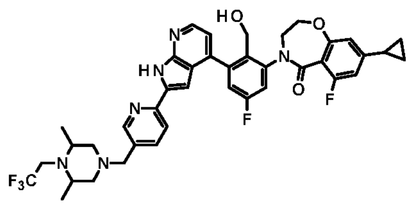

8-cyclopropyl-4-[3-[2-[5-[[3,5-dimethyl-4-(2,2,2-trifluoroethyl)piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;

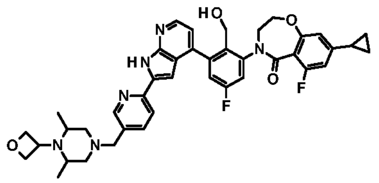

8-cyclopropyl-4-[3-[2-[5-[[3,5-dimethyl-4-(oxetan-3-yl)piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;

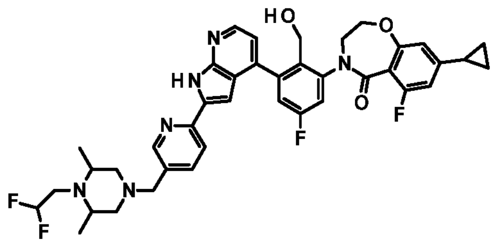

8-cyclopropyl-4-[3-[2-[5-[[4-(2,2-difluoroethyl)-3,5-dimethyl-piperazin-1 -yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;

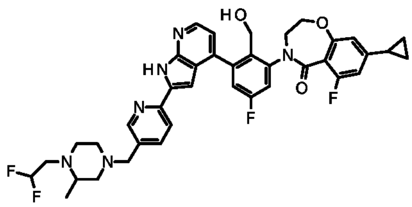

8-cyclopropyl-4-[3-[2-[5-[[4-(2,2-difluoroethyl)-3-methyl-piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;

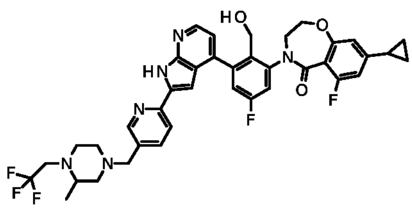

8-cyclopropyl-6-fluoro-4-[5-fluoro-2-(hydroxymethyl)-3-[2-[5-[[3-methyl-4-(2,2,2-trifluoroethyl)piperazin-1-yl]methyl]-2-pyridyl]-1H-pyrrolo[2,3-b]pyridin-4-yl]phenyl]-2,3-dihydro-1,4-benzoxazepin-5-one;

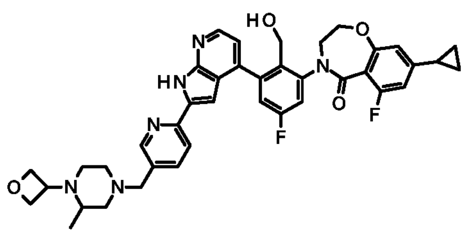

8-cyclopropyl-6-fluoro-4-[5-fluoro-2-(hydroxymethyl)-3-[2-[5-[[3-methyl-4-(oxetan-3-yl)piperazin-1-yl]methyl]-2-pyridyl]-1H-pyrrolo[2,3-b]pyridin-4-yl]phenyl]-2,3-dihydro-1,4-benzoxazepin-5-one;

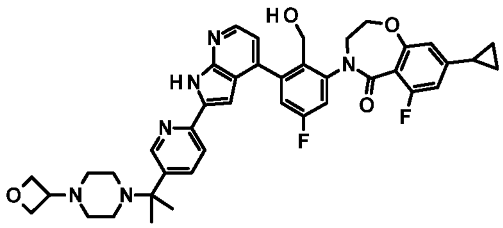

8-cyclopropyl-6-fluoro-4-[5-fluoro-2-(hydroxymethyl)-3-[2-[5-[1-methyl-1-[4-(oxetan-3-yl)piperazin-1-yl]ethyl]-2-pyridyl]-1H-pyrrolo[2,3-b]pyridin-4-yl]phenyl]-2,3-dihydro-1,4-benzoxazepin-5-one;

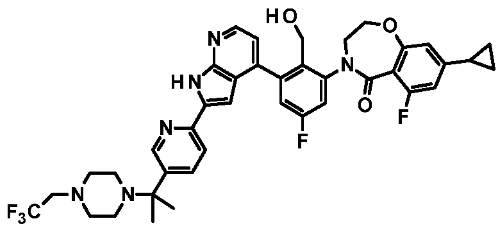

8-cyclopropyl-6-fluoro-4-[5-fluoro-2-(hydroxymethyl)-3-[2-[5-[1-methyl-1-[4-(2,2,2-trifluoroethyl)piperazin-1-yl]ethyl]-2-pyridyl]-1H-pyrrolo[2,3-b]pyridin-4-yl]phenyl]-2,3-dihydro-1,4-benzoxazepin-5-one;

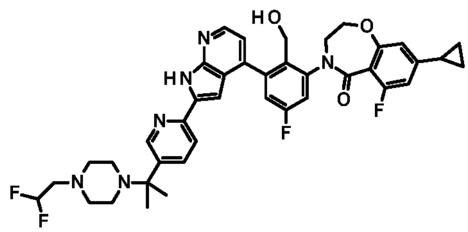

8-cyclopropyl-4-[3-[2-[5-[1-[4-(2,2-difluoroethyl)piperazin-1-yl]-1-methyl-ethyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;

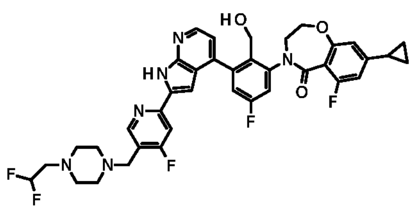

8-cyclopropyl-4-[3-[2-[5-[[4-(2,2-difluoroethyl)piperazin-1-yl]methyl]-4-fluoro-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;

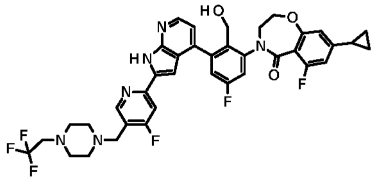

8-cyclopropyl-6-fluoro-4-[5-fluoro-3-[2-[4-fluoro-5-[[4-(2,2,2-trifluoroethyl)piperazin-1-yl]methyl]-2-pyridyl]-1H-pyrrolo[2,3-b]pyridin-4-yl]-2-(hydroxymethyl)phenyl]-2,3-dihydro-1,4-benzoxazepin-5-one;

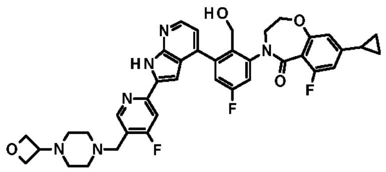

8-cyclopropyl-6-fluoro-4-[5-fluoro-3-[2-[4-fluoro-5-[[4-(oxetan-3-yl)piperazin-1-yl]methyl]-2-pyridyl]-1H-pyrrolo[2,3-b]pyridin-4-yl]-2-(hydroxymethyl)phenyl]-2,3-dihydro-1,4-benzoxazepin-5-one;

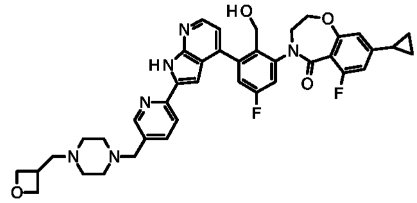

8-cyclopropyl-6-fluoro-4-[5-fluoro-2-(hydroxymethyl)-3-[2-[5-[[4-(oxetan-3-ylmethyl)piperazin-1-yl]methyl]-2-pyridyl]-1H-pyrrolo[2,3-b]pyridin-4-yl]phenyl]-2,3-dihydro-1,4-benzoxazepin-5-one;

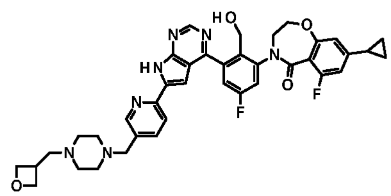

8-cyclopropyl-6-fluoro-4-[5-fluoro-2-(hydroxymethyl)-3-[6-[5-[[4-(oxetan-3-ylmethyl)piperazin-1-yl]methyl]-2-pyridyl]-7H-pyrrolo[2,3-d]pyrimidin-4-yl]phenyl]-2,3-dihydro-1,4-benzoxazepin-5-one (Reference Example)

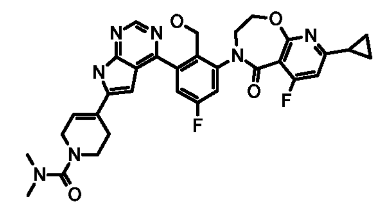

4-[4-[3-(8-cyclopropyl-6-fluoro-5-oxo-2,3-dihydropyrido[3,2-f][1,4]oxazepin-4-yl)-5-fluoro-2-(hydroxymethyl)phenyl]-7H-pyrrolo[2,3-d]pyrimidin-6-yl]-N,N-dimethyl-3,6-dihydro-2H-pyridine-1-carboxamide (Reference Example);

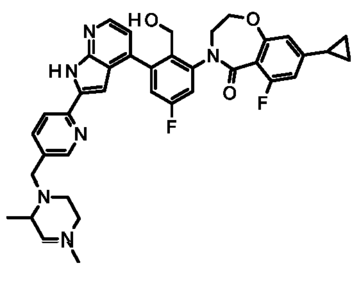

8-cyclopropyl-4-[3-[2-[5-[(2,4-dimethylpiperazin-1-yl)methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one; (Reference Example)

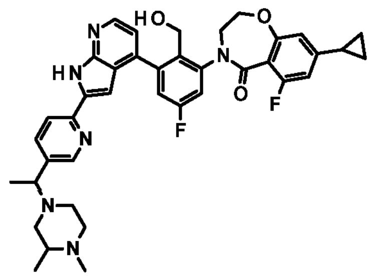

8-cyclopropyl-4-[3-[2-[5-[1-(3,4-dimethylpiperazin-1-yl)ethyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one; (Reference Example)

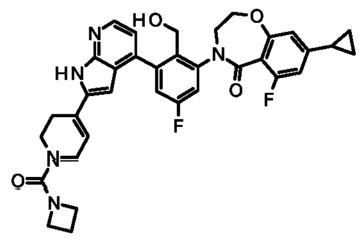

4-[3-[2-[1-(azetidine-1-carbonyl)-3,6-dihydro-2H-pyridin-4-yl]-1H-pyrrolo[2,3-b]pyridin-4-yl]-5-fluoro-2-(hydroxymethyl)phenyl]-8-cyclopropyl-6-fluoro-2,3-dihydro-1,4-benzoxazepin-5-one; (Reference Example)

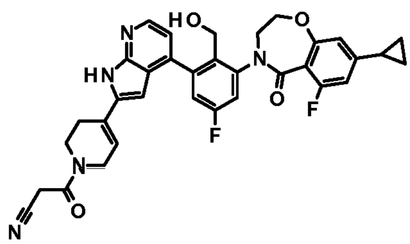

3-[4-[4-[3-(8-cyclopropyl-6-fluoro-5-oxo-2,3-dihydro-1,4-benzoxazepin-4-yl)-5-fluoro-2-(hydroxymethyl)phenyl]-1H-pyrrolo[2,3-b]pyridin-2-yl]-3,6-dihydro-2H-pyridin-1-yl]-3-oxo-propanenitrile (Reference Example).

**Biological activity**

**Recombinant Human Bruton's tyrosine kinase (Btk) inhibition assay.**

Full length human BTK (hBTK, Genbank#NM_000061.2) was cloned into pFastBac-HTB vector (Invitrogen, cat#10359-016). hBTK gene was expressed with N'-terminal 6XHis tag in Sf9 cells using Bac-to-Bac (R) Baculovirus expression system (Invitrogen, cat#10359-016) and the recombinant protein was purified by Ni-NTA method. The elution buffer for hBTK consisted of 50 mM Hepes pH 8.0, 300 mM NaCl, 10% glycerol, 1 mM dithiothreitol and 200 mM imidazole.

The inhibition assays were performed using the generic tyrosine kinase assay kit from Cis Bio (HTRF® KinEASE - TK™ # 62TK0PEB). The assay is based on the principle of fluorescence resonance energy transfer (FRET) between the Europium Cryptate labeled anti-phosphotyrosine antibody and streptavidin labeled fluorophore, XL-665. In an uninhibited kinase reaction the biotinylated peptide substrate (Tk-Biotin) gets phosphorylated at an intermediate tyrosine site which is captured by anti-phosphotyrosine antibody which eventually acts as a FRET donor. The streptavidin labeled XL-665 binds strongly to the Tk-Biotin substrate and act as a FRET acceptor. The resultant FRET signal is measured at 665 nm. A ratio of this specific fluorescence (665nm) to the background fluorescence (620nm), conferred by the free antibody, is directly proportional to the kinase activity. Inhibition of the kinase activity by a pharmacological intervention such as a Btk inhibitor of the present disclosure will result into decreased FRET signal.

The Btk inhibition assays were performed using 20 ng/ml of rhBtk in a final volume of 10μl reaction buffer containing 77 μM ATP and 1 μM TK-Biotin Substrate in 50 mM HEPES pH 7.0, 5mM $MgCl_2$, 1mM DTT, 0.01% Tween 20 and 50nM SEB®; with a range of concentrations of the inhibitors. The kinase reaction was carried out at the $K_M$ of ATP (77 μM) as well as that of Tk-Biotin substrate (1 μM). After 90 minutes, the reaction was stopped by addition of

SA-XL665 at a concentration of 62.5 nM in HTRF® detection buffer containing EDTA. Finally 5 μL of TK antibody-cryptate was added to the reaction and the plate was incubated for additional 60 minutes. The fluorescence was read using the FlexStation (Molecular Devices) with excitation at 340 nm and emissions at 620nm (for Cryptate) and 665nm (for FRET). A ratio of the 2 emissions was calculated for each well and expressed as follows:

$$\text{Specific signal} = \text{Ratio (Sample)} - \text{Ratio (Negative)}$$

$$\text{Ratio} = (665\text{nm}/620\text{nm}) \times 10^4$$

$$\text{Mean Ratio} = \sum \text{Ratio} / 3 \text{ (triplicates)}$$

For each data point, % inhibition is calculated based on uninhibited reaction which is considered as 100% activity over no enzyme or substrate controls. Dose response data is then fit using a four parameter logistic equation using GraphPad Prism 5 software to determine inhibition constant 50($IC_{50}$).

Compounds of present disclosure are considered to be active if $IC_{50}$ of the recombinant human Btk (hBtk) enzyme inhibition is below 10 μM, and more specifically below 1.0 μM.

**Inhibition of B cell activation as measured by CD69 expression in mouse splenocytes:**

To test the effect of BTK inhibitors in suppressing B-cell receptor (BCR) mediated activation of B cells, we developed a flow cytometry based assay to analyze CD69 expression in B cell population among total splenocyte from the mouse. C57/BL6 mouse spleens were dissected out and splenocytes were isolated by mincing the tissue with a syringe plunger followed by RBC lysis (0.85% ammonium chloride, (Qualigens,Cat #21405) and several washes in PBS. Finally, cell were cultured in culture medium [RPMI medium (Sigma, Cat R6504) with 10% FBS (Gibco - Cat #10270).

Test compounds were diluted half log starting from 2 mM in 100% DMSO (Sigma, cat#D2650) (200X). $1 \times 10^5$ splenocytes were incubated with 1 μL of diluted test compounds in 200 μL culture medium in a U-bottom 96 well plate (Greiner) at 37°C with 5% $CO_2$ for 30 minutes. Control wells received 1 μL of 100% DMSO without any compound. The cells were stimulated with 3 μg/mL goat anti-mouse IgM (Sigma, cat#M8644) and incubated for 18-20 hours. After the stimulation, cells were washed in cold PBS with 1% FBS and stained with 1:100 dilution of anti-CD69-PE (eBioscience, cat#12-0691-83) and anti-CD19-PE.Cy5 (eBioscience, cat#15-0193-83) for 1 hour on ice. Then the cells were washed 3 times with PBS with 1% FBS and analyzed by Guava Easycyte Mini flow cytometer system (Millipore). Flow cytometry data were analyzed using Weasel software (WEHI, Melbourne). B cells were gated using CD19 marker. Expression of CD69 was analyzed as percentage of CD69 +ve cells within CD19. Percent activity for each data point was calculated using following formula:

$$\%\ \text{Activity} = 100 \times \frac{(\text{Experimental - No stimulation control})}{(\text{No compound control - No stimulation control})}$$

Percentage activity was fit using sigmoid dose response with variable slope logistic equation in GraphPad Prism 5 software to determine $IC_{50}$.

Compounds of present disclosure are considered to be active in the B-cell specific functional assay (inhibition of CD69 expression) if $IC_{50}$ values are less than 10 μM.

**Inhibition of B cell activation as measured by CD69 expression in Human whole blood**

To test the effect of BTK inhibitors in suppressing B-cell receptor (BCR) mediated activation of B cells, we developed a flow cytometry based assay to analyze CD69 expression in B cell population in human whole blood. Freshly collected heparinized human whole blood (60 μL/well) was dispensed in 96-well U bottom plate (Greiner), treated with the test compound at a concentrations ranging from 10 μM to 0.3 nM (half log dilutions). 0.5% DMSO (Sigma, cat#D2650) was used as vehicle control. The whole blood was incubated with compounds for 1 hour, followed by stimulation with anti-IgD antibody (Goat Anti-Human IgD Antibody - affinity purified, Bethyl laboratories, cat#A80-106A) at a final concentration of 30 μg/mL at 37°C and 5% $CO_2$ for 4 hours. Subsequently, 45 μL blood was transferred to 1.5 ml microcentrifuge tubes and 1.2 μL of anti-CD20-PE.Cy5 (BD Biosciences, cat#561761)and 1.6 μL of anti-CD69-PE (eBioscience, cat#12-0691-83) antibody were added and mixed properly by gently tapping the tubes. Blood was stained for 20 minutes at room temperature (RT). After antibody incubation, 1 mL pre-warmed 1X Lyse/Fix solution (BD Biosciences, cat#558049) was added and cells were incubated at RT for 10 minutes. Then the tubes were centrifuged at 450xg for 5 minutes at RT and supernatant was removed. Pellet was disrupted by gentle tapping, cells were washed with 1 mL freshly prepared wash buffer and centrifuged at 450xg for 5 minutes at RT. Supernatant was removed completely before resuspending cells in 200 μl of wash buffer and proceeded for acquisition in Guava Easy Cyte Mini instrument. Acquisition was done with following laser settings: FSC Gain: X 16, FSC fine gain 100%, SSC: 893V, GRN: 663V, YLW: 649V, RED: 802V, Threshold Parameters Offset: FSC 0.73 V Threshold 300 units. The cells were diluted in such a way so that cell conc. should not exceed 500 cells/μL and at least 5000 cells were acquired. Viable lymphocytes were gated from the forward and side scatter profile of cells and B cells were sub-gated based on CD20-PE.Cy5 staining. Percent of CD69-PE positive cells were calculated among B lymphocyte sub-gate using Weasel software. Percent activity for each data point was calculated using following formula:

$$\%\ \text{Activity} = 100\ \text{X}\ \frac{(\text{Experimental - No stimulation control})}{(\text{No compound control - No stimulation control})}$$

Percentage activity was fit using sigmoid dose response with variable slope logistic equation in GraphPad Prism 5 software to determine $IC_{50}$.

Compounds of present disclosure are considered to be active in the B-cell specific functional assay (inhibition of CD69 expression) if $IC_{50}$ values are less than 10 μM.

Data for representative compounds of the present disclosure are given below in Table 6:

Table 6: $IC_{50}$ values for representative compounds in biological assays with recombinant *h*BTK, and B cell activation in mouse spleenocytes and human whole blood:

| Example No | $IC_{50}$ (nM) in recombinant *h*BTK assay | $IC_{50}$ (nM) in B cell activation assay in Mouse spleenocytes | $IC_{50}$ (nM) in B cell activation assay in human whole blood |
|---|---|---|---|
| 1 | 1.3 | 2.7 | 16.5 |
| 2 | 1.9 | 2.7 | 55.9 |
| 3 | 0.65 | 2.4 | 54.2 |
| 4 | 0.55 | - | - |
| 5 | 3 | - | - |
| 6 | 2.7 | - | - |
| 7 | 1.8 | - | - |
| 8 * | 1.3 | 9 | 124.4 |
| 9 * | 2.5 | 47 | - |
| 10 * | 4 | 27 | - |
| 11 | 1.15 | - | - |
| *indicates reference example | | | |

## Claims

1. A compound or its tautomers, polymorphs, stereoisomers, solvates, co-crystals or a pharmaceutically acceptable salt thereof, wherein the compound is selected from the group consisting of:

   8-cyclopropyl-6-fluoro-4-[5-fluoro-2-(hydroxymethyl)-3-[2-[5-[[4-(oxetan-3-yl)piperazin-1-yl]methyl]-2-pyridyl]-1H-pyrrolo[2,3-b]pyridin-4-yl]phenyl]-2,3-dihydro-1,4-benzoxazepin-5-one;

8-cyclopropyl-6-fluoro-4-[5-fluoro-2-(hydroxymethyl)-3-[2-[5-[[4-(2,2,2-trifluoroethyl)piperazin-1-yl]methyl]-2-pyridyl]-1H-pyrrolo[2,3-b]pyridin-4-yl]phenyl]-2,3-dihydro-1,4-benzoxazepin-5-one;
8-cyclopropyl-4-[3-[2-[5-[[4-(2,2-difluoroethyl)piperazin-l-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-6-fluoro-4-[5-fluoro-2-(hydroxymethyl)-3-[2-[5-(morpholinomethyl)-2-pyridyl]-1H-pyrrolo[2,3-b]pyridin-4-yl]phenyl]-2,3-dihydro-1,4-benzoxazepin-5-one;
8-cyclopropyl-4-[3-[2-[5-[[(3S)-4-(2,2-difluoroethyl)-3-methyl-piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-4-[3-[2-[5-[[(2S)-4-(2,2-difluoroethyl)-2-methyl-piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-4-[3-[2-[5-[[(3R)-4-(2,2-difluoroethyl)-3-methyl-piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-6-fluoro-4-[5-fluoro-2-(hydroxymethyl)-3-[2-[5-[[1-(oxetan-3-yl)-4-piperidyl]oxy]-2-pyridyl]-1H-pyrrolo[2,3-b]pyridin-4-yl]phenyl]-2,3-dihydro-1,4-benzoxazepin-5-one;
6-fluoro-4-[5-fluoro-2-(hydroxymethyl)-3-[2-[5-[[4-(oxetan-3-yl)piperazin-1-yl]methyl]-2-pyridyl]-1H-pyrrolo[2,3-b]pyridin-4-yl]phenyl]-8-(1-hydroxy-1-methylethyl)-2,3-dihydro-1,4-benzoxazepin-5-one;
6-fluoro-4-[5-fluoro-2-(hydroxymethyl)-3-[2-[5-[[4-(2,2,2-trifluoroethyl)piperazin-1-yl]methyl]-2-pyridyl]-1H-pyrrolo[2,3-b]pyridin-4-yl]phenyl]-8-(1-hydroxy-1 -methylethyl)-2,3-dihydro-1,4-benzoxazepin-5-one;
4-[3-[2-[5-[[4-(2,2-difluoroethyl)piperazin-1-yl]methyl]-2-pyridyl]-1H-pyrrolo[2,3-b]pyridin-4-yl]-5-fluoro-2-(hydroxymethyl)phenyl]-6-fluoro-8-(1-hydroxy-1-methylethyl)-2,3-dihydro-1,4-benzoxazepin-5-one;
4-[3-[2-[5-[[4-(2,2-difluoroethyl)piperazin-1-yl]methyl]-2-pyridyl]-1H-pyrrolo[2,3-b]pyridin-4-yl]-5-fluoro-2-(hydroxymethyl)phenyl]-6-fluoro-8-(1-fluorocyclopropyl)-2,3-dihydro-1,4-benzoxazepin-5-one;
6-fluoro-8-(1-fluorocyclopropyl)-4-[5-fluoro-2-(hydroxymethyl)-3-[2-[5-[[4-(2,2,2-trifluoroethyl)piperazin-1-yl]methyl]-2-pyridyl]-1H-pyrrolo[2,3-b]pyridin-4-yl]phenyl]-2,3-dihydro-1,4-benzoxazepin-5-one;
6-fluoro-8-(1-fluorocyclopropyl)-4-[5-fluoro-2-(hydroxymethyl)-3-[2-[5-[[4-(oxetan-3-yl)piperazin-1-yl]methyl]-2-pyridyl]-1H-pyrrolo[2,3-b]pyridin-4-yl]phenyl]-2,3-dihydro-1,4-benzoxazepin-5-one;
8-cyclopropyl-6-fluoro-4-[5-fluoro-2-(hydroxymethyl)-3-[2-[5-[(3-hydroxy-3-methyl-azetidin-1-yl)methyl]-2-pyridyl]-1H-pyrrolo[2,3-b]pyridin-4-yl]phenyl]-2,3-dihydro-1,4-benzoxazepin-5-one;
8-cyclopropyl-6-fluoro-4-[5-fluoro-2-(hydroxymethyl)-3-[2-[5-[(4-hydroxy-4-methyl-1-piperidyl)methyl]-2-pyridyl]-1H-pyrrolo [2,3-b]pyridin-4-yl]phenyl]-2,3-dihydro-1,4-benzoxazepin-5-one;
8-cyclopropyl-4-[3-[2-[5-[[3,5-dimethyl-4-(2,2,2-trifluoroethyl)piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-4-[3-[2-[5-[[3,5-dimethyl-4-(oxetan-3-yl)piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-4-[3-[2-[5-[[4-(2,2-difluoroethyl)-3,5-dimethyl-piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-4-[3-[2-[5-[[4-(2,2-difluoroethyl)-3-methyl-piperazin-1-yl]methyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-6-fluoro-4-[5-fluoro-2-(hydroxymethyl)-3-[2-[5-[[3-methyl-4-(2,2,2-trifluoroethyl)piperazin-1-yl]methyl]-2-pyridyl]-1H-pyrrolo[2,3-b]pyridin-4-yl]phenyl]-2,3-dihydro-1,4-benzoxazepin-5-one;
8-cyclopropyl-6-fluoro-4-[5-fluoro-2-(hydroxymethyl)-3-[2-[5-[[3-methyl-4-(oxetan-3-yl)piperazin-1-yl]methyl]-2-pyridyl]-1H-pyrrolo[2,3-b]pyridin-4-yl]phenyl]-2,3-dihydro-1,4-benzoxazepin-5-one;
8-cyclopropyl-6-fluoro-4-[5-fluoro-2-(hydroxymethyl)-3-[2-[5-[1-methyl-1-[4-(oxetan-3-yl)piperazin-1-yl]ethyl]-2-pyridyl]-1H-pyrrolo[2,3-b]pyridin-4-yl]phenyl]-2,3-dihydro-1,4-benzoxazepin-5-one;
8-cyclopropyl-6-fluoro-4-[5-fluoro-2-(hydroxymethyl)-3-[2-[5-[1-methyl-1-[4-(2,2,2-trifluoroethyl)piperazin-1-yl]ethyl]-2-pyridyl]-1H-pyrrolo[2,3-b]pyridin-4-yl]phenyl]-2,3-dihydro-1,4-benzoxazepin-5-one;
8-cyclopropyl-4-[3-[2-[5-[1-[4-(2,2-difluoroethyl)piperazin-1-yl]-1-methyl-ethyl]-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-4-[3-[2-[5-[[4-(2,2-difluoroethyl)piperazin-1-yl]methyl]-4-fluoro-2-pyridyl]-1H-pyrrolo[2,3-b]pyridin-4-yl]-5-fluoro-2-(hydroxymethyl)phenyl]-6-fluoro-2,3-dihydro-1,4-benzoxazepin-5-one;
8-cyclopropyl-6-fluoro-4-[5-fluoro-3-[2-[4-fluoro-5-[[4-(2,2,2-trifluoroethyl)piperazin-1-yl]methyl]-2-pyridyl]-1H-pyrrolo[2,3-b]pyridin-4-yl]-2-(hydroxymethyl)phenyl]-2,3-dihydro-1,4-benzoxazepin-5-one;
8-cyclopropyl-6-fluoro-4-[5-fluoro-3-[2-[4-fluoro-5-[[4-(oxetan-3-yl)piperazin-1-yl]methyl]-2-pyridyl]-1H-pyrrolo[2,3-b]pyridin-4-yl]-2-(hydroxymethyl)phenyl]-2,3-dihydro-1,4-benzoxazepin-5-one; and
8-cyclopropyl-6-fluoro-4-[5-fluoro-2-(hydroxymethyl)-3-[2-[5-[[4-(oxetan-3-ylmethyl)piperazin-1-yl]methyl]-2-pyridyl]-1H-pyrrolo[2,3-b]pyridin-4-yl]phenyl]-2,3-dihydro-1,4-benzoxazepin-5-one.

**2.** A pharmaceutical composition comprising a compound or a pharmaceutically acceptable salt thereof of as claimed

in claim 1 together with a pharmaceutically acceptable carrier, optionally in combination with one or more other pharmaceutical compositions.

3. A pharmaceutical composition comprising a compound or a pharmaceutically acceptable salt thereof as claimed in claim 1 together with a pharmaceutically acceptable carrier, optionally in combination with one or more additional therapeutic agent.

4. A pharmaceutical composition as claimed in claim 3 wherein the additional therapeutic agent is selected from BTK inhibitors such as Ibrutinib, AVL-292, ONO-4059; B-cell depleting protein based therapeutics such as Rituxan, a CD20 antibody; calcineurin inhibitor such as cyclosporin A or FK 506; a mTOR inhibitor such as rapamycin, 40-O-(2-hydroxyethyl)-rapamycin, CCI779, ABT578, AP23573, AP23464, AP23675, AP23841 , TAFA-93, biolimus-7 or biolimus-9; an ascomycin having immunosuppressive properties such as ABT-281, ASM981; corticosteroids such as cortisone, dexamethasone, methylprednisolone, prednisolone, prednisone; cyclophosphamide; azathioprene; methotrexate; leflunomide; teriflunomide; mizoribine; etanercept; infliximab; a chemotherapeutic agent such as paclitaxel, gemcitabine, cisplatine, doxorubicin, 5-fluorouracil, imatinib, dasatinib, sunitinib, gefitinib, sorafenib, erlotinib, camptothecin, topotecan, daunomycin, etoposide, taxol, vinblastine, bortezomib, carfilzomib; mycophenolic acid or salt; mycophenolate mofetil; 15-deoxyspergualine or an immunosuppressive homologue, analogue or derivative thereof; NSAIDs such as Ibuprofen, flurbiprofen, naproxen, diclofenac, misoprostol, sulindac, oxaprozin, diflunisal, piroxicam, indomethacin, etodolac, fenoprofen, ketoprofen, sodium nabumetone, sulfasalazine, tolmetin sodium, hydroxychloroquine, celecoxib, valdecoxib, lumiracoxib, etoricoxib; JAK kinase inhibitors such as Tofacitinib, LY-3009104, VX-509, GLPG-0634, ASP-015K , N-benzyl-3,4-dihydroxy-benzylidene-cyanoacetamide $\alpha$-cyano-(3,4-dihydroxy)-]N-benzylcinnamamide (Tyrphostin AG 490), prodigiosin 25-C (PNU156804), [4-(4'-hydroxyphenyl)-amino-6,7-dimethoxyquinazoline] (WHI-P131), [4-(3'-bromo-4'-hydroxylphenyl)-amino-6,7-dimethoxyquinazoline] (WHI-P154), [4-(3',5'-dibromo-4'-hydroxylphenyl)-amino-6,7-dimethoxyquinazoline] WHI-P97, KRX-21 1, 3-{(3R,4R)-4-methyl-3-[methyl-(7H-pyrrolo[2,3-d]pyrimidin-4-yl)-amino]-piperidin-1-yl}-3-oxo-propionitrile, in free form or in a pharmaceutically acceptable salt form, e.g. mono-citrate (also called CP-690,550); SYK inhibitor such as fostamatinib; sphingosine-1-phosphate receptor modulators such as FTY720 (fingolimod); immunosuppressive monoclonal antibodies such as monoclonal antibodies to leukocyte receptors such as MHC, CD2, CD3, CD4, CD7, CD8, CD25, CD28, CD40, CD45, CD52, CD58, CD80, CD86 or their ligands; other immunomodulatory compounds such as a recombinant binding molecule having at least a portion of the extracellular domain of CTLA4 or a mutant thereof, e.g. an at least extracellular portion of CTLA4 or a mutant thereof joined to a non-CTLA4 protein sequence, e.g. CTLA41g (for ex. designated ATCC 68629) or a mutant thereof, e.g. LEA29Y; adhesion molecule inhibitors, e.g. LFA-1 antagonists, ICAM-1 or -3 antagonists, VCAM-4 antagonists or VLA-4 antagonists; or an anti-infectious agent; a PI3K inhibitor (e.g. pan, or alpha, beta, gamma, delta selectives), TNF inhibitors, ILI beta inhibitors, IL17 inhibitors, and inhibitors of IL6 or IL receptor.

5. A compound according to claim 1 or a pharmaceutically acceptable salt thereof, for use as a medicament.

6. A compound as claimed in claim 1 or its tautomers, polymorphs, stereoisomers, solvates, co-crystals or a pharmaceutically acceptable salt thereof, for use in treating conditions mediated by Btk wherein said conditions are selected from autoimmune disorders, inflammatory diseases, allergic diseases, airway diseases, comprising asthma and chronic obstructive pulmonary disease (COPD), transplant rejection, rheumatoid arthritis, systemic onset juvenile idiopathic arthritis (SOJIA), gout, pemphigus vulgaris, idiopathic thrombocytopenic purpura, systemic lupus erythematosus, multiple sclerosis, myasthenia gravis, Sjogren's syndrome, autoimmune hemolytic anemia, anti-neutrophil cytoplasmic antibodies (ANCA)-associated vasculitides, cryoglobulinemia, thrombotic thrombocytopenic purpura, chronic autoimmune urticaria, allergy (atopic dermatitis, contact dermatitis, allergic rhinitis), atherosclerosis, type 1 diabetes, type 2 diabetes, inflammatory bowel disease, ulcerative colitis, morbus Crohn, pancreatitis, glomerulonephritis, Goodpasture's syndrome, Hashimoto's thyroiditis, Grave's disease, antibody-mediated transplant rejection (AMR), graft versus host disease, B cell-mediated hyperacute, acute and chronic transplant rejection, thromboembolic disorders, myocardial infarct, angina pectoris, stroke, ischemic disorders, pulmonary embolism, multiple myeloma, leukemia, acute myelogenous leukemia, chronic myelogenous leukemia, lymphocytic leukemia, myeloid leukemia, non-Hodgkin lymphoma, lymphomas, polycythemia vera, chronic lymphocytic leukemia, Mantle cell lymphoma, essential thrombocythemia, myelofibrosis with myeloid metaplasia, and Waldenstroem disease.

## Patentansprüche

1. Verbindung oder deren Tautomere, Polymorphe, Stereoisomere, Solvate, Cokristalle oder pharmazeutisch unbedenkliches Salz davon, wobei die Verbindung aus der Gruppe bestehend aus
8-Cyclopropyl-6-fluor-4-[5-fluor-2-(hydroxymethyl)-3-[2-[5-[[4-(oxetan-3-yl)piperazin-1-yl]methyl]-2-pyridyl]-1H-pyrrolo[2,3-b]pyridin-4-yl]phenyl]-2,3-dihydro-1,4-benzoxazepin-5-on;
8-Cyclopropyl-6-fluor-4-[5-fluor-2-(hydroxymethyl)-3-[2-[5-[[4-(2,2,2-trifluorethyl)piperazin-1-yl]methyl]-2-pyridyl]-1H-pyrrolo[2,3-b]pyridin-4-yl]phenyl]-2,3-dihydro-1,4-benzoxazepin-5-on;
8-Cyclopropyl-4-[3-[2-[5-[[4-(2,2-difluorethyl)piperazin-1-yl]methyl]-2-pyridyl]-1H-pyrrolo[2,3-b]pyridin-4-yl]-5-fluor-2-(hydroxymethyl)phenyl]-6-fluor-2,3-dihydro-1,4-benzoxazepin-5-on;
8-Cyclopropyl-6-fluor-4-[5-fluor-2-(hydroxymethyl)-3-[2-[5-(morpholinomethyl)-2-pyridyl]-1H-pyrrolo[2,3-b]pyridin-4-yl]phenyl]-2,3-dihydro-1,4-benzoxazepin-5-on;
8-Cyclopropyl-4-[3-[2-[5-[[(3S)-4-(2,2-difluorethyl)-3-methylpiperazin-1-yl]methyl]-2-pyridyl]-1H-pyrrolo[2,3-b]pyridin-4-yl]-5-fluor-2-(hydroxymethyl)phenyl]-6-fluor-2,3-dihydro-1,4-benzoxazepin-5-on;
8-Cyclopropyl-4-[3-[2-[5-[[(2S)-4-(2,2-difluorethyl)-2-methylpiperazin-1-yl]methyl]-2-pyridyl]-1H-pyrrolo[2,3-b]pyridin-4-yl]-5-fluor-2-(hydroxymethyl)phenyl]-6-fluor-2,3-dihydro-1,4-benzoxazepin-5-on;
8-Cyclopropyl-4-[3-[2-[5-[[(3R)-4-(2,2-difluorethyl)-3-methylpiperazin-1-yl]methyl]-2-pyridyl]-1H-pyrrolo[2,3-b]pyridin-4-yl]-5-fluor-2-(hydroxymethyl)phenyl]-6-fluor-2,3-dihydro-1,4-benzoxazepin-5-on;
8-Cyclopropyl-6-fluor-4-[5-fluor-2-(hydroxymethyl)-3-[2-[5-[[1-(oxetan-3-yl)-4-piperidyl]oxy]-2-pyridyl]-1H-pyrrolo[2,3-6]pyridin-4-yl]phenyl]-2,3,-dihydro-1,4-benzoxazepin-5-5on;
6-Fluor-4-[5-fluor-2-(hydroxymethyl)-3-[2-[5-[[4-(oxetan-3-yl)piperazin-1-yl]methyl]-2-pyridyl]-1H-pyrrolo[2,3-b]pyridin-4-yl]phenyl]-8-(1-hydroxy-1-methylethyl)-2,3-dihydro-1,4-benzoxazepin-5-on;
6-Fluor-4-[5-fluor-2-(hydroxymethyl)-3-[2-[5-[[4-(2,2,2-trifluorethyl)piperazin-1-yl]methyl]-2-pyridyl]-1H-pyrrolo[2,3-b]pyridin-4-yl]phenyl]-8-(1-hydroxy-1-methyl-ethyl)-2,3-dihydro-1,4-benzoxazepin-5-on;
4-[3-[2-[5-[[4-(2,2-Difluorethyl)piperazin-1-yl]methyl]-2-pyridyl]-1H-pyrrolo[2,3-b]pyridin-4-yl]-5-fluor-2-(hydroxymethyl)phenyl]-6-fluor-8-(1-hydroxy-1-methyl-ethyl)-2,3-dihydro-1,4-benzoxazepin-5-on;
4-[3-[2-[5-[[4-(2,2-Difluorethyl)piperazin-1-yl]methyl]-2-pyridyl]-1H-pyrrolo[2,3-b]pyridin-4-yl]-5-fluor-2-(hydroxymethyl)phenyl]-6-fluor-8-(1-fluorcyclopropyl)-2,3-dihydro-1,4-benzoxazepin-5-on;
6-Fluor-8-(1-fluorcyclopropyl)-4-[5-fluor-2-(hydroxymethyl)-3-[2-[5-[[4-(2,2,2-trifluorethyl)piperazin-1-yl]methyl]-2-pyridyl]-1H-pyrrolo[2,3-b]pyridin-4-yl]phenyl]-2,3-dihydro-1,4-benzoxazepin-5-on;
6-Fluor-8-(1-fluorcyclopropyl)-4-[5-fluor-2-(hydroxymethyl)-3-[2-[5-[[4-(oxetan-3-yl)piperazin-1-yl]methyl]-2-pyridyl]-1H-pyrrolo[2,3-b]pyridin-4-yl]phenyl]-2,3-dihydro-1,4-benzoxazepin-5-on;
8-Cyclopropyl-6-fluor-4-[5-fluor-2-(hydroxymethyl)-3-[2-[5-[(3-hydroxy-3-methylazetidin-1-yl)methyl]-2-pyridyl]-1H-pyrrolo[2,3-b]pyridin-4-yl]phenyl]-2,3-dihydro-1,4-benzoxazepin-5-on;

8-Cyclopropyl-6-fluor-4-[5-fluor-2-(hydroxymethyl)-3-[2-[5-[(4-hydroxy-4-methyl-1-piperidyl)methyl]-2-pyridyl]-1H-pyrrolo[2,3-b]pyridin-4-yl]phenyl]-2,3-dihydro-1,4-benzoxazepin-5-on;
8-Cyclopropyl-4-[3-[2-[5-[[3,5-dimethyl-4-(2,2,2-trifluorethyl)piperazin-1-yl]methyl]-2-pyridyl]-1H-pyrrolo[2,3-b]pyridin-4-yl]-5-fluor-2-(hydroxymethyl)phenyl]-6-fluor-2,3-dihydro-1,4-benzoxazepin-5-on;
8-Cyclopropyl-4-[3-[2-[5-[[3,5-dimethyl-4-(oxetan-3-yl)piperazin-1-yl]methyl]-2-pyridyl]-1H-pyrrolo[2,3-b]pyridin-4-yl]-5-fluor-2-(hydroxymethyl)phenyl]-6-fluor-2,3-dihydro-1,4-benzoxazepin-5-on;
8-Cyclopropyl-4-[3-[2-[5-[[4-(2,2-difluorethyl)-3,5-dimethyl-piperazin-1-yl]methyl]-2-pyridyl]-1H-pyrrolo[2,3-b]pyridin-4-yl]-5-fluor-2-(hydroxymethyl)phenyl]-6-fluor-2,3-dihydro-1,4-benzoxazepin-5-on;
8-Cyclopropyl-4-[3-[2-[5-[[4-(2,2-difluorethyl)-3-methyl-piperazin-1-yl]methyl]-2-pyridyl]-1H-pyrrolo[2,3-b]pyridin-4-yl]-5-fluor-2-(hydroxymethyl)phenyl]-6-fluor-2,3-dihydro-1,4-benzoxazepin-5-on;
8-Cyclopropyl-6-fluor-4-[5-fluor-2-(hydroxymethyl)-3-[2-[5-[[3-methyl-4-(2,2,2-trifluorethyl)piperazin-1-yl]methyl]-2-pyridyl]-1H-pyrrolo[2,3-b]pyridin-4-yl]phenyl]-2,3-dihydro-1,4-benzoxazepin-5-on;
8-Cyclopropyl-6-fluor-4-[5-fluor-2-(hydroxymethyl)-3-[2-[5-[[3-methyl-4-(oxetan-3-yl)piperazin-1-yl]methyl]-2-pyridyl]-1H-pyrrolo[2,3-b]pyridin-4-yl]phenyl]-2,3-dihydro-1,4-benzoxazepin-5-on;
8-Cyclopropyl-6-fluor-4-[5-fluor-2-(hydroxymethyl)-3-[2-[5-[1-methyl-1-[4-(oxetan-3-yl)piperazin-1-yl]ethyl]-2-pyridyl]-1H-pyrrolo[2,3 -b]pyridin-4-yl]phenyl]-2,3-dihydro-1,4-benzoxazepin-5-on;
8-Cyclopropyl-6-fluor-4-[5-fluor-2-(hydroxymethyl)-3-[2-[5-[1-methyl-1-[4-(2,2,2-trifluorethyl)piperazin-1-yl]ethyl]-2-pyridyl]-1H-pyrrolo[2,3-b]pyridin-4-yl]phenyl]-2,3-dihydro-1,4-benzoxazepin-5-on;
8-Cyclopropyl-4-[3-[2-[5-[1-[4-(2,2-difluorethyl)piperazin-1-yl]-1-methylethyl]-2-pyridyl]-1H-pyrrolo[2,3-b]pyridin-4-yl]-5-fluor-2-(hydroxymethyl)phenyl]-6-fluor-2,3-dihydro-1,4-benzoxazepin-5-on;
8-Cyclopropyl-4-[3-[2-[5-[[4-(2,2-difluorethyl)piperazin-1-yl]methyl]-4-fluor-2-pyridyl]-1H-pyrrolo[2,3-b]pyridin-4-yl]-5-fluor-2-(hydroxymethyl)phenyl]-6-fluor-2,3-dihydro-1,4-benzoxazepin-5-on;
8-Cyclopropyl-6-fluor-4-[5-fluor-3-[2-[4-fluor-5-[[4-(2,2,2-trifluorethyl)piperazin-1-yl]methyl]-2-pyridyl]-1H-pyrrolo[2,3-b]pyridin-4-yl]-2-(hydroxymethyl)phenyl]-2,3-dihydro-1,4-benzoxazepin-5-on;
8-Cyclopropyl-6-fluor-4-[5-fluor-3-[2-[4-fluor-5-[[4-(oxetan-3-yl)piperazin-1-yl]methyl]-2-pyridyl]-1H-pyrrolo[2,3-b]pyridin-4-yl]-2-(hydroxymethyl)phenyl]-2,3-dihydro-1,4-benzoxazepin-5-on; und

8-Cyclopropyl-6-fluor-4-[5-fluor-2-(hydroxymethyl)-3-[2-[5-[[4-(oxetan-3-ylmethyl)piperazin-1-yl]methyl]-2-pyridyl]-1H-pyrrolo[2,3-b]pyridin-4-yl]phenyl]-2,3-dihydro-1,4-benzoxazepin-5-on
ausgewählt ist.

**2.** Pharmazeutische Zusammensetzung, umfassend eine Verbindung oder ein pharmazeutisch unbedenkliches Salz davon nach Anspruch 1 zusammen mit einem pharmazeutisch unbedenklichen Träger, gegebenenfalls in Kombination mit einer oder mehreren anderen pharmazeutischen Zusammensetzungen.

**3.** Pharmazeutische Zusammensetzung, umfassend eine Verbindung oder ein pharmazeutisch unbedenkliches Salz davon nach Anspruch 1 zusammen mit einem pharmazeutisch unbedenklichen Träger, gegebenenfalls in Kombination mit einem oder mehreren zusätzlichen therapeutischen Mitteln.

**4.** Pharmazeutische Zusammensetzung nach Anspruch 3, wobei das zusätzliche therapeutische Mittel aus BTK-Inhibitoren wie Ibrutinib, AVL-292, ONO-4059; Therapeutika auf Basis von B-Zell-depletierenden Proteinen wie Rituxan, einem CD20-Antikörper; Calcineurin-Inhibitoren wie Cyclosporin A oder FK 506; einem mTOR-Inhibitor wie Rapamycin, 40-O-(2-Hydroxyethyl)rapamycin, CCI779, ABT578, AP23573, AP23464, AP23675, AP23841, TAFA-93, Biolimus-7 oder Biolimus-9; einem Ascomycin mit immunsuppressiven Eigenschaften wie ABT-281, ASM981; Corticosteroiden wie Cortison, Dexamethason, Methylprednisolon, Prednisolon, Prednison; Cyclophosphamid; Azathiopren; Methotrexat; Leflunomid; Teriflunomid; Mizoribin; Etanercept; Infliximab; einem chemotherapeutischen Mittel wie Paclitaxel, Gemcitabin, Cisplatin, Doxorubicin, 5-Fluoruracil, Imatinib, Dasatinib, Sunitinib, Gefitinib, Sorafenib, Erlotinib, Camptothecin, Topotecan, Daunomycin, Etoposid, Taxol, Vinblastin, Bortezomib, Carfilzomib; Mycophenolsäure oder einem Salz davon; Mycophenolat-Mofetil; 15-Desoxyspergualin oder einem immunsuppressiven Homolog, Analog oder Derivat davon; NSAIDs wie Ibuprofen, Flurbiprofen, Naproxen, Diclofenac, Misoprostol, Sulindac, Oxaprozin, Diflunisal, Piroxicam, Indomethacin, Etodolac, Fenoprofen, Ketoprofen, Natrium-Nabumeton, Sulfasalazin, Tolmetin-Natrium, Hydroxychloroquin, Celecoxib, Valdecoxib, Lumiracoxib, Etoricoxib; JAK-Kinase-Inhibitoren wie Tofacitinib, LY-3009104, VX-509, GLPG-0634, ASP-015K, N-Benzyl-3,4-dihydroxy-benzylidencyanoacetamid, $\alpha$-Cyano-(3,4-dihydroxy)-]N-benzylcinnamamid (Tyrphostin AG 490), Prodigiosin 25-C (PNU156804), [4-(4'-Hydroxyphenyl)-amino-6,7-dimethoxychinazolin] (WHI-P131), [4-(3'-Brom-4'-hydroxylphenyl)amino-6,7-dimethoxy-chinazolin] (WHI-P154), [4-(3',5'-Dibrom-4'-hydroxylphenyl)-amino-6,7-dimethoxychinazolin] WHI-P97, KRX-211, 3-{(3R,4R)-4-Methyl-3-[methyl-(7H-pyrrolo[2,3-d]pyrimidin-4-yl)amino]piperidin-1-yl}-3-oxopropionitril, in freier Form oder in einer pharmazeutisch unbedenklichen Salzform (z. B. Monocitrat (auch als CP-690,550 bezeichnet); SYK-Inhibitoren wie Fostamatinib; Sphingosin-1-phosphat-Rezeptor-Modulatoren wie FTY720 (Fingolimod); immunsuppressiven monoklonalen Antikörpern wie monoklonalen Antikörpern gegen Leukozytenrezeptoren wie MHC, CD2, CD3, CD4, CD7, CD8, CD25, CD28, CD40, CD45, CD52, CD58, CD80, CD86 oder deren Liganden; anderen immunmodulatorischen Verbindungen wie einem rekombinanten Bindungsmolekül mit mindestens einem Teil der extrazellulären Domäne von CTLA4 oder einer Mutante davon, z. B. einem mindestens extrazellulären Teil von CTLA4 oder einer Mutante davon, der mit einer Nicht-CTLA4-Proteinsequenz verknüpft ist, z. B. CTLA41g (zum Beispiel als ATCC 68629 bezeichnet) oder einer Mutante davon, z. B. LEA29Y; Adhäsionsmolekülinhibitoren, z. B. LFA-1-Antagonisten, ICAM-1- oder -3-Antagonisten, VCAM-4-Antagonisten oder VLA-4-Antagonisten; oder einem Antiinfektiosum; einem PI3K-Inhibitor (z. B. pan oder alpha-, beta-, gamma-, delta-selektiv), TNF-Inhibitoren, ILI-beta-Inhibitoren, IL17-Inhibitoren und Inhibitoren von IL6 oder IL-Rezeptor ausgewählt ist.

**5.** Verbindung nach Anspruch 1 oder pharmazeutisch unbedenkliches Salz davon zur Verwendung als Medikament.

**6.** Verbindung nach Anspruch 1 oder deren Tautomere, Polymorphe, Stereoisomere, Solvate, Cokristalle oder pharmazeutisch unbedenkliches Salz davon zur Verwendung bei der Behandlung von Leiden, die durch Btk vermittelt werden, wobei die Leiden aus Autoimmunstörungen, entzündlichen Erkrankungen, allergischen Erkrankungen, Atemwegserkrankungen, umfassend Asthma und chronische obstruktive Lungenerkrankung (COPD), Transplantatabstoßung, rheumatoider Arthritis, systemischer juveniler idiopathischer Arthritis (SOJIA), Gicht, Pemphigus vulgaris, idiopathischer thrombozytopenischer Purpura, systemischem Lupus erythematodes, multipler Sklerose, Myasthenia gravis, Sjögren-Syndrom, autoimmunhämolytischer Anämie, mit ANCA (anti-neutrophil cytoplasmic antibodies) assoziierten Vaskulitiden, Kryoglobulinämie, thrombozytisch-thrombozytopenischer Purpura, chronischer Autoimmun-Nesselsucht, Allergie (atopischer Dermatitis, Kontaktdermatitis, allergischer Rhinitis), Atherosklerose, Typ-1-Diabetes, Typ-2-Diabetes, entzündlicher Darmerkrankung, Colitis ulcerosa, Morbus Crohn, Pankreatitis, Glomerulonephritis, Goodpasture-Syndrom, Hashimoto-Thyreoiditis, Morbus Basedow, antikörpervermittelter Transplantatabstoßung (AMR, antibody-mediated transplant rejection), Graftversus-Host-Erkrankung, B-Zell-vermittelter hyperakuter, akuter und chronischer Transplantatabstoßung, thromboembolischen Störungen, Myokardinfarkt, Angina pectoris, Schlaganfall, ischämischen Störungen, Lungenembolie, multiplem Myelom; Leukämie, akuter myeloischer Leukämie, chronischer myeloischer Leukämie, lymphatischer Leukämie, myeloischer Leukämie, Non-Hodgkin-Lymphom, Lymphomen, Polycythemia vera, Mantelzelllymphom, essenzieller Thrombozythämie, Myelofibrose mit myeloischer Metaplasie und Morbus Waldenström ausgewählt sind.

## Revendications

1. Composé ou ses formes tautomères, ses formes polymorphes, ses stéréoisomères, ses solvates, ses cocristaux ou un sel pharmaceutiquement acceptable correspondant, le composé étant choisi dans le groupe constitué par :

   8-cyclopropyl-6-fluoro-4-[5-fluoro-2-(hydroxyméthyl)-3-[2-[5-[[4-(oxétan-3-yl)pipérazin-1-yl]méthyl]-2-pyridinyl]-1*H*-pyrrolo[2,3-*b*]pyridin-4-yl]phényl]-2,3-dihydro-1,4-benzoxazépin-5-one ;
   8-cyclopropyl-6-fluoro-4-[5-fluoro-2-(hydroxyméthyl)-3-[2-[5-[[4-(2,2,2-trifluoroéthyl)pipérazin-1-yl]méthyl]-2-pyridinyl]-1*H*-pyrrolo[2,3-*b*]pyridin-4-yl]phényl]-2,3-dihydro-1,4-benzoxazépin-5-one ;
   8-cyclopropyl-4-[3-[2-[5-[[4-(2,2-difluoroéthyl)pipérazin-1-yl]méthyl]-2-pyridinyl]-1*H*-pyrrolo[2,3-*b*]pyridin-4-yl]-5-fluoro-2-(hydroxyméthyl)phényl]-6-fluoro-2,3-dihydro-1,4-benzoxazépin-5-one ;
   8-cyclopropyl-6-fluoro-4-[5-fluoro-2-(hydroxyméthyl)-3-[2-[5-(morpholinométhyl)-2-pyridinyl]-1*H*-pyrrolo[2,3-*b*]pyridin-4-yl]phényl]-2,3-dihydro-1,4-benzoxazépin-5-one ;
   8-cyclopropyl-4-[3-[2-[5-[[(3*S*)-4-(2,2-difluoroéthyl)-3-méthyl-pipérazin-1-yl]méthyl]-2-pyridinyl]-1*H*-pyrrolo[2,3-*b*]pyridin-4-yl]-5-fluoro-2-(hydroxyméthyl)phényl]-6-fluoro-2,3-dihydro-1,4-benzoxazépin-5-one ;
   8-cyclopropyl-4-[3-[2-[5-[[(2*S*)-4-(2,2-difluoroéthyl)-2-méthyl-pipérazin-1-yl]méthyl]-2-pyridinyl]-1*H*-pyrrolo[2,3-*b*]pyridin-4-yl]-5-fluoro-2-(hydroxyméthyl)phényl]-6-fluoro-2,3-dihydro-1,4-benzoxazépin-5-one ;
   8-cyclopropyl-4-[3-[2-[5-[[(3*R*)-4-(2,2-difluoroéthyl)-3-méthyl-pipérazin-1-yl]méthyl]-2-pyridinyl]-1*H*-pyrrolo[2,3-*b*]pyridin-4-yl]-5-fluoro-2-(hydroxyméthyl)phényl]-6-fluoro-2,3-dihydro-1,4-benzoxazépin-5-one ;
   8-cyclopropyl-6-fluoro-4-[5-fluoro-2-(hydroxyméthyl)-3-[2-[5-[[1-(oxétan-3-yl)-4-pipéridyl]oxy]-2-pyridinyl]-1*H*-pyrrolo[2,3-*b*]pyridin-4-yl]phényl]-2,3-dihydro-1,4-benzoxazépin-5-one ;
   6-fluoro-4-[5-fluoro-2-(hydroxyméthyl)-3-[2-[5-[[4-(oxétan-3-yl)pipérazin-1-yl]méthyl]-2-pyridinyl]-1*H*-pyrrolo[2,3-*b*]pyridin-4-yl]phényl]-8-(1-hydroxy-1-méthyl-éthyl)-2,3-dihydro-1,4-benzoxazépin-5-one ;
   6-fluoro-4-[5-fluoro-2-(hydroxyméthyl)-3-[2-[5-[[4-(2,2,2-trifluoroéthyl)pipérazin-1-yl]méthyl]-2-pyridinyl]-1*H*-pyrrolo[2,3-*b*]pyridin-4-yl]phényl]-8-(1-hydroxy-1-méthyl-éthyl)-2,3-dihydro-1,4-benzoxazépin-5-one ;
   4-[3-[2-[5-[[4-(2,2-difluoroéthyl)pipérazin-1-yl]méthyl]-2-pyridinyl]-1*H*-pyrrolo[2,3-*b*]pyridin-4-yl]-5-fluoro-2-(hydroxyméthyl)phényl]-6-fluoro-8-(1-hydroxy-1-méthyl-éthyl)-2,3-dihydro-1,4-benzoxazépin-5-one ;
   4-[3-[2-[5-[[4-(2,2-difluoroéthyl)pipérazin-1-yl]méthyl]-2-pyridinyl]-1*H*-pyrrolo[2,3-*b*]pyridin-4-yl]-5-fluoro-2-(hydroxyméthyl)phényl]-6-fluoro-8-(1-fluorocyclopropyl)-2,3-dihydro-1,4-benzoxazépin-5-one ;
   6-fluoro-8-(1-fluorocyclopropyl)-4-[5-fluoro-2-(hydroxyméthyl)-3-[2-[5-[[4-(2,2,2-trifluoroéthyl)pipérazin-1-yl]méthyl]-2-pyridinyl]-1*H*-pyrrolo[2,3-*b*]pyridin-4-yl]phényl]-2,3-dihydro-1,4-benzoxazépin-5-one ;
   6-fluoro-8-(1-fluorocyclopropyl)-4-[5-fluoro-2-(hydroxyméthyl)-3-[2-[5-[[4-(oxétan-3-yl)pipérazin-1-yl]méthyl]-2-pyridinyl]-1*H*-pyrrolo[2,3-*b*]pyridin-4-yl]phényl]-2,3-dihydro-1,4-benzoxazépin-5-one ;
   8-cyclopropyl-6-fluoro-4-[5-fluoro-2-(hydroxyméthyl)-3-[2-[5-[(3-hydroxy-3-méthyl-azétidin-1-yl)méthyl]-2-pyridinyl]-1*H*-pyrrolo[2,3-*b*]pyridin-4-yl]phényl]-2,3-dihydro-1,4-benzoxazépin-5-one ;
   8-cyclopropyl-6-fluoro-4-[5-fluoro-2-(hydroxyméthyl)-3-[2-[5-[(4-hydroxy-4-méthyl-1-pipéridyl)méthyl]-2-pyridinyl]-1*H*-pyrrolo[2,3-*b*]pyridin-4-yl]phényl]-2,3-dihydro-1,4-benzoxazépin-5-one ;
   8-cyclopropyl-4-[3-[2-[5-[[3,5-diméthyl-4-(2,2,2-trifluoroéthyl)pipérazin-1-yl]méthyl]-2-pyridinyl]-1*H*-pyrrolo[2,3-*b*]pyridin-4-yl]-5-fluoro-2-(hydroxyméthyl)phényl]-6-fluoro-2,3-dihydro-1,4-benzoxazépin-5-one ;
   8-cyclopropyl-4-[3-[2-[5-[[3,5-diméthyl-4-(oxétan-3-yl)pipérazin-1-yl]méthyl]-2-pyridinyl]-1*H*-pyrrolo[2,3-*b*]pyridin-4-yl]-5-fluoro-2-(hydroxyméthyl)phényl]-6-fluoro-2,3-dihydro-1,4-benzoxazépin-5-one ;
   8-cyclopropyl-4-[3-[2-[5-[[4-(2,2-difluoroéthyl)-3,5-diméthyl-pipérazin-1-yl]méthyl]-2-pyridinyl]-1*H*-pyrrolo[2,3-*b*]pyridin-4-yl]-5-fluoro-2-(hydroxyméthyl)phényl]-6-fluoro-2,3-dihydro-1,4-benzoxazépin-5-one ;
   8-cyclopropyl-4-[3-[2-[5-[[4-(2,2-difluoroéthyl)-3-méthyl-pipérazin-1-yl]méthyl]-2-pyridinyl]-1*H*-pyrrolo[2,3-*b*]pyridin-4-yl]-5-fluoro-2-(hydroxyméthyl)phényl]-6-fluoro-2,3-dihydro-1,4-benzoxazépin-5-one ;
   8-cyclopropyl-6-fluoro-4-[5-fluoro-2-(hydroxyméthyl)-3-[2-[5-[[3-méthyl-4-(2,2,2-trifluoroéthyl)pipérazin-1-yl]méthyl]-2-pyridinyl]-1*H*-pyrrolo[2,3-*b*]pyridin-4-yl]phényl]-2,3-dihydro-1,4-benzoxazépin-5-one ;
   8-cyclopropyl-6-fluoro-4-[5-fluoro-2-(hydroxyméthyl)-3-[2-[5-[[3-méthyl-4-(oxétan-3-yl)pipérazin-1-yl]méthyl]-2-pyridinyl]-1*H*-pyrrolo[2,3-*b*]pyridin-4-yl]phényl]-2,3-dihydro-1,4-benzoxazépin-5-one ;
   8-cyclopropyl-6-fluoro-4-[5-fluoro-2-(hydroxyméthyl)-3-[2-[5-[1-méthyl-1-[4-(oxétan-3-yl)pipérazin-1-yl]éthyl]-2-pyridinyl]-1*H*-pyrrolo[2,3-*b*]pyridin-4-yl]phényl]-2,3-dihydro-1,4-benzoxazépin-5-one ;
   8-cyclopropyl-6-fluoro-4-[5-fluoro-2-(hydroxyméthyl)-3-[2-[5-[1-méthyl-1-[4-(2,2,2-trifluoroéthyl)pipérazin-1-yl]éthyl]-2-pyridinyl]-1*H*-pyrrolo[2,3-*b*]pyridin-4-yl]phényl]-2,3-dihydro-1,4-benzoxazépin-5-one ;

8-cyclopropyl-4-[3-[2-[5-[1-[4-(2,2-difluoroéthyl)pipérazin-1-yl]-1-méthyl-éthyl]-2-pyridinyl]-1*H*-pyrrolo[2,3-*b*]pyridin-4-yl]-5-fluoro-2-(hydroxyméthyl)phényl]-6-fluoro-2,3-dihydro-1,4-benzoxazépin-5-one ;
8-cyclopropyl-4-[3-[2-[5-[[4-(2,2-difluoroéthyl)pipérazin-1-yl]méthyl]-4-fluoro-2-pyridinyl]-1*H*-pyrrolo[2,3-*b*]pyridin-4-yl]-5-fluoro-2-(hydroxyméthyl)phényl]-6-fluoro-2,3-dihydro-1,4-benzoxazépin-5-one ;
8-cyclopropyl-6-fluoro-4-[5-fluoro-3-[2-[4-fluoro-5-[[4-(2,2,2-trifluoroéthyl)pipérazin-1-yl]méthyl]-2-pyridinyl]-1*H*-pyrrolo[2,3-*b*]pyridin-4-yl]-2-(hydroxyméthyl)phényl]-2,3-dihydro-1,4-benzoxazépin-5-one ;
8-cyclopropyl-6-fluoro-4-[5-fluoro-3-[2-[4-fluoro-5-[[4-(oxétan-3-yl)pipérazin-1-yl]méthyl]-2-pyridinyl]-1*H*-pyrrolo[2,3-*b*]pyridin-4-yl]-2-(hydroxyméthyl)phényl]-2,3-dihydro-1,4-benzoxazépin-5-one ;
et
8-cyclopropyl-6-fluoro-4-[5-fluoro-2-(hydroxyméthyl)-3-[2-[5-[[4-(oxétan-3-ylméthyl)pipérazin-1-yl]méthyl]-2-pyridinyl]-1*H*-pyrrolo[2,3-*b*]pyridin-4-yl]phényl]-2,3-dihydro-1,4-benzoxazépin-5-one.

**2.** Composition pharmaceutique comprenant un composé ou un sel pharmaceutiquement acceptable correspondant selon la revendication 1 conjointement avec un vecteur pharmaceutiquement acceptable, éventuellement en combinaison avec une ou plusieurs autres composition(s) pharmaceutique (s).

**3.** Composition pharmaceutique comprenant un composé ou sel pharmaceutiquement acceptable correspondant selon la revendication 1 conjointement avec un vecteur pharmaceutiquement acceptable, éventuellement en combinaison avec un ou plusieurs agent(s) thérapeutique(s) supplémentaire(s).

**4.** Composition pharmaceutique selon la revendication 3, l'agent thérapeutique supplémentaire étant choisi parmi les inhibiteurs de BTK tels que l'ibrutinib, l'AVL-292, l'ONO-4059 ; les agents thérapeutiques à base de protéines à action appauvrissante sur les cellules B tels que le rituxan, un anticorps anti-CD20 ; un inhibiteur de calcineurine tel que la cyclosporine A ou le FK 506 ; un inhibiteur de mTOR tel que la rapamycine, la 40-O-(2-hydroxyéthyl)-rapamycine, le CCI779, l'ABT578, l'AP23573, l'AP23464, l'AP23675, l'AP23841, le TAFA-93, le biolimus-7 ou le biolimus-9 ; une ascomycine possédant des propriétés immunosuppressives telle que l'ABT-281, l'ASM981 ; les corticostéroïdes tels que la cortisone, la dexaméthasone, la méthylprednisolone, la prednisolone, la prednisone ; le cyclophosphamide ; l'azathioprène ; le méthotrexate ; le léflunomide ; le tériflunomide ; la mizoribine ; l'étanercept ; l'infliximab ; un agent chimiothérapeutique tel que le paclitaxel, la gemcitabine, le cisplatine, la doxorubicine, le 5-fluorouracil, l'imatinib, le dasatinib, le sunitinib, le géfitinib, le sorafénib, l'erlotinib, la camptothécine, le topotécane, la daunomycine, l'étoposide, le taxol, la vinblastine, le bortézomib, le carfilzomib ; l'acide mycophénolique ou un sel correspondant ; le mycophénolate mofétil ; la 15-déoxyspergualine ou un homologue immunosuppresseur, un analogue immunosuppresseur ou un dérivé correspondant ; des médicaments AINS (anti-inflammatoires non stéroïdiens) tels que l'ibuprofène, le flurbiprofène, le naproxène, le diclofénac, le misoprostol, le sulindac, l'oxaprozine, le diflunisal, le piroxicam, l'indométhacine, l'étodolac, le fenoprofène, le kétoprofène, le sodium nabumétone, la sulfasalazine, la tolmétine sodique, l'hydroxychloroquine, le célécoxib, le valdécoxib, le lumiracoxib, l'étoricoxib; les inhibiteurs de JAK kinase tels que le tofacitinib, le LY-3009104, le VX-509, le GLPG-0634, l'ASP-015K, le N-benzyl-3,4-dihydroxybenzylidène-cyanoacétamide, l'$\alpha$-cyano-(3,4-dihydroxy)-N-benzylcinnamamide (tyrphostin AG 490), la prodigiosine 25-C (PNU156804), la [4-(4'-hydroxyphényl)-amino-6,7-diméthoxyquinazoline] (WHI-P131), la [4-(3'-bromo-4'-hydroxylphényl)-amino-6,7-diméthoxyquinazoline] (WHI-P154), la [4-(3',5'-dibromo-4'-hydroxyphényl)-amino-6,7-diméthoxyquinazoline] WHI-P97, le KRX-211, le 1,3-{(3*R*,4*R*)-4-méthyl-3-[méthyl-(7*H*-pyrrolo[2,3-*d*]pyrimidin-4-yl)-amino]-pipéridin-1-yl}-3-oxo-propionitrile, sous forme libre ou sous forme d'un sel pharmaceutiquement acceptable, par exemple un monocitrate (également appelé CP-690,550) ; un inhibiteur de SYK tel que le fostamatinib ; les modulateurs du récepteur du sphingosine-1-phosphate tels que le FTY720 (fingolimod) ; les anticorps monoclonaux immunosuppresseurs tels que les anticorps monoclonaux dirigés contre les récepteurs de leucocytes tels que le MHC, le CD2, le CD3, le CD4, le CD7, le CD8, le CD25, le CD28, le CD40, le CD45, le CD52, le CD58, le CD80, le CD86 ou leurs ligands ; autres composés immunomodulateurs tels qu'une molécule de liaison recombinante possédant au moins une portion du domaine extracellulaire de CTLA4 ou d'un mutant correspondant, par exemple au moins une portion du domaine extracellulaire de CTLA4 ou d'un mutant correspondant lié à une séquence protéique qui n'est pas de CTLA4, par exemple le CTLA41g (par exemple appelé ATCC 68629) ou un mutant correspondant, par exemple le LEA29Y ; les inhibiteurs de molécules d'adhérence, par exemple les antagonistes de LFA-1, les antagonistes de ICAM-1 ou les antagonistes de ICAM-3, les antagonistes de VCAM-4 ou les antagonistes de VLA-4 ; ou un agent anti-infectieux ; un inhibiteur de PI3K (par exemple à sélectivité pan ou à sélectivité alpha, bêta, gamma, delta), des inhibiteurs de TNF, les inhibiteurs de ILI-bêta, les inhibiteurs de IL17 et les inhibiteurs du récepteur d'IL6 ou d'IL.

**5.** Composé selon la revendication 1 ou un sel pharmaceutiquement acceptable correspondant, pour une utilisation en tant que médicament.

**6.** Composé selon la revendication 1 ou ses formes tautomères, ses formes polymorphes, ses stéréoisomères, ses solvates, ses cocristaux ou un sel pharmaceutiquement acceptable correspondant, pour une utilisation dans le traitement d'affections médiées par le Btk, lesdites affections étant choisies parmi les troubles auto-immunes, les maladies inflammatoires, les maladies allergiques, les maladies des voies respiratoires, comprenant l'asthme et la bronchopneumopathie chronique obstructive (BPCO), les rejets de greffe, la polyarthrite, l'arthrite juvénile idiopathique à début systémique (SOJIA), la goutte, le pemphigus vulgaire, le purpura thrombopénique idiopathique, le lupus érythémateux systémique, la sclérose en plaques, la myasthénie grave, le syndrome de Sjogren, l'anémie hémolytique auto-immune, les vascularites associées aux anticorps antineutrophiles cytoplasmiques (ANCA), la cryoglobulinémie, le purpura thrombocytopénique thrombotique, l'urticaire auto-immune chronique, une allergie (dermatite atopique, dermatite de contact, rhinite allergique), l'athérosclérose, le diabète de type 1, le diabète de type 2, une maladie intestinale inflammatoire, la colite ulcéreuse, la maladie de Crohn, la pancréatite, la glomérulonéphrite, le syndrome de Goodpasture, la thyroïdite d'Hashimoto, la maladie de Grave, le rejet de greffe médié par des anticorps (AMR), la maladie du greffon contre l'hôte, le rejet de greffe hyper aigu, aigu et chronique médié par les lymphocytes B, les troubles thromboemboliques, l'infarctus du myocarde, l'angine de poitrine, l'accident vasculaire cérébral, les troubles ischémiques, l'embolie pulmonaire, le myélome multiple, la leucémie, la leucémie myélogène aiguë, la leucémie myélogène chronique, la leucémie lymphoïde, la leucémie myéloïde, le lymphome non hodgkinien, les lymphomes, la maladie de Vaquez, la leucémie lymphoïde chronique, le lymphome à cellule du manteau, la thrombocytémie essentielle, la myélofibrose avec métaplasie myéloïde et la maladie de Waldenström.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011046964 A2
- WO 2010123870 A1
- WO 2010068810 A2
- WO 2010068788 A1
- WO 2010068806 A1
- WO 2010122038 A1
- WO 2010100070 A1
- WO 2013024078 A
- WO 2010006970 A1
- WO 2007027729 A1
- WO 2011019780 A1
- WO 2010144647 A1
- WO 2010080481 A1
- WO 2013008095 A
- WO 2013157022 A
- WO 2010000633 A
- WO 2010006947 A
- WO 2010100070 A
- WO 03000695 A
- WO 2003000688 A
- WO 2010003133 A


### Non-patent literature cited in the description


- **SCHAEFFER ; SCHWARTZBERG.** *Curr. Op. Imm.,* 2000, 282
- **NIIRO ; CLARK.** *Nature Rev. Immumol.,* 2002, 945
- **DI PAOLO ; CURRIE.** *Nat. Chem. Biol.,* 2011, 7
- **JEFFRIES et al.** *J. Biol. Chem.,* 2003, 26258
- **HORWOOD et al.** *J. Experimental Med.,* 2003, 1603
- **JANSSON ; HOLMDAHL.** *Clin. Exp. Immumol.,* 1993, 459
- **PAN et al.** *Chem. Med. Chem.,* 2007, 58
- **DI PAOLO.** *Nat. Chem. Biol.,* 2010, 41
- **BURGER et al.** *Blood,* 2010, 32
- *J Clin Oncol,* 2011, 6508
- **LOU et al.** *J. Med. Chem.,* 2012
- **KIM et al.** *Bio-org. Med. Chem. Lett.,* 2011, 6258
- **E.W. MARTIN.** *Remington's Pharmaceutical Sciences*
- *Chemical Reviews,* vol. 95 (7), 2457-2483
- *J. Org. Chem.,* 2009, vol. 74, 5599-5602
- *Chem. Sci.,* vol. 2, 27-50
- *Pure Appl. Chem,* vol. 71 (8), 1416-1423
- *J. Am. Chem. Soc.,* 2001, vol. 123 (31), 7727-7729
- *Organic Reactions,* 2002, vol. 1, 59
- *Chem. Commun.,* 2008, 1100-1102
- Remington's Pharmaceutical Sciences. Mack Pub. Co, 1991